# EUROPEAN PATENT APPLICATION

(11) **EP 1 854 485 A1**
(43) Date of publication of application: **14.11.2007**
(21) Application number: 06715040.9
(22) Date of filing: 23.02.2006
(51) Int. Cl.: A61K 45/00, A61K 31/7088, A61K 39/395, A61K 48/00, A61P 35/00, A61P 43/00, C07K 14/47, C12N 15/09, C12Q 1/02, C12Q 1/68, G01N 33/15, G01N 33/50

(54) **PREVENTIVES/REMEDIES FOR CANCER**

(30) Priority: 24.02.2005 JP 2005049331
(71) Applicant: Takeda Pharmaceutical Company Limited, Osaka-shi, Osaka 541-0045 (JP)
(72) Inventor: TOJO, Hideaki, ai, Tsukuba-shi, Ibaraki, 3004247 (JP); KAWAMURA, Mihoko, ai, Tsukuba-shi, Ibaraki, 3004247 (JP); SAGIYA, Yoji, ai, Tsukuba-shi, Ibaraki, 3004247 (JP)
(74) Representative: Jones, Nicholas Andrew
(86) International application number: PCT/JP2006/303927
(87) International publication number: WO 2006/090900

(57) **Abstract**

A compound or salt thereof that inhibits the activity of a protein that has an amino acid sequence that is the same or substantially the same as the amino acid sequence shown by SEQ ID NO: 1, a compound or salt thereof that inhibits the expression of the gene for this protein, an antisense polynucleotide that contains a base sequence that is complementary or substantially complementary to the base sequence of a polynucleotide encoding the aforementioned protein or a partial peptide thereof, or that contains a portion of such a base sequence, an antibody against the aforementioned protein, etc., can be used as agents for preventing/treating, for example, cancer, and as agents for promoting cancer cell apoptosis. Protein that has an amino acid sequence that is the same or substantially the same as the amino acid sequence shown by SEQ ID NO: 1, etc., is useful for screening for compounds and salts thereof that act to prevent/treat, for example, cancer.

## Description

### TECHNICAL FIELD

The present invention relates to agents for the prevention and/or treatment of cancer, to diagnostic agent for cancer, and to screening for a prophylactic/therapeutic agent for cancer.

### BACKGROUND ART

In acyl-coenzyme A (CoA), the carboxyl group of a fatty acid is bonded through a thioester bond to the sulfhydryl group of CoA. Through its bonding with CoA, a fatty acid is made available as a substrate for various enzymes and becomes, via reactions such as β-oxidation, desaturation, carbon-chain elongation, acyl group transfer to protein, etc., a precursor for energy production or for the biosynthesis of various endogenous biosubstances that contain fatty acid. The enzymes that synthesize acyl-CoA from fatty acid are collectively known as the acyl-CoA synthetases. The acyl-CoA synthetases include a number of enzymes that exhibit different substrate specificities with regard to the carbon chain length of the fatty acid (Coleman et al., J. Nutr., Volume 132, pp. 2123-2126, 2002; Mashek et al., J. Lipid Res., Volume 45, pp. 1958-1961, 2004).

Very-long chain fatty acyl-CoA synthetase (VLACS) is one of the acyl-CoA synthetases and is an acyl-CoA synthetase that catalyzes the thioester bond-forming reaction between the sulfhydryl group of CoA and the carboxyl group of fatty acids having a C₂₂ or longer carbon chain (very-long chain fatty acid, VLCFA) in the peroxisomes of the liver and kidney. It was firstly purified from rat liver peroxisomes (Uchida et al., J. Biochem., Volume 119, pp. 565-571, 1995). Its amino acid sequence and nucleic acid sequence have also been elucidated by cDNA cloning (Uchiyama et al., J. Biol. Chem., Volume 271, pp. 30360-30365, 1996). Human VLACS has been cloned based on sequence homology with rat VLACS (Steinberg et al., Biochem. Biophys. Res. Commun., Volume 257, pp. 615-621, 1999), and its chromosomal location has also been established (Wakui et al., Cytogenet. Cell Genet., Volume 81, pp. 292-293, 1998). Human VLACS, rat VLACS, and mouse VLACS are all membrane proteins constituted of 620 amino acids, and their expression has been detected in normal tissue in the liver, kidney, and pancreas (Stahl et al., Trends Endocrinol. Metab., Volume 12, pp. 266-273, 2001). VLACS is a protein with the same sequence as FATP2, which is a member of the fatty acid transporter protein (FATP) family, which are separately discovered fatty acid transporters (Hirsch et al., Proc. Natl. Acad. Sci. U.S.A., Volume 95, pp. 8625-8629, 1998). A fatty acid transport activity has been confirmed for mouse FATP2 in a yeast assay system (Li et al., Anal. Biochem., Volume 336, pp. 11-19, 2005). Since the single protein has been reported with two names during the course of this research, its name has been standardized on solute carrier family 27 (fatty acid transporter), member 2 (SLC27A2) at the National Center for Biotechnology Information (NCBI).

With regard to the participation of SLC27A2 in human disease, the suggestion has been made that it may be one of the causative genes in the genetic disease X-linked adrenoleukodystrophy (X-ALD) (Singh et al., Pediatr. Res., Volume 18, pp. 286-290, 1984; Rizzo et al., Neurology, Volume 34, pp. 163-169, 1984). This hypothesis is based on the fact that VLACS activity is reduced in X-ALD patients and on the observation of VLCFA accumulation in patient tissues; however, a genetic mutation in SLC27A related to X-ALD has not been discovered to date.

### DISCLOSURE OF THE INVENTION

There is demand for safe and highly therapeutically effective agents for the prevention and treatment of cancer.

The present inventors carried out extensive and intensive investigations in order to address this issue and as a result discovered SLC27A2 in a group of genes that exhibit an upregulated expression in cancer tissue as compared to normal tissue and that cause an inhibition of cell proliferation when their expression is inhibited in cancer cells. This research was continued and the present invention was achieved as a result.

That is, the present invention provides
(1) A prophylactic/therapeutic agent for cancer, which comprises a compound or its salt inhibiting the activity of a protein comprising the same or substantially the same amino acid sequence as the amino acid sequence represented by SEQ ID NO: 1, its partial peptide, or a salt thereof;
(1a) the agent according to (1), wherein the activity is an acyl-CoA synthetase activity or (and) a fatty acid transport activity;
(2) A prophylactic/therapeutic agent for cancer, which comprises a compound or its salt inhibiting the expression of a gene for a protein comprising the same or substantially the same amino acid sequence as the amino acid sequence represented by SEQ ID NO: 1, or its partial peptide, or a salt thereof;
(3) The prophylactic/therapeutic agent according to (1) or (2), wherein said cancer is a tyrosine kinase signaling pathway inhibitor-resistant cancer;
(4) An antisense polynucleotide comprising the entire or part of a base sequence complementary or substantially complementary to a base sequence of a polynucleotide encoding a protein comprising the same or substantially the same amino acid sequence as the amino acid sequence represented by SEQ ID NO: 1, or its partial peptide;
(5) A pharmaceutical comprising the antisense polynucleotide according to (4);
(6) The pharmaceutical according to (5), which is a prophylactic/therapeutic agent for cancer;
(7) The pharmaceutical according to (6), wherein said cancer is a tyrosine kinase signaling pathway inhibitor-resistant cancer;
(8) A pharmaceutical comprising siRNA or shRNA against a polynucleotide encoding a protein comprising the same or substantially the same amino acid sequence as the amino acid sequence represented by SEQ ID NO: 1, or its partial peptide;
(9) The pharmaceutical according to (8), which is a prophylactic/therapeutic agent for cancer;
(10) The pharmaceutical according to (9), wherein said cancer is a tyrosine kinase signaling pathway inhibitor-resistant cancer;
(11) A prophylactic/therapeutic agent for cancer, which comprises an antibody against a protein comprising the same or substantially the same amino acid sequence as the amino acid sequence represented by SEQ ID NO: 1, or its partial peptide, or a salt thereof;
(12) The pharmaceutical according to (11), wherein said cancer is a tyrosine kinase signaling pathway inhibitor-resistant cancer;
(13) A diagnostic agent for cancer, which comprises an antibody against a protein comprising the same or substantially the same amino acid sequence as the amino acid sequence represented by SEQ ID NO: 1, or its partial peptide, or a salt thereof;
(14) The diagnostic agent according to (13), wherein said cancer is a tyrosine kinase signaling pathway inhibitor-resistant cancer;
(15) A diagnostic agent for cancer, which comprises a polynucleotide encoding a protein comprising the same or substantially the same amino acid sequence as the amino acid sequence represented by SEQ ID NO: 1, or its partial peptide;
(16) The diagnostic agent according to (15), wherein said cancer is a tyrosine kinase signaling pathway inhibitor-resistant cancer;
(17) A method of diagnosing cancer, which comprises using an antibody against a protein comprising the same or substantially the same amino acid sequence as the amino acid sequence represented by SEQ ID NO: 1, its partial peptide, or a salt thereof, or a polynucleotide encoding said protein, or its partial peptide;
(18) The diagnostic method according to (17), wherein said cancer is a tyrosine kinase signaling pathway inhibitor-resistant cancer;
(19) Use of a protein comprising the same or substantially the same amino acid sequence as the amino acid sequence represented by SEQ ID NO: 1, its partial peptide, or a salt thereof, as a diagnostic marker for cancer;
(19a) the use according to (19), wherein the cancer is a tyrosine kinase signaling pathway inhibitor-resistant cancer;
(20) A method of screening a prophylactic/therapeutic agent for cancer, which comprises using a protein comprising the same or substantially the same amino acid sequence as the amino acid sequence represented by SEQ ID NO: 1, its partial peptide, or a salt thereof;
(20a) the screening method according to (20), wherein the drug for the prevention/treatment of cancer is a drug that inhibits the acyl-CoA synthetase activity or (and) fatty acid transport activity of a protein that contains an amino acid sequence that is the same or substantially the same as the amino acid sequence shown by SEQ ID NO: 1, or of a partial peptide of said protein or a salt of said protein;
(21) A kit for screening a prophylactic/therapeutic agent for cancer, comprising a protein comprising the same or substantially the same amino acid sequence as the amino acid sequence represented by SEQ ID NO: 1, its partial peptide, or a salt thereof;
(21a) the screening kit according to (21), wherein the drug for the prevention/treatment of cancer is a drug that inhibits the acyl-CoA synthetase activity or (and) fatty acid transport activity of a protein that contains an amino acid sequence that is the same or substantially the same as the amino acid sequence shown by SEQ ID NO: 1, or of a partial peptide of said protein or a salt of said protein;
(22) A method of screening a prophylactic/therapeutic agent for cancer, which comprises using a polynucleotide encoding a protein comprising the same or substantially the same amino acid sequence as the amino acid sequence represented by SEQ ID NO: 1, or its partial peptide;
(23) A kit for screening a prophylactic/therapeutic agent for cancer, comprising a polynucleotide encoding a protein comprising the same or substantially the same amino acid sequence as the amino acid sequence represented by SEQ ID NO: 1, or its partial peptide;
(24) The method of screening according to (20) or (22), wherein said cancer is a tyrosine kinase signaling pathway inhibitor-resistant cancer;
(25) The kit for screening according to (21) or (23), wherein said cancer is a tyrosine kinase signaling pathway inhibitor-resistant cancer;
(26) A method of screening for a prophylactic/therapeutic agent for cancer, wherein said method comprises measuring the activity of a protein comprising the same or substantially the same amino acid sequence as the amino acid sequence represented by SEQ ID NO: 1, its partial peptide, or a salt thereof;
(27) The method of screening according to (26), wherein said activity is acyl-CoA synthase activity or (and) fatty acid transport activity;
(28) A method of screening for a prophylactic/therapeutic agent for cancer, wherein said method comprises measuring the amount of a protein comprising the same or substantially the same amino acid sequence as the amino acid sequence represented by SEQ ID NO: 1, its partial peptide, or a salt thereof;
(28a) the screening method according to any of (26) to (28), wherein the cancer is a tyrosine kinase signaling pathway inhibitor-resistant cancer;
(29) A method of preventing/treating cancer, which comprises inhibiting the activity of a protein comprising the same or substantially the same amino acid sequence as the amino acid sequence represented by SEQ ID NO: 1, its partial peptide, or a salt thereof, or inhibiting the expression of a gene for said protein, its partial peptide, or a salt thereof;
(29a) the prevention/treatment method according to (29), wherein the activity is acyl-CoA synthetase activity or (and) fatty acid transport activity;
(30) The preventing/treating method according to (29), wherein said cancer is a tyrosine kinase signaling pathway inhibitor-resistant cancer;
(31) A method of preventing/treating cancer, comprising administering to a mammal an effective amount of (i) a compound or its salt that inhibits the activity of a protein comprising the same or substantially the same amino acid sequence as the amino acid sequence represented by SEQ ID NO: 1, its partial peptide, or a salt thereof, (ii) a compound or its salt that inhibits the expression of a gene for said protein, or its partial peptide, or a salt thereof, (iii) an antibody against said protein, or its partial peptide, or a salt thereof, or (iv) an antisense polynucleotide comprising the entire or part of a base sequence complementary or substantially complementary to a base sequence of a polynucleotide encoding said protein, or its partial peptide;
(31a) the prevention/treatment method according to (31), wherein the activity is acyl-CoA synthetase activity or (and) fatty acid transport activity;
(31b) a method of preventing/treating cancer, comprising administering to a mammal an effective amount of siRNA or shRNA against a polynucleotide that encodes a protein that contains an amino acid sequence that is the same or substantially the same as the amino acid sequence shown by SEQ ID NO: 1, or that encodes a partial peptide of said protein;
(32) The preventing/treating method according to (31), wherein said cancer is a tyrosine kinase signaling pathway inhibitor-resistant cancer;
(33) Use of (i) a compound or its salt that inhibits the activity of a protein comprising the same or substantially the same amino acid sequence as the amino acid sequence represented by SEQ ID NO: 1, its partial peptide, or a salt thereof, (ii) a compound or its salt that inhibits the expression of a gene for said protein, or its partial peptide, or a salt thereof, (iii) an antibody against said protein, or its partial peptide, or a salt thereof, or (iv) an antisense polynucleotide comprising the entire or part of a base sequence complementary or substantially complementary to a base sequence of a polynucleotide encoding said protein, or its partial peptide, to manufacture a prophylactic/therapeutic agent for cancer;
(33a) the use according to (33), wherein the activity is acyl-CoA synthetase activity or (and) fatty acid transport activity;
(33b) use of siRNA or shRNA against a polynucleotide that encodes a protein that contains an amino acid sequence that is the same or substantially the same as the amino acid sequence shown by SEQ ID NO: 1, or that encodes a partial peptide of said protein, to produce a cancer preventive/treatment agent;
(34) The use according to (33), wherein said cancer is a tyrosine kinase signaling pathway inhibitor-resistant cancer;
(35) A prophylactic/therapeutic agent for cancer, comprising a compound or its salt that inhibits the activity of a fatty acid transporter protein;
(35a) the agent according to (35), wherein the activity is a fatty acid transport activity;
(36) A prophylactic/therapeutic agent for cancer, comprising a compound or its salt that inhibits the expression of a gene for a fatty acid transporter protein;
(36a) the cancer preventive/treatment agent according to any of (35) to (36), wherein the cancer is a tyrosine kinase signaling pathway inhibitor-resistant cancer;
(37) A method of preventing/treating cancer, which comprises inhibiting the activity of a fatty acid transporter protein, or inhibiting the expression of a gene for a fatty acid transporter protein;
(37a) the method of preventing/treating cancer according to (37), wherein the activity is a fatty acid transport activity;
(37b) the method of preventing/treating cancer according to (37) or (37a), wherein the cancer is a tyrosine kinase signaling pathway inhibitor-resistant cancer;
(37c) use of a compound or salt thereof that inhibits the activity of a fatty acid transporter protein or a compound or salt thereof that inhibits the expression of a gene for a fatty acid transporter protein, to produce a cancer preventive/treatment agent;
(38) A cancer cell apoptosis promoter or a cancer cell proliferation inhibitor, comprising a compound or its salt that inhibits the activity of a protein comprising the same or substantially the same amino acid sequence as the amino acid sequence represented by SEQ ID NO: 1, its partial peptide, or a salt thereof;
(38a) the cancer cell apoptosis promoter or cancer cell proliferation inhibitor according to (38), wherein the activity is acyl-CoA synthetase activity or (and) fatty acid transport activity;
(39) A cancer cell apoptosis promoter or a cancer cell proliferation inhibitor, comprising a compound or its salt that inhibits the expression of a gene for a protein comprising the same or substantially the same amino acid sequence as the amino acid sequence represented by SEQ ID NO: 1, its partial peptide, or a salt thereof;
(40) The pharmaceutical according to (5), which is a cancer cell apoptosis promoter or a cancer cell proliferation inhibitor;
(41) The pharmaceutical according to (8), which is a cancer cell apoptosis promoter or a cancer cell proliferation inhibitor;
(42) A cancer cell apoptosis promoter or a cancer cell proliferation inhibitor, comprising an antibody against a protein comprising the same or substantially the same amino acid sequence as the amino acid sequence represented by SEQ ID NO: 1, or its partial peptide, or a salt thereof;
(42a) the cancer cell apoptosis promoter or cancer cell proliferation inhibitor according to any of (38) to (42), wherein the cancer is a tyrosine kinase signaling pathway inhibitor-resistant cancer;
(43) A method of screening a pharmaceutical for promoting apoptosis of cancer cells or a pharmaceutical for inhibiting proliferation of cancer cells, wherein said method comprises using a protein comprising the same or substantially the same amino acid sequence as the amino acid sequence represented by SEQ ID NO: 1, its partial peptide, or a salt thereof;
(44) A kit for screening a pharmaceutical for promoting apoptosis of cancer cells or a pharmaceutical for inhibiting proliferation of cancer cells, wherein said kit comprises a protein comprising the same or substantially the same amino acid sequence as the amino acid sequence represented by SEQ ID NO: 1, its partial peptide, or a salt thereof;
(45) A method of screening a pharmaceutical for promoting apoptosis of cancer cells or a pharmaceutical for inhibiting proliferation of cancer cells, wherein said method comprises using a polynucleotide encoding a protein comprising the same or substantially the same amino acid sequence as the amino acid sequence represented by SEQ ID NO: 1, or its partial peptide;
(46) A kit for screening a pharmaceutical for promoting apoptosis of cancer cells or a pharmaceutical for inhibiting proliferation of cancer cells, wherein said kit comprises a polynucleotide encoding a protein comprising the same or substantially the same amino acid sequence as the amino acid sequence represented by SEQ ID NO: 1, or its partial peptide;
(47) A method of screening a pharmaceutical for promoting apoptosis of cancer cells or a pharmaceutical for inhibiting proliferation of cancer cells, wherein said method comprises measuring the activity of a protein comprising the same or substantially the same amino acid sequence as the amino acid sequence represented by SEQ ID NO: 1, its partial peptide, or a salt thereof;
(47a) the screening method according to (47), wherein the activity is acyl-CoA synthetase activity or (and) fatty acid transport activity;
(47b) the screening method according to (43), (45), (47), or (47a), wherein the cancer is a tyrosine kinase signaling pathway inhibitor-resistant cancer;
(47c) the screening kit according to (44) or (46), wherein the cancer is a tyrosine kinase signaling pathway inhibitor-resistant cancer;
(48) A method of promoting apoptosis of cancer cells or inhibiting proliferation of cancer cells, wherein said method comprises inhibiting the activity of a protein comprising the same or substantially the same amino acid sequence as the amino acid sequence represented by SEQ ID NO: 1, its partial peptide, or a salt thereof, or inhibiting the expression of a gene for said protein, its partial peptide, or a salt thereof;
(49) A method of promoting apoptosis of cancer cells or inhibiting proliferation of cancer cells, wherein said method comprises administering to a mammal an effective amount of (i) a compound or its salt that inhibits the activity of a protein comprising the same or substantially the same amino acid sequence as the amino acid sequence represented by SEQ ID NO: 1, its partial peptide, or a salt thereof, (ii) a compound or its salt that inhibits the expression of a gene for said protein, or its partial peptide, or a salt thereof, (iii) an antibody against said protein, or its partial peptide, or a salt thereof, or (iv) an antisense polynucleotide comprising the entire or part of a base sequence complementary or substantially complementary to a base sequence of a polynucleotide encoding said protein, or its partial peptide;
(49a) the method according to (49), wherein the activity is acyl-CoA synthetase activity or (and) fatty acid transport activity;
(50) Use of (i) a compound or its salt that inhibits the activity of a protein comprising the same or substantially the same amino acid sequence as the amino acid sequence represented by SEQ ID NO: 1, its partial peptide, or a salt thereof, (ii) a compound or its salt that inhibits the expression of a gene for said protein, or its partial peptide, or a salt thereof, (iii) an antibody against said protein, or its partial peptide, or a salt thereof, or (iv) an antisense polynucleotide comprising the entire or part of a base sequence complementary or substantially complementary to a base sequence of a polynucleotide encoding said protein, or its partial peptide, to manufacture a cancer cell apoptosis promoter or a cancer cell proliferation inhibitor;
(50a) the use according to (50), wherein the activity is acyl-CoA synthetase activity or (and) fatty acid transport activity;
(51) A cancer cell apoptosis promoter or a cancer cell proliferation inhibitor, comprising a compound or its salt that inhibits the activity of a fatty acid transporter protein;
(51a) a cancer cell apoptosis promoter or a cancer cell proliferation inhibitor according to (51), wherein the activity is a fatty acid transport activity;
(52) A cancer cell apoptosis promoter or a cancer cell proliferation inhibitor, comprising a compound or its salt that inhibits the expression of a gene for a fatty acid transporter protein;
(53) A method of promoting apoptosis of cancer cells or inhibiting proliferation of cancer cells, wherein said method comprises inhibiting the activity of a fatty acid transporter protein, or inhibiting the expression of a gene for a fatty acid transporter protein; and
(53a) the method of (53), wherein the activity is a fatty acid transport activity; and (53b) use of a compound or salt thereof that inhibits the activity of a fatty acid transporter protein or a compound or salt thereof that inhibits the expression of a gene for a fatty acid transporter protein, to produce a cancer cell apoptosis promoter or a cancer cell proliferation inhibitor.

A "drug for the prevention/treatment of cancer" may be a substance (for example, a synthetic compound, peptide, protein, antibody, nonpeptide compound, fermentation product, cell extract, plant extract, animal tissue extract, blood plasma, etc.) that itself has the ability to prevent/treat cancer or may be a drug that contains such a substance.

A "drug for promoting cancer cell apoptosis" may be a substance that itself functions to promote cancer cell apoptosis or may be a drug that contains such a substance (for example, a synthetic compound, peptide, protein, antibody, nonpeptide compound, fermentation product, cell extract, plant extract, animal tissue extract, blood plasma, etc.).

A "drug for inhibiting cancer cell proliferation" may be a substance (for example, a synthetic compound, peptide, protein, antibody, nonpeptide compound, fermentation product, cell extract, plant extract, animal tissue extract, blood plasma, etc.) that itself functions to inhibit the proliferation of cancer cells or may be a drug that contains such a substance.

### BRIEF DESCRIPTION OF THE DRAWINGS

Figure 1 shows the inhibition of the proliferation of VMRC-LCD cells by SLC27A2 knockdown. In the figure, the vertical axis shows the viable cell count as a percentage (%) of the control after cultivation for 5 days.
Figure 2 shows apoptosis induction in VMRC-LCD cells due to SLC27A2 knockdown. In the figure, the vertical axis shows DNA fragmentation as a percentage (%) of the control after cultivation for 2 days.
Figure 3 shows the inhibition of cell proliferation induced by the addition of U0126 in (a) NCI-H23 cells, (b) NCI-H522 cells, (c) VMRC-LCD cells, and (d) NCI-H520 cells. In the figure, the vertical axis shows the viable cell count as a percentage (%) of the control after cultivation for 5 days. The horizontal axis shows the concentration (M) of U0126 addition.
Figure 4 shows the relative expression level of SLC27A2 mRNA in NCI-H23 cells, NCI-H522 cells, VMRC-LCD cells, and NCI-H520 cells.
Figure 5 shows the inhibition of proliferation for NCI-H23 cells, NCI-H522 cells, and NCI-H520 cells after transfection with SLC27A2 siRNA. In the figure, the vertical axis shows the viable cell count as a percentage (%) of the control after cultivation for 5 days.

### BEST MODE FOR CARRYING OUT THE INVENTION

The protein comprising the same or substantially the same as the amino acid sequence as the amino acid sequence represented by SEQ ID NO: 1 (this protein is referred to below as the protein of the present invention or the protein used by the present invention) may be a protein derived from human or warm-blooded animal (for example, guinea pig, rat, mouse, chicken, rabbit, pig, sheep, cow, monkey, etc.) cells (for example, retinal cells, hepatocytes, splenocytes, nerve cells, glial cells, pancreatic β-cells, bone marrow cells, mesangial cells, Langerhans cells, epidermal cells, epithelial cells, endothelial cells, fibroblasts, fibrocytes, muscle cells, fat cells, immune cells (for example, macrophages, T-cells, B-cells, natural killer cells, mast cells, neutrophils, basophils, eosinophils, monocytes, platelets, etc.), megakaryocytes, synoviocytes, chondrocytes, osteocytes, osteoblasts, osteoclasts, mammary cells, hepatocytes, or interstitial cells, or stem cells or precursor cells for the preceding cells, or cancer cells, etc.) or from any tissue in which such cells are present, for example, brain and individual regions thereof (for example, retina, olfactory bulb, amygdaloid nucleus, basal ganglia, hippocampus, thalamus, hypothalamus, cerebral cortex, medulla oblongata, cerebellum), spinal cord, pituitary gland, stomach, pancreas, kidney, liver, reproductive glands, thyroid gland, gall bladder, bone marrow, adrenal gland, skin, muscle, lung, digestive tract (for example, colon, small intestine), blood vessels, heart, thymus gland, spleen, submandibular glands, peripheral blood, prostate gland, testicles, ovaries, placenta, uterus, bone, joints, skeletal muscle, etc., or from a hemocyte-type cell or cultured cell line thereof (for example, MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.), or may be a synthetic protein.

An amino acid sequence that is substantially the same as the amino acid sequence represented by SEQ ID NO: 1 is, for example, an amino acid sequence having at least an approximately 50% homology, preferably at least an approximately 60% homology, more preferably at least an approximately 70% homology, even more preferably at least an approximately 80% homology, particularly preferably at least an approximately 90% homology, and most preferably at least an approximately 95% homology with the amino acid sequence shown by SEQ ID NO: 1.

Homology of the amino acid sequences can be calculated using the NCBI BLAST (National Center for Biotechnology Information, Basic Local Alignment Search Tool) homology computation algorithm under the following conditions (expect value = 10, gaps are allowed, matrix = BLOSUM62, filtering = OFF).

Protein comprising substantially the same amino acid sequence as the amino acid sequence represented by SEQ ID NO: 1 is preferably, for example, protein comprising substantially the same amino acid sequence as the amino acid sequence shown by SEQ ID NO: 1 and having activity of substantially the same nature as protein comprising the amino acid sequence shown by SEQ ID NO: 1.

The activity of substantially the same nature is, for example, acyl-CoA synthetase activity, fatty acid transport activity, etc.. "Substantially the same nature" indicates that these properties are of substantially the same nature qualitatively (for example, physiologically or pharmacologically). Accordingly, while, for example, the acyl-CoA synthetase activity or fatty acid transport activity is preferably the same (for example, approximately 0.01- to 100-fold, preferably approximately 0.1- to 10-fold, and more preferably 0.5- to 2-fold), the quantitative features, such as the degree of this activity, the molecular weight of the protein, etc., may differ.

Measurement of the acyl-CoA synthetase can be carried out according to methods that are known as such, for example, according to the method described in J. Biol. Chem., Volume 256, pp. 5702-5707, 1981, or methods based thereon. In specific terms, the protein of the present invention is reacted for 10 minutes at 35°C in 0.5 mL of a solution that contains 0.2 mM Tris-HCI buffer (pH 7.5), 2.5 mM ATP, 8 mM MgCl₂, 2 mM EDTA, 20 mM NaF, 0.1% (w/v) Triton X-100, 10 µM [1-¹⁴C]palmitic acid (5 µCi/µmol), and 0.5 mM coenzyme A. The reaction is started by addition of the CoA and is stopped by the addition of 2.5 mL isopropanol : n-heptane : 1 M sulfuric acid (40 : 10 : 1, v/v). After the reaction has been stopped, 0.5 mL water and 2.5 mL n-heptane are added; the organic solvent phase containing the unreacted fatty acid is removed; the aqueous phase is washed 3 times with 2.5 mL n-heptane; and the radioactivity remaining in the aqueous phase is measured with a scintillation counter.

Measurement of the fatty acid transport activity can be carried out according to methods that are known as such, for example, according to the method described in Anal. Biochem., Volume 336, pp. 11-19, 2005, or methods based thereon. In specific terms, the protein of the present invention is introduced into a mutant yeast *(Saccharomyces cerevisiae)* strain deficient in fat 1, a lipid transporter gene, followed by incubation. The incubated yeast is washed with phosphate buffer solution (PBS) and then suspended in PBS to give a cell density of 6 × 10⁷/mL. To this is then added C₁-BODIPY-C₁₂ (4,4-difluoro-5-methyl-4-bora-3a,4a-diaza-*s*-indacene-3-dodecanoic acid) to a final concentration of 2.5 µM and fatty acid-free bovine serum albumin (BSA) to a final concentration of 7.5 µM followed by standing in the dark at room temperature for 3 minutes. The fluorescence of the C₁-BODIPY-C₁₂ not taken up by the yeast is quenched by trypan blue at a final concentration of 0.33 mM, and the fluorescence intensity emitted by the C₁-BODIPY-C₁₂ taken into the yeast cells is measured with a fluorescence plate reader.

The protein of the present invention includes, for example, so called muteins, such as proteins comprising (1) (i) the amino acid sequence represented by SEQ ID NO: 1, wherein at least 1 or 2 amino acids (for example approximately 1 to 100 amino acids, preferably approximately 1 to 30 amino acids, more preferably approximately 1 to 10 amino acids and most preferably several (1 to 5) amino acids) are deleted, (ii) the amino acid sequence represented by SEQ ID NO: 1, to which at least 1 or 2 amino acids (for example approximately 1 to 100 amino acids, preferably approximately 1 to 30 amino acids, more preferably approximately 1 to 10 amino acids and most preferably several (1 to 5) amino acids) are added, (iii) the amino acid sequence represented by SEQ ID NO: 1, into which at least 1 or 2 amino acids (for example approximately 1 to 100 amino acids, preferably approximately 1 to 30 amino acids, more preferably approximately 1 to 10 amino acids and most preferably several (1 to 5) amino acids) are inserted, (iv) the amino acid sequence represented by SEQ ID NO: 1, wherein at least 1 or 2 amino acids (for example approximately 1 to 100 amino acids, preferably approximately 1 to 30 amino acids, more preferably approximately 1 to 10 amino acids and most preferably several (1 to 5) amino acids) are substituted by other amino acids or (v) an amino acid sequence which is a combination of these sequences.

There are no particular limitations on the position or positions of insertion, deletion, or substitution in the case of the aforementioned amino acid sequences subjected to insertion, deletion, or substitution.

The proteins in the present specification are represented in accordance with the conventional way of describing peptides, that is, the N-terminus (amino terminus) at the left hand and the C-terminus (carboxyl terminus) at the right hand. In the proteins of the present invention including the protein comprising the amino acid sequence represented by SEQ ID NO: 1, the C-terminus may be either a carboxyl group (-COOH), a carboxylate (-COO⁻), an amide (-CONH₂) or an ester (-COOR).

The R in this ester can be, for example, a C₁₋₆ alkyl group such as methyl, ethyl, n-propyl, isopropyl, or n-butyl; a C₃₋₈ cycloalkyl group such as cyclopentyl or cyclohexyl; a C₆₋₁₂ aryl group such as phenyl or α-naphthyl; a C₇₋₁₄ aralkyl group such as a phenyl-C₁₋₂ alkyl group for example, benzyl and phenethyl, or an α-naphthyl-C₁₋₂ alkyl group, for example, α-naphthylmethyl; or the pivaloyloxymethyl group.

When the protein used by the present invention has a carboxyl group (or carboxylate) at a position other than the C-terminus, the presence of this carboxyl group in the form of the amide or ester is also encompassed by the protein used by the present invention. The ester in such a case may be exemplified by the esters cited for the C-terminus hereinabove.

The protein used by the present invention also encompasses protein in which the amino group of the N-terminus amino acid residue (for example, a methionine residue) is protected by a protecting group (for example, a C₁₋₆ acyl group such as C₁₋₆ alkanoyl, for example, the formyl group or acetyl group); protein in which the N-terminus glutamine residue produced by in vivo cleavage is pyroglutaminated; protein in which an amino acid side-chain substituent (for example, -OH, -SH, the amino group, the imidazole group, the indole group, the guanidino group) in the molecule is protected by a suitable protecting group (for example, a C₁₋₆ acyl group such as C₁₋₆ alkanoyl, for example, the formyl group or acetyl group); and conjugated protein, for example, a so-called glycoprotein having an attached sugar chain.

The protein used by the present invention can be specifically exemplified by protein comprising the amino acid sequence shown by SEQ ID NO: 1, protein comprising the amino acid sequence shown by SEQ ID NO: 3, and protein comprising the amino acid sequence shown by SEQ ID NO: 5.

A partial peptide of the protein used by the present invention is a partial peptide of the hereinabove-described protein used by the present invention and preferably may be any partial peptide that has properties equivalent to those of the hereinabove-described protein used by the present invention.

For example, a peptide can be used that has an at least 20-residue amino acid sequence, preferably an at least 50-residue amino acid sequence, more preferably an at least 70-residue amino acid sequence, even more preferably an at least 100-residue amino acid sequence, and most preferably an at least 200-residue amino acid sequence of the constituent amino acid sequence of the protein used by the present invention.

The partial peptide of the present invention includes partial peptides of the amino acid sequence described above, wherein 1 or more amino acids (preferably about 1 to 20 amino acids, more preferably about 1 to 10 amino acids, still more preferably several (1 to 5) amino acids) may be deleted; to which 1 or more amino acids (preferably about 1 to 20 amino acids, more preferably about 1 to 10 amino acids, still more preferably several (1 to 5) amino acids) may be added; into which 1 or more amino acids (preferably about 1 to 20 amino acids, more preferably 1 to 10 amino acids, still more preferably about 1 to 5 amino acids) may be inserted; or in which 1 or more amino acids ((preferably about 1 to 20 amino acids, more preferably about 1 to 10 amino acids, still more preferably several (1 to 5) amino acids) may be substituted by other amino acids.

Moreover, the C-terminus of the partial peptide used by the present invention may be the carboxyl group (-COOH), a carboxylate (-COO⁻), an amide (-CONH₂), or an ester (-COOR).

Furthermore, the partial peptide used by the present invention also encompasses, just as for the above-described protein used by the present invention, partial peptide having the carboxyl group (or carboxylate) at a position other than the C-terminus, partial peptide in which the amino group of the N-terminus amino acid residue (for example, a methionine residue) is protected by a protecting group; partial peptide in which a glutamine residue produced by in vivo cleavage on the N-terminus side is pyroglutaminated; partial peptide in which an amino acid side-chain substituent in the molecule is protected by a protecting group; and conjugated peptide, for example, a so-called glycopeptide having an attached sugar chain.

The partial peptide used by the present invention may also be used as an antigen for antibody production.

Physiologically acceptable salts with acids (for example, inorganic acids, organic acids) or bases (for example, alkali metal salts) can be used as the salt of the protein or partial peptide used by the present invention, and physiologically acceptable acid-addition salts are particularly preferred. Usable as such salts are, for example, salts with an inorganic acid (for example, hydrochloric acid, phosphoric acid, hydrobromic acid, sulfuric acid) and salts with an organic acid (for example, acetic acid, formic acid, propionic acid, fumaric acid, maleic acid, succinic acid, tartaric acid, citric acid, malic acid, oxalic acid, benzoic acid, methanesulfonic acid, and benzenesulfonic acid).

The protein, partial peptide thereof, or its salt used by the present invention can be prepared by protein purification procedures that are known as such, from the above described human or warm-blooded animal cells or tissues, and can also be prepared by incubation of a transformant that contains DNA that encodes the protein. Production can also be carried out based on the peptide synthesis procedures described below.

In the case of production from human or mammal tissue or cells, the human or mammal tissue or cells may be homogenized and then extracted with, for example, an acid, and purification and isolation may be carried out on the extract using a combination of chromatographic techniques such as reverse-phase chromatography, ion-exchange chromatography, etc..

The usual commercially available resins for protein synthesis can be used for the synthesis of the protein, its partial peptide, its salt, or amide form thereof used by the present invention. Such resins can be exemplified by chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethyl-phenylacetamidomethyl resin, polyacrylamide resin, 4-(2',4'-dimethoxyphenyl-hydroxymethyl)phenoxy resin, and 4-(2',4'-dimethoxyphenyl-Fmoc-aminoethyl)phenoxy resin. Using these resins, amino acids having an appropriately protected α-amino group and an appropriately protected side-chain functional group are condensed on the resin, using any of various condensation procedures that are known as such, in accordance with and over the sequence of the target protein. The protein or partial peptide is cleaved from the resin after the final reaction, and at the same time the various protecting groups are removed. A reaction that generates the intramolecular disulfide bonding is then carried out in a highly dilute solution, thereby yielding the target protein or partial peptide or amide thereof.

While various activating reagents usable for protein synthesis can be used for condensation of the aforementioned protected amino acids, carbodiimides are particularly preferred. For example, DCC, N,N'-diisopropylcarbodiimide, N-ethyl-N'-(3-dimethylaminopropyl)carbodiimide, etc. can be used as the carbodiimide. For activation by these reagents, a racemization-inhibiting additive (for example, HOBt, HOOBt) and the protected amino acid can be added directly to the resin, or the protected amino acid can be preliminarily activated as the symmetric acid anhydride or HOBt ester or HOOBt ester and this can thereafter be added to the resin.

The solvent used for activation of the protected amino acid and for condensation with the resin can be selected as appropriate from those solvents known to be applicable for protein condensation reactions. For example, acid amides such as N,N-dimethylformamide, N,N-dimethylacetamide, and N-methylpyrrolidone; halogenated hydrocarbons such as methylene chloride and chloroform; alcohols such as trifluoroethanol; sulfoxides such as dimethyl sulfoxide; pyridine; ethers such as dioxane and tetrahydrofuran; nitriles such as acetonitrile and propionitrile; esters such as methyl acetate and ethyl acetate; and suitable mixtures of the preceding can be used. The reaction temperature is selected as appropriate from the range known to be applicable for peptide bond-forming reactions and in general an appropriate selection is made from the range of approximately -20°C to 50°C. The activated amino acid derivatives are generally used in a 1.5- to 4-fold excess. When the result of testing using the ninhydrin reaction indicates an inadequate condensation, an adequate condensation can be achieved by repeating the condensation reaction without removing the protecting group. When an adequate condensation is not obtained even when the reaction has been repeated, the unreacted amino acid can be acetylated with acetic anhydride or acetylimidazole in order to prevent it from influencing subsequent reactions.

The protecting group used for the amino group in the starting material can be, for example, Z, Boc, t-pentyloxycarbonyl, isobornyloxycarbonyl, 4-methoxybenzyloxycarbonyl, Cl-Z, Br-Z, admantyloxycarbonyl, trifluoroacetyl, phthaloyl, formyl, 2-nitrophenylsulfenyl, diphenylphosphinothioyl, Fmoc, etc.

The carboxyl group can be protected by, for example, alkyl esterification (for example, straight-chain, branched, or cyclic alkyl esterification with methyl, ethyl, propyl, butyl, t-butyl, cyclopentyl, cyclohexyl, cycloheptyl, cyclooctyl, 2-adamantyl, etc.), aralkyl esterification (for example, the benzyl ester, 4-nitrobenzyl ester, 4-methoxybenzyl ester, 4-chlorobenzyl ester, benzhydryl esterification), phenacyl esterification, benzyloxycarbonyl hydrazidation, t-butoxycarbonyl hydrazidation, trityl hydrazidation, etc.

The hydroxyl group of serine can be protected by, for example, esterification or etherification. Groups appropriate for this esterification are, for example, lower (C₁₋₆) alkanoyl groups such as acetyl, aroyl groups such as benzoyl, and groups derived from carbonic acid such as benzyloxycarbonyl and ethoxycarbonyl. Groups appropriate for etherification are, for example, benzyl, tetrahydropyranyl, and t-butyl.

For example, Bzl, Cl₂-Bzl, 2-nitrobenzyl, Br-Z, t-butyl, etc. can be used as the protecting group for the phenolic hydroxyl group of tyrosine.

The protecting group for the imidazole of histidine can be, for example, Tos, 4-methoxy-2,3,6-trimethylbenzenesulfonyl, DNP, benzyloxymethyl, Bum, Boc, Trt, Fmoc, etc.

Species in which the carboxyl group of the starting material has been activated can be exemplified by the corresponding acid anhydride, azide, activated ester (ester with an alcohol (for example, pentachlorophenol, 2,4,5-trichlorophenol, 2,4-dinitrophenol, cyanomethyl alcohol, para-nitrophenol, HONB, N-hydroxysuccimide, N-hydroxyphthalimide, HOBt)). Species in which the amino group of the starting material has been activated can be exemplified by the corresponding phosphoramide.

The method used to remove (eliminate) the protecting group can be exemplified by catalytic reduction in a hydrogen current in the presence of a catalyst such as Pd black or Pd/carbon; treatment with an acid such as anhydrous hydrofluoric acid, methanesulfonic acid, trifluoromethanesulfonic acid, trifluoroacetic acid, or a mixture thereof; treatment with a base such as diisopropylethylamine, triethylamine, piperidine, piperazine, etc.; and reduction with sodium in liquid ammonia. The elimination reaction by the aforementioned acid treatment is generally carried out at a temperature of approximately -20°C to 40°C, and the addition of a cation scavenger, such as anisole, phenol, thioanisole, m-cresol, p-cresol, dimethyl sulfide, 1,4-butanedithiol, or 1,2-ethanedithiol, to the acid treatment is useful. In addition, the 2,4-dinitrophenyl group used as an imidazole protecting group for histidine may be removed by treatment with thiophenol, while the formyl group used as an indole protecting group with tryptophan, in addition to deprotection by acid treatment in the presence of the aforementioned 1,2-ethanediol or 1,4-butanediol, may also be removed by treatment with a base such as a dilute sodium hydroxide solution or dilute ammonia.

Suitable selections from known groups and known procedures can be used for the protection of functional groups that are not to participate in the reaction of the starting material, for the protecting groups used in this protection, for the elimination of these protecting groups, for activation of the functional groups that are to participate in the reaction, etc.

As an example of another method for obtaining the amide of the protein or partial peptide, the α-carboxyl group of the carboxy-terminus amino acid is first protected by conversion into the amide, the peptide (protein) chain is then extended on the amino group side to the desired chain length, and a protein or partial peptide is then prepared in which only the protecting group on the N-terminus α-amino group of this peptide chain has been removed; a protein or partial peptide is also similarly prepared in which only the protecting group on the C-terminus carboxyl group has been removed; and these proteins or peptides are condensed with each other in a mixed solvent as described above. The details of the condensation reaction are the same as already described above. After purification of the protected protein or peptide yielded by the condensation, all of the protecting groups are removed using the methods already described above to give the desired protein or peptide in crude form. This crude protein or peptide can be purified using any of the various known purification procedures, and the desired protein or peptide in the form of its amide can then be obtained by freeze-drying the pertinent fraction or fractions.

To obtain the ester form of the protein or peptide, for example, the amino acid ester is obtained by condensing the α-carboxyl group of the carboxy-terminus amino acid with a desired alcohol, and the desired protein or peptide in the ester form can then be obtained by proceeding in the same manner as for the amide form of the protein or peptide.

The partial peptide used by the present invention or its salt can be produced in accordance with peptide synthesis procedures that are known as such, or by cleaving the protein used by the present invention with a suitable peptidase. The peptide synthesis procedure may be, for example, a solid-phase procedure or a liquid-phase procedure. That is, the target peptide can be produced by condensing the residual moiety with amino acid(s) or partial peptide that can constitute the partial peptide used by the present invention and, when the product contains protecting groups, eliminating the protecting groups. The methods described in (i) to (v) below can be given as examples of the known condensation procedures and known protecting group elimination.
(i) M. Bodanszky and M. A. Ondetti: Peptide Synthesis, Interscience Publishers, New York (1966)
(ii) Schroeder and Luebke: The Peptide, Academic Press, New York (1965)
(iii) Nobuo Izumiya, et al.: Peptide Synthesis: Basics and Experiments, Maruzen Co. (1975)
(iv) Haruaki Yajima and Shunpei Sakakibara: Biochemical Experiments 1, Chemistry of Proteins IV, 205 (1977)
(v) Haruaki Yajima ed.: Drug Development Series, Volume 14, Peptide Synthesis, published by Hirokawa Shoten

After the reaction, the partial peptide used by the present invention can be purified and isolated through a combination of the usual purification techniques, such as, for example, solvent extraction, distillation, column chromatography, liquid chromatography, recrystallization, etc. When the partial peptide obtained by the methods described above is in free form, it can be converted to a suitable salt by known techniques or techniques based thereon. Conversely, when it is obtained in salt form, it can be converted into the free form or into another salt by known techniques or techniques based thereon.

The polynucleotide that encodes protein used by the present invention may be any polynucleotide comprising a base sequence that encodes the above-described protein used by the present invention. It is preferably DNA. This DNA may be any selection from genomic DNA, genomic DNA libraries, cDNA derived from the cells and/or tissues described above, cDNA libraries derived from the cells and/or tissues described above, and synthetic DNA.

The vector used for the libraries may be any selection from bacteriophages, plasmids, cosmids, phagemids, etc. Amplification may also be carried out by the direct reverse transcription polymerase chain reaction (abbreviated below as RT-PCR) using an mRNA fraction or the total RNA prepared from the cells and/or tissues described above.

DNA that encodes protein used by the present invention may be, for example, any selection from DNA comprising the base sequence shown by SEQ ID NO: 2 and DNA comprising a base sequence that hybridizes under high-stringency conditions with the base sequence shown by SEQ ID NO: 2 and encoding a protein having properties of substantially the same nature as protein containing the amino acid sequence shown by SEQ ID NO: 1 as described above.

For example, DNA comprising a base sequence having approximately at least 50%, preferably approximately at least 60%, more preferably approximately at least 70%, even more preferably approximately at least 80%, particularly preferably approximately at least 90%, and most preferably approximately at least 95% homology with the base sequence shown by SEQ ID NO: 2 can be used as the DNA that hybridizes under highly stringent conditions with the base sequence shown by SEQ ID NO: 2.

The homology among base sequences can be calculated using homology calculation algorism NCBI BLAST (National Center for Biotechnology Information Basic Local Alignment Search Tool) under the following conditions: expected value = 10; gap is allowable; filtering = ON; match score =1; mismatch score = -3.

Hybridization can be carried out according to publicly known methods or methods based thereon, for example, the procedures described in Molecular Cloning, 2nd (J. Sambrook et al., Cold Spring Harbor Lab. Press, 1989). When a commercially obtained library is used, hybridization can be carried out according to the procedures described in the accompanying instructions for use. Preferably, the hybridization can be carried out under highly stringent conditions.

Highly stringent conditions refer, for example, to a sodium concentration of approximately 19 to 40 mM and preferably approximately 19 to 20 mM and a temperature of approximately 50 to 70°C and preferably approximately 60 to 65°C. As a particular matter, a sodium concentration of approximately 19 mM and a temperature of approximately 65°C are most preferred.

More specifically, DNA comprising the base sequence shown by SEQ ID NO: 2 can be used as DNA that encodes protein comprising the amino acid sequence shown by SEQ ID NO: 1; DNA comprising the base sequence shown by SEQ ID NO: 4 can be used as DNA that encodes protein comprising the amino acid sequence shown by SEQ ID NO: 3; and DNA comprising the base sequence shown by SEQ ID NO: 6 can be used as DNA that encodes protein comprising the amino acid sequence shown by SEQ ID NO: 5.

DNA encoding the partial peptide used by the present invention may be any DNA comprising a base sequence encoding the above-described partial peptide used by the present invention. In addition, it may be any selection from genomic DNA, genomic DNA libraries, cDNA derived from the cells and/or tissues described above, cDNA libraries derived from the cells and/or tissues described above, and synthetic DNA.

DNA encoding the partial peptide used by the present invention may be, for example, DNA having a portion of a DNA comprising the base sequence shown by SEQ ID NO: 2 or DNA comprising a portion of a DNA comprising a base sequence that hybridizes under high-stringency conditions with the base sequence shown by SEQ ID NO: 2 and encoding a protein having an activity of substantially the same nature as protein according to the present invention.

DNA hybridizable with the base sequence shown by SEQ ID NO: 2 has the same meaning as above.

The hybridization procedure and highly stringent conditions used here are the same as described above.

As means for cloning DNA that completely encodes the protein or partial peptide used by the present invention (in the following description of the cloning and expression of the DNA that encodes the protein or partial peptide, the description proceeds in some stances with reference to only the protein of the present invention for the sake of simplicity), selection can be carried out by amplification by PCR using synthetic DNA primers that have a portion of the base sequence that encodes the protein of the present invention, or by hybridization of the DNA incorporated in a suitable vector with labeled synthetic DNA or a DNA fragment that encodes a portion or the complete region of the protein of the present invention. The hybridization procedure can be carried out, for example, according to the procedures described in Molecular Cloning, 2nd (J. Sambrook et al., Cold Spring Harbor Lab. Press, 1989). When a commercially obtained library is used, hybridization can be carried out according to the procedures described in the accompanying instructions for use.

Modification of the base sequence of the DNA can be carried out by publicly known methods, for example, the ODA-LA PCR method, the gapped duplex method, the Kunkel method, etc., or by methods based thereon, using PCR or a known kit, for example, Mutan^{™}-Super Express Km (Takara Shuzo Co., Ltd.), Mutan^{™}-K (Takara Shuzo Co., Ltd.), etc.

The cloned protein-encoding DNA can, depending on the goal, be used as such or as desired after digestion with a restriction enzyme and/or attachment of a linker. This DNA can have ATG as a translation initiation codon on its 5' end side and can have TAA, TGA, or TAG as a translation termination codon on its 3' end side. These translation initiation and translation termination codons can also be added using an appropriate synthetic DNA adapter.

The expression vector for the protein of the present invention can be constructed by (i) excising the target DNA fragment from DNA that encodes protein of the present invention and (ii) ligating this DNA fragment downstream from a promoter in a suitable expression vector.

The following, for example, can be used as the vector: plasmids derived from *E. coli* (for example, pBR322, pBR325, pUC12, pUC13); plasmids derived from *Bacillus subtilis* (for example, pUB110, pTP5, pC194); plasmids derived from yeast (for example, pSH19, pSH15); bacteriophages such as λ-phage; animal viruses such as retroviruses, vaccinia viruses, and baculoviruses; as well as pA1-11, pXT1, pRc/CMV, pRc/RSV, pcDNAI/Neo.

The promoter used by the present invention may be any promoter suitable for the host used for gene expression. In the case of animal cell hosts, examples are the SRα promoter, SV40 promoter, LTR promoter, CMV promoter, and HSV-TK promoter.

Preferred thereamong is the use of, for example, the CMV (cytomegalovirus) promoter and SRα promoter. When the host is a bacterium from genus *Escherichia,* the trp promoter, lac promoter, recA promoter, λP_{L} promoter, lpp promoter, T7 promoter, etc., are preferred; when the host is a bacterium from genus *Bacillus,* the SPO1 promoter, SP02 promoter, penP promoter, etc., are preferred; and when the host is a yeast, the PHO5 promoter, PGK promoter, GAP promoter, ADH promoter, etc., are preferred. When the host is an insect cell, the polyhedrin promoter, P 10 promoter, etc., are preferred.

The expression vector may also contain, in addition to the preceding, the following as desired: an enhancer, splicing signal, polyA addition signal, selection marker, SV40 origin of replication (in some instances abbreviated below as SV40ori), etc. The selection marker can be exemplified by the dihydrofolate reductase (hereinafter abbreviated as dhfr in some instances) gene (methotrexate (MTX) resistance), the ampicillin resistance gene (hereinafter abbreviated as Amp^{r} in some instances), and the neomycin resistance gene (hereinafter abbreviated as Neo^{r} in some instances, G418 resistance). In particular, the target gene can also be selected by means of a thymidine-free medium when using dhfr gene-deficient Chinese hamster cells and employing the dhfr gene as the selection marker.

As necessary, a host-compatible signal sequence may also be added to the N-terminus side of protein of the present invention. Examples of applicable signal sequences are as follows: the PhoA signal sequence, OmpA signal sequence, etc., when the host is a bacterium from genus *Escherichia;* the α-amylase signal sequence, subtilisin signal sequence, etc., when the host is a bacterium from genus *Bacillus;* the MFα signal sequence, SUC2 signal sequence, etc., when the host is a yeast; and the insulin signal sequence, α-interferon signal sequence, antibody molecule signal sequence, etc., when the host is an animal cell.

A transformant can be prepared using the thusly constructed vector containing DNA that encodes protein of the present invention.

Hosts such as, for example, bacteria in genus *Escherichia,* bacteria in genus *Bacillus,* yeast, insect cells, insects, animal cells, etc., can be used.

Specific examples of bacteria in genus *Escherichia* include *Escherichia coli* K12 · DH1 (Proc. Natl. Acad. Sci. USA, Volume 60, 160 (1968)), JM103 (Nucleic Acids Research, Volume 9, 309 (1981)), JA221 (Journal of Molecular Biology, Volume 120, 517 (1978)), HB 101 (Journal of Molecular Biology, Volume 41, 459 (1969)), and C600 *(*Genetics, Volume 39, 440 (1954)).

For example, *Bacillus subtilis* MI114 (Gene, Volume 24, 255 (1983)), 207-21 (Journal of Biochemistry, Volume 95, 87 (1984)), etc., can be used as bacteria in genus *Bacillus.*

Usable yeast can be exemplified by *Saccharomyces cerevisiae* AH22, AH22R, NA87-11A, DKD-5D, and 20B-12; *Schizosaccharomyces pombe* NCYC1913 and NCYC2036; *Pichia pastoris* KM71; etc.

Usable insect cells can be exemplified as follows: for the AcNPV virus, cell lines derived from the larva of *Spodoptera frugiperda* (Spodoptera frugiperda cells, Sf cells), MG1 cells derived from the mid-gut of *Trichoplusia ni,* High Five^{™} cells derived from the egg of *Trichoplusia ni,* cells derived from *Mamestra brassicae,* cells derived from *Estigmena acrea,* etc.; and for the BmNPV virus, cell lines derived from *Bombyx mori* (Bombyx mori N cells, BmN cells) etc. Usable Sf cells can be exemplified by Sf9 cells (ATCC CRL1711), Sf21 cells (both are described in Vaughn, J. L. et al., In Vivo, 13, 213-217 (1977)), etc.

Usable insects can be exemplified by the larva *of Bombyx mori* (Maeda et al., Nature, Volume 315, 592 (1985)) etc.

Applicable animal cells can be exemplified by the monkey cell COS-7, Vero, Chinese hamster cells CHO (abbreviated below as CHO cells), dhfr gene-deficient Chinese hamster cells CHO (abbreviated below as CHO (dhfr⁻) cells), mouse L cells, mouse AtT-20, mouse myeloma cells, mouse ATDC5 cells, rat GH3, human FL cells, etc.

The transformation of bacteria in genus *Escherichia coli* can be carried out, for example, according to the methods described in Proc. Natl. Acad. Sci. USA, Volume 69, 2110 (1972), Gene, Volume 17, 107 (1982), etc.

The transformation of bacteria in genus *Bacillus* can be carried out, for example, according to the method described in Molecular & General Genetics, Volume 168, 111 (1979) etc.

The transformation of yeast can be carried out, for example, according to the methods described *in* Methods in Enzymology, Volume 194, 182-187 (1991), Proc. Natl. Acad. Sci. USA, Volume 75, 1929 (1978), etc.

The transformation of insect cells and insects can be carried out, for example, according to the method described in Bio/Technology, 6, 47-55 (1988), etc.

The transformation of animal cells can be carried out, for example, according to the methods described in Cell Engineering, Supplement 8, New Cell Engineering Experimental Protocols, 263-267 (1995) (published by Shujunsha Co., Ltd.) and Virology, Volume 52, 456 (1973).

Proceeding in this manner enables the acquisition of a transformant that has been transformed with an expression vector containing the protein-encoding DNA.

When the host for the transformant is a bacterium from genus *Escherichia* or *Bacillus,* the medium used for culture of the transformant is suitably a liquid medium that contains, inter alia, a carbon source, nitrogen source, and inorganic substances as required for the growth of the particular transformant. The carbon source can be exemplified by glucose, dextrin, soluble starch, sucrose, etc.; the nitrogen source can be exemplified by inorganic and organic nitrogen sources such as ammonium salts, nitrate salts, corn steep liquor, peptone, casein, meat extract, soybean cake, potato extract, etc.; and the inorganic substances can be exemplified by calcium chloride, sodium dihydrogenphosphate, magnesium chloride, etc. Yeast extract, vitamins, growth promoting factors, etc., may also be added. The pH of the medium is desirably approximately 5 to 8.

M9 medium containing glucose and casamino acids (Miller, Journal of Experiments in Molecular Genetics, 431-433, Cold Spring Harbor Laboratory, New York, 1972) is a preferred example of a medium for the cultivation of *Escherichia* species. If necessary, a chemical such as, for example, 3 β-indolylacrylic acid, can be added in order to induce highly efficient promoter activity.

When the host is an *Escherichia* species, cultivation is generally carried out at approximately 15 to 43°C for approximately 3 to 24 hours, and aeration and/or stirring may also be added as necessary.

When the host is a *Bacillus* species, cultivation is generally carried out at approximately 30 to 40°C for approximately 6 to 24 hours, and aeration and/or stirring may also be added as necessary.

For the cultivation of a transformant for which the host is a yeast, the medium can be exemplified by Burkholder's minimal medium (Bostian, K. L. et al., Proc. Natl. Acad. Sci. USA, Volume 77, 4505 (1980)) and SD medium containing 0.5% casamino acids (Bitter, G. A. et al., Proc. Natl. Acad. Sci. USA, Volume 81, 5330 (1984)). The pH of the medium is preferably adjusted to approximately 5 to 8. Cultivation is generally carried out at approximately 20 to 35°C for approximately 24 to 72 hours, and aeration and/or stirring may be added as necessary.

For the cultivation of a transformant for which the host is an insect or insect cell, the medium can be, for example, Grace's Insect Medium (Grace, T. C. C., Nature, 195, 788 (1962)) to which an additive such as immobilized 10% bovine serum has been added as appropriate. The pH of the medium is preferably adjusted to approximately 6.2 to 6.4. Cultivation is generally carried out at approximately 27°C for approximately 3 to 5 days, and aeration and/or stirring may be added as necessary.

For the cultivation of a transformant for which the host is an animal cell, usable media can be exemplified by MEM medium containing approximately 5 to 20% fetal bovine serum *(*Science, Volume 122, 501 (1952)), DMEM medium (Virology, Volume 8, 396 (1959)), RPMI 1640 medium (The Journal of the American Medical Association, Volume 199, 519 (1967)), 199 medium *(*Proceedings of the Society for the Biological Medicine, Volume 73, 1 (1950)), etc. The pH is preferably approximately 6 to 8. Cultivation is generally carried out at approximately 30 to 40°C for approximately 15 to 60 hours, and aeration and/or stirring may be added as necessary.

Proceeding as described above, the protein of the present invention can be produced within the cells of the transformant, in the cell membrane of the transformant, or outside the transformant cells.

The protein of the present invention can be separated and purified from the above-described culture material using, for example, the following procedures.

An example of a method suitably used to extract the protein of the present invention from cultured bacteria or cells is as follows: the bacteria or cells are collected post-cultivation by a known method and suspended in a suitable buffer, and the bacteria or cells are then disrupted by, for example, ultrasound, lysozyme, and/or freeze-thaw cycling, followed by centrifugal separation, filtration, etc., to produce a crude extract of the protein. The buffer may contain a protein denaturant such as urea or guanidine hydrochloride, and/or a surfactant such as Triton X-100^{™}, etc. When the protein is secreted into the culture broth, the supernatant can be separated post-cultivation from the bacteria or cells by method known as such and the supernatant can be collected.

The protein present in the culture supernatant or extract obtained in this manner can be purified by appropriately combining methods of separation and purification that are known as such. These known methods of separation and purification include methods that exploit solubility, such as salting out, solvent precipitation, etc.; methods that primarily exploit differences in molecular weight, such as dialysis, ultrafiltration, gel filtration, SDS-polyacrylamide gel electrophoresis, etc.; methods that exploit differences in electric charge, such as ion-exchange chromatography etc.; methods that utilize specific affinity, such as affinity chromatography etc.; methods that utilize differences in hydrophobicity, such as reverse-phase high performance liquid chromatography etc.; and methods that utilize isoelectric point differences, such as isoelectrofocusing electrophoresis etc.

When the thusly obtained protein is in a free form, it can be converted into the salt by publicly known methods or methods based thereon. When, on the other hand, the protein is obtained in the form of a salt, it can be converted into the free form or into a different salt by publicly known methods or methods based thereon.

The protein produced by the recombinant can be treated, prior to purification or after purification, with an appropriate protein-modifying enzyme in order to add an appropriate modification or remove a partial polypeptide. For example, trypsin, chymotrypsin, arginyl endopeptidase, a protein kinase, glycosidase, etc., can be used as this protein-modifying enzyme.

The presence of the thusly produced protein of the present invention can be measured by an enzyme immunoassay or western blotting using a specific antibody.

Antibody to the protein or partial peptide used by the present invention or its salt may be any polyclonal antibody or monoclonal antibody that can recognize the protein or partial peptide used by the present invention or salt thereof.

Antibody to the protein or partial peptide used by the present invention or its salt (in some instances abbreviated simply as the protein of the present invention in the following description of the antibody) can be produced, using the protein of the present invention as antigen, by a method known as such for producing an antibody or antiserum.

### Preparation of monoclonal antibody

### (a) Preparation of monoclonal antibody-producing cells

The protein of the present invention is administered, either as such or in combination with a carrier and/or diluent, to a warm-blooded animal at a site that enables antibody production due to the administration to occur. Freund's complete adjuvant or Freund's incomplete adjuvant may be administered at the time of administration in order to potentiate antibody productivity. Administration is usually carried out once every 2 to 6 weeks and approximately 2 to 10 times in total. Examples of applicable warm-blooded animals are monkeys, rabbits, dogs, guinea pigs, mice, rats, sheep, goats, and the chicken, with the use of mice and rats being preferred.

To prepare monoclonal antibody-producing cells, individuals with a confirmed antibody titer can be selected from warm-blooded animals, for example, mice, that have been immunized with the antigen, the spleen or lymph nodes can be collected 2 to 5 days after the final immunization, and the antibody-producing cells contained therein can be fused with myeloma cells from allogenic or heterogenic animals to give monoclonal antibody-producing hybridomas. Measurement of the antibody titer in the antiserum may be carried out, for example, by reacting a labeled protein, vide infra, with the antiserum followed by measuring the activity of the label that has bonded to the antibody. Fusion may be carried out by a known method, for example, according to the method of Koehler and Milstein (Nature, 256, 495, (1975)). Examples of fusion promoters are polyethylene glycol (PEG), Sendai virus, etc., wherein the use of PEG is preferred.

The myeloma cells can be exemplified by myeloma cells from warm-blooded animals, such as NS-1, P3U1, SP2/0, AP-1, etc., and P3U1 is preferably used. A preferred ratio of the number of antibody-producing cells used (spleen cells) to the number of myeloma cells is approximately 1 : 1 to 20 : 1. Cell fusion can be carried out at good efficiency when PEG (preferably PEG 1000 to PEG 6000) is added at a concentration of approximately 10 to 80% followed by incubation at 20 to 40°C and preferably at 30 to 37°C for 1 to 10 minutes.

Various methods can be used to screen monoclonal antibody-producing hybridomas. For example, the supernatant from a hybridoma culture can be added to a solid phase (for example, a microplate) that has adsorbed the protein antigen directly or with a carrier and the monoclonal antibody bound to the solid phase can then be detected by adding protein A or an anti-immunoglobulin antibody (when mouse cells are used for cell fusion, anti-mouse immunoglobulin antibody is employed) labeled with a radioactive substance or an enzyme. In another method, the supernatant from a hybridoma culture can be added to a solid phase that has adsorbed an anti-immunoglobulin antibody or protein A and the monoclonal antibody bound to the solid phase can then be detected by adding the protein labeled with a radioactive substance or an enzyme.

The monoclonal antibody can be selected according to publicly known methods or methods based thereon. In general, selection can be carried out in an animal cell medium supplemented with HAT (hypoxanthine, aminopterin, thymidine). Any medium that enables hybridoma growth can be used for the selection and growth medium. For example, RPMI 1640 medium containing 1 to 20% and preferably 10 to 20% fetal bovine serum, GIT medium (Wako Pure Chemical Industries, Ltd.) containing 1 to 10% fetal bovine serum, a serum-free medium for hybridoma cultivation (SFM-101, Nissui Seiyaku Co., Ltd.), etc., can be used. The cultivation temperature is generally 20 to 40°C and preferably approximately 37°C, and the cultivation time is generally 5 days to 3 weeks and preferably 1 to 2 weeks. Cultivation is generally carried out under 5% carbon dioxide. The antibody titer in the hybridoma culture supernatant can be measured in the same manner as the above-described measurement of the antibody titer in the antiserum.

### (b) Purification of the monoclonal antibody

The separation and purification of the monoclonal antibody can be carried out by publicly known methods, for example, using a method for the separation and purification of immunoglobulins (for example, salting-out, alcohol precipitation, isoelectric point precipitation, electrophoresis, adsorption and desorption on an ion exchanger (for example, DEAE), ultracentrifugation, gel filtration, and specific purification methods in which only the antibody is collected using an antigen-bonded solid phase or an activated adsorbent such as protein A or protein G and obtaining the antibody by bond dissociation).

### Preparation of polyclonal antibody

Polyclonal antibody of the present invention can be produced by publicly known methods or methods based thereon. For example, production can be carried out by preparing the immunogen per se (protein antigen) or preparing its complex with a carrier protein; immunizing a warm-blooded animal therewith in the same manner as in the above-described method for producing monoclonal antibody; collecting material containing the antibody against the protein of the present invention from the immunized animal; and separating and purifying the antibody.

With regard to the immunogen/carrier protein complex used to immunize the warm-blooded animal and specifically with regard to the type of carrier protein and mixing ratio between the carrier and hapten, any type of carrier protein may be crosslinked at any ratio as long as antibody is efficiently produced against the hapten immunized crosslinked to the carrier. For example, a procedure can be used in which bovine serum albumin, bovine thyroglobulin, or hemocyanin is coupled to the hapten at a carrier-to-hapten weight ratio of approximately 0.1 to 20 and preferably approximately 1 to 5.

Various condensing agents can be used to couple the hapten to the carrier. Glutaraldehyde, carbodiimide, maleimide activated ester, activated ester reagents containing a thiol group or dithiopyridyl group, etc., may be used.

The condensation product is administered, either as such or in combination with a carrier and/or diluent, to a warm-blooded animal at a site that enables antibody production to occur. Freund's complete adjuvant or Freund's incomplete adjuvant may be administered at the time of administration in order to potentiate antibody productivity. Administration is usually carried out once every 2 to 6 weeks and approximately 3 to 10 times in total.

The polyclonal antibody can be collected from the blood, ascites fluid, etc., and preferably from the blood, of the warm-blooded animal immunized by the method described above.

The polyclonal antibody titer in the antiserum can be measured in the same manner as for the above-described measurement of the antibody titer in antiserum. Separation and purification of the polyclonal antibody can be carried out in accordance with the same methods for immunoglobulin separation/purification as in the above-described separation and purification of monoclonal antibody.

The antisense polynucleotide having a base sequence, or a portion of said base sequence, that is complementary or substantially complementary to the base sequence of polynucleotide (preferably DNA) that encodes the protein or partial peptide used by the present invention (in some instances in the following description of the antisense polynucleotide, this latter DNA is referred to in an abbreviated form as the DNA of the present invention), can be any antisense polynucleotide that has a base sequence, or a portion of said base sequence, complementary or substantially complementary to the base sequence of DNA of the present invention and that has the capacity to suppress the expression of said DNA; antisense DNA is preferred.

A base sequence substantially complementary to the DNA of the present invention is exemplified by a base sequence that has at least approximately 70% homology, preferably at least approximately 80% homology, more preferably at least approximately 90% homology, and most preferably at least approximately 95% homology with the total base sequence, or a partial base sequence, of a base sequence complementary (i.e., the complementary strand for the DNA of the present invention) to the DNA of the present invention. With respect, in particular, to the total base sequence of the complementary strand for the DNA of the present invention, (i) in the case of antisense polynucleotide directed to translation inhibition, a very suitable antisense polynucleotide has at least approximately 70% homology, preferably at least approximately 80% homology, more preferably at least approximately 90% homology, and most preferably at least approximately 95% homology to the complementary strand for the base sequence of the region that encodes the N-terminal region of the protein of the present invention (for example, the base sequence flanking the initiation codon), while (ii) in the case of antisense polynucleotide directed to RNA degradation by RNaseH, a very suitable antisense polynucleotide has at least approximately 70% homology, preferably at least approximately 80% homology, more preferably at least approximately 90% homology, and most preferably at least approximately 95% homology to the complementary strand for the intron-containing total base sequence of the DNA of the present invention.

Specific examples comprise antisense polynucleotide that has a base sequence, or a portion thereof, that is complementary or substantially complementary to the base sequence of DNA that contains the base sequence shown by SEQ ID NO: 2, and preferably, for example, antisense polynucleotide that has a base sequence, or a portion thereof, that is complementary to the base sequence of DNA that contains the base sequence shown by SEQ ID NO: 2 (more preferably, antisense polynucleotide that has a base sequence, or a portion thereof, that is complementary to the base sequence of DNA that contains the base sequence shown by SEQ ID NO: 2).

The antisense polynucleotide is constituted generally of approximately 10 to 40 and preferably approximately 15 to 30 bases.

To prevent degradation by hydrolases such as nucleases, the phosphate residue of each nucleotide making up the antisense DNA may be replaced by a chemically modified phosphate residue, for example, phosphorothioate, methyl phosphonate, phosphorodithionate, etc. In addition, the sugar (deoxyribose) in each nucleotide may be replaced by a chemically modified sugar structure, for example, by 2' -O-methylation, and the base moiety (pyrimidine, purine) may also be chemically modified; any such modification may be carried out as long as the resulting antisense polynucleotide can hybridize with DNA having the base sequence shown by SEQ ID NO: 2. These antisense polynucleotides can be produced, for example, using a known DNA synthesizer.

According to the present invention, the antisense polynucleotide (nucleic acid) that corresponds to a gene for the protein of the present invention and is capable of inhibiting the replication or expression of said gene, can be designed and synthesized based on the base sequence information for the DNA that codes for the cloned or identified protein. Such an antisense polynucleotide can hybridize with RNA for the gene for the protein of the present invention and can thereby inhibit the synthesis or function of said RNA, or can modulate and/or control the expression of the gene for the protein of the present invention via interaction with RNA related to the protein of the present invention. Polynucleotide complementary to selected sequences of RNA related to the protein of the present invention and polynucleotide specifically hybridizable to RNA related to the protein of the present invention are useful for modulating and/or controlling the in vivo and in vitro expression of the gene for the protein of the present invention and are useful for the treatment or diagnosis of diseases etc. The term "corresponds" means complementary to or having homology with a particular sequence of the gene-containing nucleotide, base sequence, or nucleic acid. "Correspondence" between a peptide and a nucleotide, base sequence, or nucleic acid generally denotes the amino acids of the protein derived (in order) from a nucleotide sequence (nucleic acid) or a species complementary thereto. The 5' hairpin loop, 5' 6-base pair repeats, 5' nontranslated region, polypeptide translation initiation codon, protein coding region, ORF translation stop codon, 3' nontranslated region, 3' palindrome region, 3' hairpin loop, etc. of the gene for the protein may be selected as preferred target regions; however, any region within the gene for the protein may be selected as a target.

With regard to the relationship between a nucleic acid under consideration and polynucleotide complementary to at least a part of a target region, this nucleic acid under consideration can be said to be "antisense" with respect to the polynucleotide of this target region when the nucleic acid under consideration can hybridize with the target region. Antisense polynucleotides can be exemplified by polynucleotides containing 2-deoxy-D-ribose, polynucleotides containing D-ribose, other types of polynucleotides that are N-glycosides of a purine or pyrimidine base, other polymers that have a non-nucleotide skeleton (for example, commercially available nucleic acids for proteins and synthetic sequence-specific nucleic acid polymers), and other polymers that contain special bonds (provided that these polymers contain nucleotides that have a configuration that allows base pairing or base stacking as found in DNA or RNA). These antisense polynucleotides may be double-stranded DNA, single-stranded DNA, double-stranded RNA, single-stranded RNA, or a DNA:RNA hybrid, and may also be an unmodified polynucleotide (or unmodified oligonucleotide), a polynucleotide with a known modification, for example, polynucleotide having a label as known in the pertinent field, capped polynucleotide, methylated polynucleotide, polynucleotide in which at least one naturally occurring nucleotide has been substituted by an analogue, polynucleotide having an intramolecular nucleotide modification, for example, having an uncharged linkage (for example, methyl phosphonate, phosphotriester, phosphoramidate, carbamate, etc.) or a charged linkage or sulfur-containing linkage (for example, phosphorothioates, phosphorodithioates, etc.), polynucleotide having side chain groups, for example, protein (for example, nucleases, nuclease inhibitors, toxins, antibodies, signal peptides, poly-L-lysine, etc.) and/or saccharide (for example, monosaccharides etc.), polynucleotide having an intercalation compound (for example, acridine, psoralen, etc.), polynucleotide containing a chelation compound (for example, metals, radioactive metals, boron, oxidizing metals, etc.), polynucleotide containing an alkylating agent, and polynucleotide having modified linkages (for example, α-anomeric nucleic acids etc.). Here, the terms "nucleoside", "nucleotide", and "nucleic acid" encompass same that not only contain purine and pyrimidine bases, but also other modified heterocyclic bases. Such modified heterocyclic bases encompass methylated purines and pyrimidines, acylated purines and pyrimidines, and other heterocyclic rings. Modified nucleotides and modified nucleotides also include modifications to the sugar moiety, for example, the substitution of one or more hydroxyl groups with halogen or an aliphatic group, conversion to a functional group such as an ether or amine, etc.

The antisense polynucleotide of the present invention is RNA, DNA, or a modified nucleic acid (RNA, DNA). Specific examples of modified nucleic acids are, inter alia, sulfur derivatives and thiophosphate derivatives of nucleic acids and degradation-resistant species of polynucleoside amides and oligonucleoside amides. The antisense polynucleotide of the present invention can be designed, for example, based on increasing the intracellular stability of the antisense polynucleotide, raising the cell permeability of the antisense polynucleotide, increasing the affinity to the sense strand target, and reducing the toxicity, if any, of the antisense polynucleotide. These modifications have been widely reported, for example, by Pharm. Tech. Japan, Volume 8, pages 247 and 395, 1992, and Antisense Research and Applications, CRC Press, 1993.

The antisense polynucleotide of the present invention may contain altered or modified sugars, bases, and/or bonds, and can be provided in a specialized form such as a liposome or microsphere, or may be applied through gene therapy, or may be provided in a conjugated form. The species used in such conjugated forms can be exemplified by polycations, such as polylysine, that act to neutralize the charge on the phosphate backbone, and hydrophobic species such as lipids (for example, phospholipid, cholesterol, etc.) that enhance interaction with the cell membrane and/or increase nucleic acid uptake. Lipids preferred for conjugation can be exemplified by cholesterol and derivatives thereof (for example, cholesteryl chloroformate, cholic acid, etc.). These may be attached at the 3' or 5' end of the nucleic acid or through the base, sugar, or an intramolecular nucleoside bond. Other groups may be capping groups specifically disposed at the 3' or 5' end of the nucleic acid to prevent degradation by nucleases such as exonuclease, RNase, etc. These capping groups can be exemplified by, but are not limited to, hydroxyl protecting groups as known in the pertinent field, most prominently glycols such as polyethylene glycol, tetraethylene glycol, etc.

The inhibitory activity of the antisense polynucleotide can be evaluated using a transformant of the present invention, an in vivo or in vitro gene expression system of the present invention, or an in vivo or in vitro translation system for the protein of the present invention.

Applications of the protein and partial peptide of the present invention and salts thereof (in some instances hereinafter abbreviated as the protein of the present invention), polynucleotide (for example, DNA) encoding the protein or partial peptide of the present invention (in some instances hereinafter abbreviated as the DNA of the present invention), antibody against the protein or partial peptide of the present invention or its salt (in some instances hereinafter abbreviated as the antibody of the present invention), and antisense polynucleotide for the polynucleotide (for example, DNA) of the present invention (in some instances hereinafter abbreviated as antisense polynucleotide of the present invention) are described herebelow.

The protein of the present invention has an upregulated expression in cancer cells and has, for example, an acyl-CoA synthetase activity, fatty acid transport activity, etc. The protein of the present invention is therefore useful as a marker for cancer tissue for early diagnosis, for evaluating disease severity, and for predicting disease progression. In addition, the expression of the protein of the present invention is further upregulated in the case of tyrosine kinase signaling pathway inhibitor-resistant cancer cells (for example, MEK inhibitor-resistant cancer cells, EGFR inhibitor-resistant cancer cells, and HER2 inhibitor-resistant cancer cells). The protein of the present invention is therefore useful in particular as a marker for tyrosine kinase signaling pathway inhibitor-resistant cancer tissue for early diagnosis, for evaluating disease severity, and for predicting disease progression. In addition, compounds that inhibit the activity of the protein of the present invention and the salts of such compounds, antibody against the protein of the present invention, compounds that inhibit the expression of a gene for protein of the present invention and salts of such compounds, antisense polynucleotide for a gene encoding protein of the present invention, siRNA or shRNA for a gene encoding protein of the present invention, etc., can be used as an agent for the prevention/treatment of cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), as a promoter of cancer cell apoptosis, or as an inhibitor of cancer cell proliferation, and preferably as an agent for the prevention/treatment of tyrosine kinase signaling pathway inhibitor-resistant cancers (for example, MEK inhibitor-resistant cancers, EGFR inhibitor-resistant cancers, HER2 inhibitor-resistant cancers, etc.), as a promoter of the apoptosis of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.), or as an inhibitor of the proliferation of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.).

### (1) Screening of candidate drug compounds for diseases

The protein of the present invention has an upregulated expression in cancer cells (particularly tyrosine kinase signaling pathway inhibitor-resistant cancer cells) and has, for example, an acyl-CoA synthetase activity, fatty acid transport activity, etc. Compounds that inhibit the activity of the protein of the present invention and the salts of such compounds can therefore be used as a low-toxicity agent for the prevention/treatment of cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), as a promoter of cancer cell apoptosis, or as an inhibitor of cancer cell proliferation, and preferably as an agent for the prevention/treatment of tyrosine kinase signaling pathway inhibitor-resistant cancers, as a promoter of the apoptosis of tyrosine kinase signaling pathway inhibitor-resistant cancer cells, or as an inhibitor of the proliferation of tyrosine kinase signaling pathway inhibitor-resistant cancer cells.

Accordingly, protein of the present invention is useful as a reagent for screening for compounds and salts thereof that inhibit the activity of protein of the present invention.

That is, the present invention provides a method of screening for compounds and salts thereof that inhibit the activity of protein of the present invention (for example, acyl-CoA synthetase activity, fatty acid transport activity, etc.), said method being characterized by the use of protein of the present invention.

The methods described below in (A) and (B) are specific examples thereof.
(A) Screening for compounds and salts thereof that inhibit the activity of protein of the present invention by comparing (i) the acyl-CoA synthetase behavior of protein of the present invention with (ii) the acyl-CoA synthetase activity of a mixture of the protein of the invention with a test compound.
   (A-1) Screening for compounds and salts thereof that inhibit the activity of protein of the present invention by measuring the acyl-CoA synthetase activity (i) when protein of the present invention and fatty acid labeled with a radioisotope or a fluorescent substance are reacted and (ii) when protein of the present invention and fatty acid labeled with a radioisotope or a fluorescent substance are reacted in the presence of a test compound.
      These reactions are carried out in a suitable buffer. After the enzymatic reaction, for example, the reaction product is separated by, for example, partition between the aqueous phase and an organic solvent phase and extracting the acyl-CoA that is dissolved in the aqueous phase. The radioactivity or fluorescence intensity of the acyl-CoA is measured according to a known method using, for example, a scintillation counter or fluorography. For example, [¹²⁵I], [¹³¹I], [³H], [¹⁴C], [³²P], [³³P], or [³⁵S] can be used as the radioisotope, while, for example, a cyanine fluorescent dye (for example, Cy2, Cy3, Cy5, Cy5.5, Cy7 (available from Amersham Bioscience)), fluorescamine, fluorescein isothiocyanate, NBD (7-nitrobenz-2-oxa-1,3-diazol), or BODIPY (boron dipyrromethene) can be used as the fluorescent substances.
   (A-2) Screening for compounds and salts thereof that inhibit the activity of protein of the present invention by measuring the acyl-CoA synthetase activity (i) when protein of the present invention and radioisotope-labeled fatty acid are reacted and (ii) when protein of the present invention and radioisotope-labeled fatty acid are reacted in the presence of a test compound.
      In specific terms, the protein of the present invention is reacted, in the presence of a test compound and in the absence of the test compound, for 10 minutes at 35°C in 0.5 mL of a solution that contains 0.2 mM Tris-HCl buffer (pH 7.5), 2.5 mM ATP, 8 mM MgCl₂, 2 mM EDTA, 20 mM NaF, 0.1% (w/v) Triton X-100, 10 µM [1-¹⁴C]-palmitic acid (5 µCi/µL), and 0.5 mM coenzyme A. The reaction is started by the addition of the CoA and is stopped by the addition of 2.5 mL isopropanol : n-heptane : 1 M sulfuric acid (40 : 10 : 1, v/v). After the reaction has been stopped, 0.5 mL water and 2.5 mL n-heptane are added; the organic solvent phase, which contains the unreacted fatty acid, is removed; the aqueous phase is washed 3 times with 2.5 mL n-heptane; and the radioactivity remaining in the aqueous phase is measured according to a known method using, for example, a scintillation counter.
   (A-3) Screening for compounds and salts thereof that inhibit the activity of protein of the present invention by measuring the acyl-CoA synthetase activity (i) when protein of the present invention and fatty acid labeled with a fluorescent substance are reacted and (ii) when protein of the present invention and fatty acid labeled with a fluorescent substance are reacted in the presence of a test compound.
      In specific terms, the protein of the present invention is reacted, in the presence of a test compound and in the absence of the test compound, for 10 minutes at 35°C in 0.5 mL of a solution that contains 0.2 mM Tris-HCI buffer (pH 7.5), 2.5 mM ATP, 8 mM MgCl₂, 2 mM EDTA, 20 mM NaF, 0.1% (w/v) Triton X-100, 10 µM C₁-BODIPY-C₁₂ (4,4-difluoro-5-methyl-4-bora-3a,4a-diaza-*s*-indacene-3-dodecanoic acid), and 0.5 mM coenzyme A. The reaction is started by the addition of the CoA and is stopped by the addition of 2.5 mL isopropanol : n-heptane : 1 M sulfuric acid (40 : 10 : 1, v/v). After the reaction has been stopped, 0.5 mL water and 2.5 mL n-heptane are added; the organic solvent phase, which contains the unreacted fatty acid, is removed; the aqueous phase is washed 3 times with 2.5 mL n-heptane; and the fluorescence intensity remaining in the aqueous phase is measured according to a known method using, for example, fluorography.
(B) Screening for compounds or salts thereof that inhibit the activity of protein of the present invention by comparing (i) the fatty acid transport activity of the protein of the present invention with (ii) the fatty acid transport activity of a mixture of the protein of the present invention with a test compound.
   (B-1) Screening for compounds or salts thereof that inhibit the activity of protein of the present invention by measuring the fatty acid transport activity (i) for the cultivation of cells that have the capacity to produce protein of the present invention and (ii) for the cultivation, in the presence of a test compound, of the cells that have the capacity to produce protein of the present invention.
      Measurement of the fatty acid transport activity is carried out in a suitable buffer using fatty acid that has been labeled with a radioisotope or a fluorescent substance. After the reaction of fatty acid transport into suitable cells, for example, the fatty acid not incorporated into the cells is removed by washing the cells with a suitable buffer and the radioactivity or fluorescence intensity of the remaining fatty acid incorporated into the cells is measured. The radioactivity or fluorescence intensity is measured according to a known method using, for example, a scintillation counter or fluorography. For example, [¹²⁵I], [¹³¹I], [³H], [¹⁴C], [³²P], [³³P], or [³⁵S] can be used as the radioisotope, while, for example, a cyanine fluorescent dye (for example, Cy2, Cy3, Cy5, Cy5.5, Cy7 (available from Amersham Bioscience)), fluorescamine, fluorescein isothiocyanate, NBD (7-nitrobenz-2-oxa-1,3-diazol), or BODIPY (boron dipyrromethene) can be used as the fluorescent substances.
   (B-2) Screening for compounds or salts thereof that inhibit the activity of protein of the present invention by measuring the radioisotope-labeled fatty acid transport activity (i) for the cultivation of cells that have the capacity to produce protein of the present invention and (ii) for the cultivation, in the presence of a test compound, of the cells that have the capacity to produce protein of the present invention.
      In specific terms, protein of the present invention is introduced into a mutant strain of yeast *(Saccharomyces cerevisiae)* that is deficient in fat 1, a lipid transporter gene, followed by cultivation in the presence of a test compound or in the absence of the test compound. After the cultivated yeast has been washed with phosphate buffer (PBS), the cells are suspended in PBS so as to give a cell density of 6 × 10⁷/mL. To this is added [1-¹⁴C]palmitic acid to a final concentration of 2.5 µM and fatty acid-free bovine serum albumin (BSA) to a final concentration of 7.5 µM followed by standing for 3 minutes at room temperature. The [1-¹⁴C]palmitic acid not incorporated into the yeast is removed by washing twice with PBS containing 50 µM fatty acid-free BSA. The radioactivity generated by the [1-¹⁴C]palmitic acid incorporated into the yeast cells is measured by a known method using, for example, a scintillation counter.
   (B-3) Screening for compounds or salts thereof that inhibit the activity of protein of the present invention by measuring the fluorescent substance-labeled fatty acid transport activity (i) for the cultivation of cells that have the capacity to produce protein of the present invention and (ii) for the cultivation, in the presence of a test compound, of the cells that have the capacity to produce protein of the present invention.

In specific terms, protein of the present invention is introduced into a mutant strain of yeast *(Saccharomyces cerevisiae)* that is deficient in fat1, a lipid transporter gene, followed by cultivation in the presence of a test compound or in the absence of the test compound. After the cultivated yeast has been washed with phosphate buffer (PBS), the cells are suspended in PBS so as to give a cell density of 6 × 10⁷/mL. To this is added C₁-BODIPY-C₁₂ (4,4-difluoro-5-methyl-4-bora-3a,4a-diaza-*s*-indacene-3-dodecanoic acid) to a final concentration of 2.5 µM and fatty acid-free bovine serum albumin (BSA) to a final concentration of 7.5 µM followed by standing for 3 minutes at room temperature in the dark. The fluorescence of the C₁-BODIPY-C₁₂ not incorporated into the yeast is quenched with trypan blue at a final concentration of 0.33 mM and the fluorescence intensity generated by the C₁-BODIPY-C₁₂ incorporated into the yeast cells is measured by a known method using, for example, a fluorescence plate reader.

The protein of the present invention as described above is preferably produced by the cultivation of a transformant that contains DNA that encodes the protein of the present invention. In addition, the reactions can be similarly carried out using cells that can express protein of the present invention.

For example, a host transformed (transformant) with a vector comprising DNA that encodes protein of the present invention as described above can be used as the cells that have the capacity to produce protein of the present invention. For example, yeast cells and animal cells such as COS7 cells, CHO cells, HEK293 cells, etc., are preferably used as the host. The use is preferred in the aforementioned screening of, for example, a transformant that expresses protein of the present invention by cultivation by the aforementioned procedures. The procedure for cultivating the cells capable of expressing the protein of the present invention is the same as the procedure for cultivating a transformant of the present invention as described above.

The test compound can be exemplified by peptides, proteins, antibodies, nonpeptide compounds, synthetic compounds, fermentation products, cell extracts, plant extracts, animal tissue extracts, blood serum, etc. The test compound may be in salt form, and the test compound salts can be, for example, physiologically acceptable metal salts, ammonium salts, salts with organic bases, salts with inorganic acids, salts with organic acids, and salts with basic or acidic amino acids. Suitable metal salts can be exemplified by alkali metal salts such as the sodium salt, potassium salt, etc.; alkaline-earth metal salts such as the calcium salt, magnesium salt, barium salt, etc.; and aluminum salts. Suitable examples of salts with organic bases are, for example, salts with trimethylamine, triethylamine, pyridine, picoline, 2,6-lutidine, ethanolamine, diethanolamine, triethanolamine, cyclohexylamine, dicyclohexylamine, and N,N'-dibenzylethylenediamine. Suitable examples of salts with inorganic acids are, for example, salts with hydrochloric acid, hydrobromic acid, nitric acid, sulfuric acid, and phosphoric acid. Suitable examples of salts with organic acids are, for example, salts with formic acid, acetic acid, trifluoroacetic acid, propionic acid, phthalic acid, fumaric acid, oxalic acid, tartaric acid, maleic acid, citric acid, succinic acid, malic acid, methanesulfonic acid, benzoic acid, benzenesulfonic acid, and p-toluenesulfonic acid. Suitable examples of salts with basic amino acids are, for example, salts with arginine, lysine, and ornithine. Suitable examples of salts with acidic amino acids are, for example, salts with aspartic acid and glutamic acid.

For example, a test compound that causes the acyl-CoA synthetase activity or fatty acid transport activity in the aforementioned case (ii) to be reduced at least approximately 20%, preferably at least 30%, and more preferably at least approximately 50% with respect to that in the aforementioned case (i) can be selected as a compound that inhibits the activity of protein of the present invention.

A compound that has an inhibitory activity with respect to the activity of protein of the present invention is useful as a safe, low-toxicity drug for inhibiting the physiological activity of protein of the present invention, for example, as an agent for the prevention/treatment of cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), as a promoter of cancer cell apoptosis, or as an inhibitor of cancer cell proliferation, and preferably as an agent for the prevention/treatment of tyrosine kinase signaling pathway inhibitor-resistant cancers (for example, MEK inhibitor-resistant cancers, EGFR inhibitor-resistant cancers, HER2 inhibitor-resistant cancers, etc.), as a promoter of the apoptosis of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.), or as an inhibitor of the proliferation of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.).

The compound or its salt obtained using the screening method or screening kit of the present invention is, for example, a compound selected from peptides, proteins, antibodies, nonpeptide compounds, synthetic compounds, fermentation products, cell extracts, plant extracts, animal tissue extracts, blood plasma, etc. The same salts described above in connection with the test compound are used for the salts of this compound.

Moreover, since the gene that encodes protein of the present invention also has an upregulated expression in cancer cells (particularly in tyrosine kinase signaling pathway inhibitor-resistant cancer cells) and produces protein that has an acyl-CoA synthetase activity or fatty acid transport activity, a compound or its salt that inhibits the expression of the gene coding for protein of the present invention is also useful as an agent for the prevention/treatment of cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), as a promoter of cancer cell apoptosis, or as an inhibitor of cancer cell proliferation, and preferably as an agent for the prevention/treatment of tyrosine kinase signaling pathway inhibitor-resistant cancers, a promoter of the apoptosis of tyrosine kinase signaling pathway inhibitor-resistant cancer cells, or an inhibitor of the proliferation of tyrosine kinase signaling pathway inhibitor-resistant cancer cells.

Accordingly, the DNA of the present invention is useful as a reagent for screening for compounds or salts thereof that inhibit the expression of the gene that encodes protein of the present invention.

The screening method can be exemplified by a screening method that characteristically comprises comparing (iii) the cultivation of cells that have the capacity to produce protein of the present invention with (iv) the cultivation in the presence of a test compound of the cells that have a capacity to produce the protein used by the present invention.

In this method, the level of expression (specifically, the amount of protein of the present invention or the amount of mRNA encoding this protein) of the gene in (iii) and (iv) is measured and compared.

The test compounds and cells capable of producing protein of the present invention can be exemplified by the previously cited test compounds and cells.

The amount of protein can be measured by known methods; for example, the protein of the present invention that is present in, for example, a cell extract can be measured using antibody that recognizes this protein in accordance with, for example, western analysis or ELISA or procedures based thereon.

The amount of mRNA can be measured by known methods. For example, the amount of mRNA can be measured by northern hybridization using nucleic acid containing SEQ ID NO: 2 or a portion thereof as the probe, or by PCR using nucleic acid containing SEQ ID NO: 2 or a portion thereof as probes, or by procedures based on the preceding.

For example, a test compound that provides at least an approximately 20%, preferably at least a 30%, and more preferably at least an approximately 50% inhibition of the level of gene expression in the aforementioned case (iv) relative to that in the aforementioned case (iii) can be selected as a compound that inhibits expression of the gene that encodes the protein of the present invention.

The screening kit of the present invention contains protein used by the present invention or a partial peptide or its salt, or contains cells that have the capacity to produce the protein used by the present invention or a partial peptide thereof.

A compound or its salt obtained using the screening method or screening kit of the present invention is a compound or its salt selected from test compounds as described above, for example, peptides, proteins, antibodies, nonpeptide compounds, synthetic compounds, fermentation products, cell extracts, plant extracts, animal tissue extracts, blood plasma, etc., that inhibits the activity of protein of the present invention (for example, acyl-CoA synthetase activity, fatty acid transport activity, etc.), or that inhibits the expression of the gene for said protein, or that inhibits the expression of the protein of the present invention.

The salts of this compound can be the same salts as cited above for the test compound.

Compounds or salts thereof that inhibit the activity of protein of the present invention, compounds or salts thereof that inhibit the expression of the gene for protein of the present invention, and compounds or salts thereof that inhibit the expression of protein of the present invention have a low toxicity and are useful, for example, as an agent for the prevention/treatment of cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), as a promoter of cancer cell apoptosis, or as an inhibitor of cancer cell proliferation.

Moreover, because these compounds and salts thereof exhibit an even stronger action against, for example, tyrosine kinase signaling pathway inhibitor-resistant cancers (for example, MEK inhibitor-resistant cancers, EGFR inhibitor-resistant cancers, HER2 inhibitor-resistant cancers, etc.) and tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.), they are particularly useful as drugs such as agents for the prevention/treatment of tyrosine kinase signaling pathway inhibitor-resistant cancers (for example, MEK inhibitor-resistant cancers, EGFR inhibitor-resistant cancers, HER2 inhibitor-resistant cancers, etc.), promoters of the apoptosis of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.), and inhibitors of the proliferation of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.).

A compound or its salt obtainable using the screening method or screening kit of the present invention can be formulated according to conventional procedures when it is to be used as an agent as cited above.

For example, compositions for oral administration can be exemplified by solid and liquid dosage forms and specifically by tablets (including sugar-coated tablets and film-coated tablets), pills, granules, powders, capsules (including soft capsules), syrups, emulsions, suspensions, etc. Such a composition can be prepared by publicly known methods and contain a vehicle, diluent, or excipient as ordinarily used in the formulation field. Examples of the vehicle or excipient for tablets are lactose, starch, sucrose, magnesium stearate, etc.

Compositions for non-oral administration are exemplified by injectable preparations, suppositories, etc. Injectable preparations encompass formulations such as intravenous injections, subcutaneous injections, intracutaneous injections, intramuscular injections, drip infusions, intraarticular injections, etc. These injectable preparations are prepared by publicly known methods. For example, an injectable preparation may be prepared by dissolving, suspending, or emulsifying an antibody as described above, or salt thereof, in a sterile aqueous or oil-based medium as ordinarily used for injectable preparations. The aqueous medium for injection can be exemplified by physiological saline, an isotonic solution containing glucose and/or other auxiliary agents, etc., which may be used in combination with an appropriate solubilizing agent such as an alcohol (for example, ethanol), a polyalcohol (for example, propylene glycol, polyethylene glycol), a nonionic surfactant (for example, polysorbate 80, HCO-50 (polyoxyethylene (50 mol) adduct of hydrogenated castor oil)), etc. The oil-based medium can be exemplified by sesame oil, soybean oil, etc., which may be used in combination with a solubilizing agent such as benzyl benzoate, benzyl alcohol, etc. The prepared injectable preparation is usually filled into an appropriate ampul. A suppository for rectal administration may be prepared by mixing the aforementioned antibody or its salt with a conventional suppository base.

The above-described oral and non-oral drug compositions are desirably produced as unit dosage forms adapted to the active component dose. Examples of such unit dosage forms are tablets, pills, capsules, injectable preparations (ampuls), suppositories, etc. The content of the above-described antibody is preferably generally 5 to 500 mg per unit dosage form wherein 5 to 100 mg is particularly preferred for injectable preparations and 10 to 250 mg is particularly preferred for other dosage forms.

The compositions described above may additionally contain other active components as long as no adverse interaction is produced upon blending with the above-described antibody.

Since the formulations prepared in this manner are safe and low toxic, they can be administered to humans and warm-blooded animals (for example, mouse, rat, rabbit, sheep, pig, cow, horse, fowl, cat, dog, monkey, chimpanzee, etc.) by either an oral or non-oral route.

The dose of the compound or its salt will vary depending on its action, target disease, recipient, status of the disease, route of administration, etc. For example, when a compound or its salt that inhibits the activity of protein of the present invention is administered orally to treat lung cancer, the compound or its salt is administered to adult (60 kg body weight) at approximately 0.1 to 100 mg, preferably at approximately 1.0 to 50 mg, and more preferably at approximately 1.0 to 20 mg per day. When administration is carried out by a non-oral route, the dose of the compound or its salt will vary depending on the target disease, recipient, status of the disease, route of administration, etc. For example, when a compound or its salt that inhibits the activity of protein of the present invention is administered in the form of an injectable in order to treat breast cancer, the compound or its salt is advantageously administered to adult (60 kg body weight) by injection at the cancerous lesion at approximately 0.01 to 30 mg, preferably approximately 0.1 to 20 mg, and more preferably approximately 0.1 to 10 mg per day. For other animal species, an amount calculated per 60 kg body weight can be administered.

In addition, a compound or its salt that inhibits the activity of protein of the present invention, a compound or its salt that inhibits the expression of the gene that encodes protein of the present invention, and a compound or its salt that inhibits the expression of protein of the present invention can each be used in combination with a hormone therapy drug, an anticancer agent (for example, a chemotherapeutic drug, an immunotherapeutic drug, a tyrosine kinase signaling pathway inhibitor (a drug that inhibits the action of a cell growth factor and its receptor)), etc. (abbreviated below as the concomitant drug).

Compounds of the present invention exhibit an excellent anticancer action even when used alone; however, their effect can be further strengthened by use in combination (multidrug combination) with one or several of the aforementioned concomitant drugs.

The hormone therapy drugs can be exemplified by fosfestrol, diethylstilbestrol, chlorotrianisene, medroxyprogesterone acetate, megestrol acetate, chlormadinone acetate, cypropterone acetate, danazol, dienogest, asoprisnil, allylestrenol, gestrinone, nomegestrol, tadenan, mepartricin, raloxifene, ormeloxifene, levormeloxifene, antiestrogens (for example, tamoxifen citrate, toremifene citrate, etc.), ER down regulators (for example, fulvestrant etc.), human menopausal gonadotropin, follicle-stimulating hormone, follicular hormone + lutein hormone combinations, mepitiostane, testolactone, aminogluthetimide, LH-RH agonists (for example, goserelin acetate, buserelin, leuprorelin, etc.), droloxifene, epitiostanol, ethynylestradiol sulfonate, aromatase inhibitors (for example, fadrozole hydrochloride, anastrozole, letrozole, exemestane, vorozole, formestane, etc.), antiandrogens (for example, flutamide, bicalutamide, nilutamide, etc.), 5α-reductase inhibitors (for example, finasteride, dutasteride, epristeride, etc.), adrenocortical hormone-type drugs (for example, dexamethasone, prednisolone, betamethasone, triamcinolone, etc.), androgen synthesis inhibitors (abiraterone etc.), and retinoids and drugs that retard retinoid metabolism (for example, liarozole etc.). LH-RH agonists (for example, goserelin acetate, buserelin, leuprorelin, etc.) are preferred.

Chemotherapeutic drugs can be exemplified by alkylating agents, antimetabolites, anticancer antibiotics, and plant-derived anticancer agents.

The alkylating agents can be exemplified by nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, estramustine phosphate sodium, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, adozelesin, cystemustine, bizelesin, etc.

The antimetabolites can be exemplified by mercaptopurine, 6-mercaptopurine riboside, thioinosine, methotrexate, enocitabine, cytarabine, cytarabine ocfosfate, ancitabine hydrochloride, 5-FU drugs (for example, fluorouracil, tegafur, UFT, doxifluridine, carmofur, gallocitabine, emitefur, etc.), aminopterin, leucovorin calcium, Tabloid, butocine, folinate calcium, levofolinate calcium, cladribine, emitefur, fludarabine, gemcitabine, hydroxycarbamide, pentostatin, piritrexim, idoxuridine, mitoguazone, thiazophrine, ambamustine, etc.

The anticancer antibiotics can be exemplified by actinomycin-D, actinomycin-C, mitomycin-C, chromomycin-A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride, etc.

The plant-derived anticancer agents can be exemplified by etoposide, etoposide phosphate, vinblastine sulfate, vincristine sulfate, vindesine sulfate, teniposide, paclitaxel, docetaxel, vinorelbine, etc.

The immunotherapeutic agents (BRM) can be exemplified by picibanil, krestin, sizofiran, lentinan, ubenimex, interferons, interleukins, macrophage colony-stimulating factor, granulocyte colony-stimulating factor, erythropoietin, lymphotoxin, BCG vaccine, *Corynebacterium parvum,* levamisole, polysaccharide K, procodazole, etc.

The cell growth factor in "a drug that inhibits the action of a cell growth factor and its receptor" may be any substance that promotes cell growth and is generally a factor that is a peptide with a molecular weight no greater than 20,000 and that exhibits its action at low concentrations by binding with a receptor. Specific examples are (1) epidermal growth factor (EGF) and substances that have substantially the same activity as EGF (for example, EGF, heregulin (HER2 ligand), etc.), (2) insulin and substances that have substantially the same activity as insulin (for example, insulin, insulin-like growth factor-1 (IGF-1), IGF-2, etc.), (3) fibroblast growth factor (FGF) and substances that have substantially the same activity as FGF (for example, acidic FGF, basic FGF, keratinocyte growth factor (KGF), FGF-10, etc.), (4) other cell growth factors (for example, colony-stimulating factor (CSF), erythropoietin (EPO), interleukin-2 (IL-2), nerve growth factor (NGF), platelet-derived growth factor (PDGF), transforming growth factor β (TGF β), hepatocyte growth factor (HGF), vascular endothelial growth factor (VEGF), etc.), etc.

The receptor for the cell growth factor may be any receptor that has the capacity to bind with a cell growth factor as described above, and specific examples are the EGF receptor, heregulin receptor (HER2), insulin receptor, IGF receptor, FGF receptor-1, FGF receptor-2, etc.

The tyrosine kinase signaling pathway inhibitor can be exemplified by trastuzumab (Herceptin (trademark); HER2 antibody), imatinib mesylate, ZD1839 or cetuximab, antibodies to VEGF (for example, bevacizumab), antibodies to the VEGF receptor, gefitinib, erlotinib, lapatinib, etc.

The following can also be used in addition to the drugs cited above: L-asparaginase, aceglatone, procarbazine hydrochloride, protoporphyrin cobalt complex salt, mercury hematoporphyrin sodium, topoisomerase I inhibitors (for example, irinotecan, topotecan, etc.), topoisomerase II inhibitors (for example, sobuzoxan etc.), differentiation inducers (for example, retinoids, D vitamins, etc.), angiogenesis inhibitors (for example, thalidomide, SU11248, etc.), α-blockers (for example, tamsulosin hydrochloride, naftopidil, urapidil, alfuzosin, terazosin, prazosin, silodosin, etc.), serine threonine kinase inhibitors, endoserine receptor antagonists (for example, atrasentan etc.), proteasome inhibitors (for example, bortezomib etc.), Hsp90 inhibitors (for example, 17-AAG etc.), spironolactone, minoxidil, 11α-hydroxyprogesterone, bone resorption inhibitors/metastasis inhibitors (for example, zoledronic acid, alendronic acid, pamidronic acid, etidronic acid, ibandronic acid, clodronic acid, etc.), etc.

Tyrosine kinase signaling pathway inhibitors are a preferred concomitant drug among the preceding because the co-use of a tyrosine kinase signaling pathway inhibitor makes it possible to further strengthen the effect of the hereinabove described compound or its salt and/or the tyrosine kinase signaling pathway inhibitor.

The use of a combination makes it possible to lower the dose of the hereinabove-described compound or its salt and/or the dose of the concomitant drug without lowering the anticancer activity required for prevention and/or treatment and thereby enables a lessening of side effects (for example, toxic effects on normal cells) without lessening the prophylactic and/or therapeutic effect. There is no limitation here on the administration schedule, and these may be administered to the recipient simultaneously or with an interval. The doses can be selected as appropriate based on the doses in use clinically. In addition, the ratio between the hereinabove-described compound and the concomitant drug may be selected as appropriate in view of the recipient, route of administration, target disease, status of the disease, the particular combination, etc.

### (2) Quantification of the protein of the present invention, its partial peptide, or a salt thereof

Antibody to protein of the present invention (in some instances referred to below as the antibody of the present invention) can specifically recognize protein of the present invention and can therefore be used to quantify protein of the present invention in a test fluid and in particular for quantification by sandwich immunoassay etc.

That is, the present invention provides:
(i) a method of quantifying the protein of the present invention in a test fluid, comprising competitively reacting antibody of the present invention, the test fluid, and labeled protein of the present invention, and measuring the proportion of the labeled protein of the present invention bound to the antibody; and,
(ii) a method of quantifying the protein of the present invention in a test fluid, comprising reacting the test fluid simultaneously or sequentially with antibody of the present invention insolubilized on a carrier and another, labeled antibody of the present invention, and thereafter measuring the activity of the label on the insolubilizing carrier.

In the aforementioned quantification method (ii), it is desirable that one antibody be antibody capable of recognizing the N-terminal region of the protein of the present invention while the other antibody be antibody capable of reacting with the C-terminal region of the protein of the present invention.

In addition to using monoclonal antibody to the protein of the present invention (in some instances referred to herebelow as the monoclonal antibody of the present invention) to quantify the protein of the present invention, detection by, for example, tissue staining can also be carried out. For these purposes, the antibody molecule itself may be used or the F(ab')₂, Fab', or Fab moieties of the antibody molecule may be used.

There are no particular limitations on the method of quantifying protein of the present invention using antibody of the present invention, and any measurement method may be used that can detect, by chemical or physical means, the amount of antibody, antigen, or antibody-antigen complex corresponding to the amount of antigen (for example, the amount of protein) in the solution being analyzed and that can calculate this amount from a reference curve constructed using reference solutions containing known amounts of the antigen. For example, nephelometry, a competitive procedure, an immunometric procedure, the sandwich method, etc. are suitably used, while the use of the sandwich method described below is particularly preferred from the standpoints of sensitivity and specificity.

The labeling agent used in measurement methods that employ a labeled material can be, for example, a radioisotope (for example, [¹²⁵I], [¹³¹I], [³H], [¹⁴C], [³²P], [³³P], [³⁵S], etc.), a fluorescent substance (for example, a fluorescent cyanine dye (for example, Cy2, Cy3, Cy5, Cy5.5, Cy7 (available from Amersham Bioscience)), fluorescamine, fluorescein isothiocyanate, NBD (7-nitrobenz-2-oxa-1,3-diazol), BODIPY (boron dipyrromethene), etc.), an enzyme (for example, β-galactosidase, β-glucosidase, an alkaline phosphatase, a peroxidase, malate dehydrogenase, etc.), a luminescent substance (for example, luminol, a luminol derivative, luciferin, lucigenin, etc.), biotin, a lanthanide element, etc. In addition, a biotin-avidin system may also be used to bind the antibody or antigen to a labeling agent.

Physical adsorption may be used for insolubilization of the antigen or antibody. Chemical binding techniques conventionally used for the insolubilization or immobilization of proteins, enzymes, etc. may also be used. The carrier can be exemplified by insoluble polysaccharides such as agarose, dextran, cellulose, etc.; synthetic resins such as polystyrene, polyacrylamide, silicone, etc.; glass; etc.

In the sandwich method, insolubilized monoclonal antibody of the present invention is reacted with a test fluid (primary reaction) and is further reacted with another, labeled monoclonal antibody of the present invention (secondary reaction); the amount of the protein of the present invention in the test fluid can then be quantified by measuring the activity of the label on the insolubilizing carrier. The order of the primary and secondary reactions may be reversed, and the reactions may be carried out simultaneously or with an intervening interval of time. The labeling agent and the procedure for insolubilizing can be in accordance with those already described above. In the sandwich immunoassay method, the antibody used for the solid-phase antibody is not necessarily a single species of antibody, nor is the antibody used for the labeled antibody necessarily a single species of antibody; rather, a mixture of two or more species of antibody may be used to increase the measurement sensitivity.

The monoclonal antibody of the present invention used in the primary reaction and the monoclonal antibody of the present invention used in the secondary reaction in the method of measuring the protein of the present invention by the sandwich method of the present invention are preferably antibodies that bind to different sites of the protein of the present invention. That is, with regard to the antibody used for the primary reaction and the antibody used for the secondary reaction, when, for example, the antibody used in the secondary reaction recognizes the C-terminal region of the protein of the present invention, the antibody used in the primary reaction is preferably antibody that recognizes a region other than the C-terminal region, for example, antibody that recognizes the N-terminal region.

The monoclonal antibody of the present invention can be used in assay systems other than the sandwich method; for example, it can be used in the competitive method, immunometric method, nephelometry, etc.

In the competitive procedure, antigen in a test fluid and labeled antigen are competitively reacted with antibody, and the unreacted labeled antigen (F) and the labeled antigen bound to the antibody (B) are separated (B/F separation) and the amount of the antigen in the test fluid is quantified by measuring the amount of the label in either B or F. This reaction procedure can be a liquid-phase procedure that uses, a soluble antibody as the antibody, polyethylene glycol for B/F separation, and a second antibody to the preceding antibody, or a solid-phase procedure that uses either an immobilized antibody as the first antibody or a soluble antibody as the first antibody and that uses immobilized antibody as the second antibody.

In the immunometric procedure, the antigen in a test fluid and immobilized antigen are competitively reacted with a prescribed amount of labeled antibody and the solid phase is separated from the liquid phase; or the antigen in the test fluid and an excess amount of labeled antibody are reacted, immobilized antigen is then added and the unreacted labeled antibody is bound to the solid phase, and the solid phase is separated from the liquid phase. The amount of antigen in the test fluid is then quantified by measuring the amount of label in either phase.

In nephelometry, the amount of insoluble precipitate produced as a result of an antigen-antibody reaction in a gel or solution is measured. When there is little antigen in the test fluid and only a small amount of precipitate is obtained, laser nephelometry using laser scattering is advantageously employed.

It is not necessary to designate particular conditions or procedures for the application of these immunological assay methods to the quantification method in the present invention. For each of these methods, a system for measuring the protein of the present invention can be constructed by the application of the usual technical considerations by the individual skilled in the art to the conventional conditions and procedures. Reference can be made to the following reviews and texts for the details of these general technical means.

Reference can be made, for example, to Hiroshi Irie, ed., Radioimmunoassay (Kodansha, published in 1974), Hiroshi Irie, ed., Sequel to Radioimmunoassay (Kodansha, published in 1979), Eiji Ishikawa et al., ed., Enzyme Immunoassay (Igakushoin, published in 1978), Eiji Ishikawa et al., ed., Enzyme Immunoassay (2nd ed.) (Igakushoin, published in 1982), Eiji Ishikawa et al., ed., Enzyme Immunoassay (3rd ed.) (Igakushoin, published in 1987), and Methods in Enzymology, Vol. 70 (Immunochemical Techniques (Part A)), *ibid*., Vol. 73 (Immunochemical Techniques (Part B)), *ibid*., Vol. 74 (Immunochemical Techniques (Part C)), *ibid*., Vol. 84 (Immunochemical Techniques (Part D: Selected Immunoassays)), *ibid*., Vol. 92 (Immunochemical Techniques (Part E: Monoclonal Antibodies and General Immunoassay Methods)), *ibid.,* Vol. 121 (Immunochemical Techniques (Part I: Hybridoma Technology and Monoclonal Antibodies))(all published by Academic Press Publishing).

Proceeding in the manner described above, protein of the present invention can be quantified at high sensitivities using antibody of the present invention.

Furthermore, when an increased concentration of protein of the present invention is detected through quantification of the concentration of protein of the present invention using antibody of the present invention, it can be diagnosed that one suffers from, for example, cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.) or it is highly likely for one to suffer from these disease in the future, and in particular it can be diagnosed that one suffers from a tyrosine kinase signaling pathway inhibitor-resistant cancer (for example, MEK inhibitor-resistant cancers, EGFR inhibitor-resistant cancers, HER2 inhibitor-resistant cancers, etc.) is present or it is highly likely for one to suffer from these disease in the future.

The antibody of the present invention can also be used to detect protein of the present invention present in a test sample, such as body fluids, tissues, etc. The antibody of the present invention can also be used to prepare an antibody column for use for the purification of protein of the present invention, to detect the protein of the present invention in the individual fractions during purification, to analyze the behavior of the protein of the present invention in cells under investigation, etc.

### (3) Genetic diagnostic agent

Through its use, for example, as a probe, the DNA of the present invention enables the detection of abnormalities (genetic abnormalities) in humans and warm-blooded animals (for example, rat, mouse, guinea pig, rabbit, fowl, sheep, pig, cow, horse, cat, dog, monkey, chimpanzee, etc.) in the DNA or mRNA that encodes the protein of the present invention or partial peptide thereof and is therefore useful, for example, as a genetic diagnostic agent, for example, of damage, mutation, or a decline in expression at the aforesaid DNA or mRNA, or an increase in the aforesaid DNA or mRNA or its overexpression.

This genetic diagnosis using DNA of the present invention can be carried out by, for example, northern hybridization or a PCR-SSCP procedure, which are publicly known *(*Genomics, Volume 5, pp. 874-879 (1989); Proceedings of the National Academy of Sciences of the United States of America, Volume 86, pp. 2766-2770 (1989)).

When, for example, overexpression is detected by northern hybridization and/or a DNA mutation is detected by PCR-SSCP, it can be diagnosed that one suffers from, for example, cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.) or it is highly likely for one to suffer from these disease in the future, and in particular it can be diagnosed that one suffers from a tyrosine kinase signaling pathway inhibitor-resistant cancer (for example, MEK inhibitor-resistant cancers, EGFR inhibitor-resistant cancers, HER2 inhibitor-resistant cancers, etc.) or it is highly likely for one to suffer from these disease in the future.

### (4) Drugs containing the antisense polynucleotide

The antisense polynucleotide of the present invention, which can bind in a complementarily to the DNA of the present invention and can thereby inhibit the expression of said DNA, exhibits, for example, an apoptosis-promoting activity on cancer cells or a proliferation-inhibiting activity on cancer cells, exhibits low toxicity, and can inhibit the in vivo function (for example, acyl-CoA synthetase activity, fatty acid transport activity, etc.) of the protein of the present invention or the DNA of the present invention. The antisense polynucleotide of the present invention can therefore be used as an agent for the prevention/treatment of cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), as a promoter of cancer cell apoptosis, or as an inhibitor of cancer cell proliferation.

Moreover, because the aforementioned antisense polynucleotide exhibits an even stronger action against tyrosine kinase signaling pathway inhibitor-resistant cancers (for example, MEK inhibitor-resistant cancers, EGFR inhibitor-resistant cancers, HER2 inhibitor-resistant cancers, etc.) and tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.), it can be very advantageously used as an agent for the prevention/treatment of tyrosine kinase signaling pathway inhibitor-resistant cancers (for example, MEK inhibitor-resistant cancers, EGFR inhibitor-resistant cancers, HER2 inhibitor-resistant cancers, etc.), as a promoter of the apoptosis of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.), or as an inhibitor of the proliferation of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.).

When the antisense polynucleotide described above is to be used as one of the above-described agents, it can be administered formulated according to publicly known methods.

For example, the antisense polynucleotide can be administered alone, or after insertion into an appropriate vector such as a retrovirus vector, adenovirus vector, adenovirus-associated virus vector, etc., by an oral or non-oral route according to the usual means to humans or mammals (for example, rat, rabbit, sheep, pig, cow, cat, dog, monkey, etc.). This antisense polynucleotide can be administered, either as such or formulated with a physiologically acceptable carrier, for example, an auxiliary to promote uptake, with a gene gun or through a catheter such as a hydrogel catheter. Or, it can be aerosolized and administered locally into the trachea as an inhalant.

Moreover, in order to improve the in vivo dynamics, extend the half-life, and improve the efficiency of uptake into the cell, the above-described antisense polynucleotide may be formulated (injectable formulation) itself or with a carrier such as a liposome and administered intravenously, subcutaneously, into a joint lumen, at the site of a cancerous lesion, etc.

The dose of the antisense polynucleotide will vary depending on the target disease, recipient, status of the disease, route of administration, etc. For example, when the antisense polynucleotide of the present invention is administered for the purpose of treating lung cancer, generally approximately 0.1 to 100 mg of the antisense polynucleotide is administered daily to an adult (60 kg body weight).

In addition, the antisense polynucleotide may also be used as a diagnostic oligonucleotide probe to investigate the presence of DNA of the present invention in tissues or cells and/or to investigate the status of the expression of DNA of the present invention in tissues or cells.

Like the above-described antisense polynucleotide, double-stranded RNA (for example, siRNA (small (short) interfering RNA), shRNA (small (short) hairpin RNA), etc. against the polynucleotide according to the present invention) containing a portion of RNA encoding the protein of the present invention, a ribozyme containing a portion of RNA encoding the protein of the present invention, etc., can also suppress expression of the gene of the present invention and can thereby suppress the in vivo function of the protein used by the present invention or the DNA used by the present invention and can as a result be used as an agent for the prevention/treatment of cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), as a promoter of cancer cell apoptosis, or as an inhibitor of cancer cell proliferation.

In addition, the aforementioned double-stranded RNA, ribozyme, etc., exhibits an even stronger action against tyrosine kinase signaling pathway inhibitor-resistant cancers (for example, MEK inhibitor-resistant cancers, EGFR inhibitor-resistant cancers, HER2 inhibitor-resistant cancers, etc.) and tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.) and can therefore be very advantageously used as an agent for the prevention/treatment of tyrosine kinase signaling pathway inhibitor-resistant cancers (for example, MEK inhibitor-resistant cancers, EGFR inhibitor-resistant cancers, HER2 inhibitor-resistant cancers, etc.), as a promoter of the apoptosis of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.), or as an inhibitor of the proliferation of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.).

The double-stranded RNA can be designed and produced in accordance with known methods (for example, Nature, Volume 411, p. 494, 2001) based on the sequence of the polynucleotide of the present invention.

The ribozyme can be designed and produced in accordance with known methods (for example, Trends in Molecular Medicine, Volume 7, p. 221, 2001) based on the sequence of the polynucleotide of the present invention. For example, it can be produced by ligating a known ribozyme to a portion of an RNA that encodes protein of the present invention. Said "portion of an RNA that encodes protein of the present invention" can be exemplified by a portion (RNA fragment) neighboring a cleavage site on an RNA of the present invention cleavable by a known ribozyme.

To use the double-stranded RNA or ribozyme described above as one of the agents cited above, the double-stranded RNA or ribozyme may be formulated and administered just as for the antisense polynucleotide.

In addition, the antisense polynucleotide described above, the double-stranded RNA described above (for example, siRNA (small (short) interfering RNA), shRNA (small (short) hairpin RNA), etc. against the polynucleotide according to the present invention), the ribozyme described above, etc., can be used in combination with a hormone therapy drug, an anticancer agent (for example, a chemotherapeutic drug, an immunotherapeutic drug, a tyrosine kinase signaling pathway inhibitor (a drug that inhibits the action of a cell growth factor and its receptor)), etc. (abbreviated below as the concomitant drug). This "concomitant drug" is the same as the "concomitant drug" described above in the section "(1) Screening of candidate drug compounds for diseases".

### (5) Drugs containing antibody of the present invention

Antibody against protein of the present invention, and preferably antibody that has the capacity to neutralize the activity of protein of the present invention, can be used, for example, as an agent for the prevention/treatment of cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), as a promoter of cancer cell apoptosis, or as an inhibitor of cancer cell proliferation.

In addition, this antibody exhibits an even stronger action against tyrosine kinase signaling pathway inhibitor-resistant cancers (for example, MEK inhibitor-resistant cancers, EGFR inhibitor-resistant cancers, HER2 inhibitor-resistant cancers, etc.) and tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.) and can therefore be very advantageously used as an agent for the prevention/treatment of tyrosine kinase signaling pathway inhibitor-resistant cancers (for example, MEK inhibitor-resistant cancers, EGFR inhibitor-resistant cancers, HER2 inhibitor-resistant cancers, etc.), as a promoter of the apoptosis of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.), or as an inhibitor of the proliferation of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.).

These agents containing antibody of the present invention and directed against the above-cited diseases exhibit a low toxicity and can be administered, as a liquid preparation or as a suitably formulated drug composition, to humans and mammals (for example, rat, rabbit, sheep, pig, cow, cat, dog, monkey, etc.) by an oral or non-oral (for example, intravenous injection) route. The dose will vary again depending on the recipient, target disease, status of the disease, route of administration, etc.; however, for the example of use to prevent/treat adult lung cancer, the antibody of the present invention is favorably administered as a single dose of generally approximately 0.01 to 20 mg/kg body weight, preferably approximately 0.1 to 10 mg/kg body weight, and more preferably approximately 0.1 to 5 mg/kg body weight, approximately 1 to 5 times a day and preferably approximately 1 to 3 times a day. Amounts corresponding to this can be administered in the case of oral administration and other non-oral routes of administration. When the disease is particularly severe, the amount may be increased in correspondence to the state of the disease.

The antibody of the present invention may be administered alone or as an appropriate pharmaceutical composition. The pharmaceutical composition used for administration comprises the aforesaid antibody or salt thereof and a pharmacologically acceptable carrier, diluent, or excipient. This composition is provided in a dosage form adapted to oral or non-oral administration (for example, intravenous injection). It is preferably provided as an inhalant.

The compositions described above may additionally contain other active components as long as no adverse interaction is produced upon blending with the above-described antibody.

In addition, the antibody of the present invention can be used in combination with a hormone therapy drug, an anticancer agent (for example, a chemotherapeutic drug, an immunotherapeutic drug, a tyrosine kinase signaling pathway inhibitor (a drug that inhibits the action of a cell growth factor and its receptor)), etc. (abbreviated below as the concomitant drug). This "concomitant drug" is the same as the "concomitant drug" described above in the section "(1) Screening of candidate drug compounds for diseases".

### (6) DNA transgenic animals

The present invention provides a non-human mammal that has exogenous DNA that encodes protein of the present invention (abbreviated below as exogenous DNA of the present invention) or a variant DNA thereof (in some instances abbreviated below as exogenous variant DNA of the present invention).

That is, the present invention provides
(1) a non-human mammal that has the exogenous DNA of the present invention or variant DNA thereof;
(2) the animal according to (1), wherein the non-human mammal is a rodent;
(3) the animal according to (2), wherein the rodent is a mouse or rat; and
(4) a recombinant vector that contains the exogenous DNA of the present invention or variant DNA thereof and that is expressible in a mammal.

The non-human mammal having the exogenous DNA of the present invention or variant DNA thereof (abbreviated below as the DNA transgenic animal of the present invention) can be produced by transfecting a desired DNA into an unfertilized egg, a fertilized egg, a spermatozoon, a germ cell including a primordial germ cell therefor, etc., preferably in the embryonic stage of the development of the non-human mammal (more preferably in the single cell or fertilized egg cell stage and generally before the 8-cell phase), by, for example, the calcium phosphate method, electric pulse method, lipofection method, agglutination method, microinjection method, particle gun method, DEAE-dextran method, etc. In addition, a desired exogenous DNA of the present invention can be transfected into a somatic cell, an organ of an organism, a tissue cell, etc., by the aforementioned DNA transfection methods, and the product can be utilized for cell culture, tissue culture, etc. These cells can also be fused with the aforementioned germ cells by a publicly known cell fusion procedure to produce the DNA transgenic animal of the present invention.

The non-human mammal can be exemplified by the cow, pig, sheep, goat, rabbit, dog, cat, guinea pig, hamster, mouse, rat, etc. Viewed from the standpoint of producing animal models of diseases, rodents that have a relatively short ontogeny and life cycle and are easy to breed are preferred thereamong, particularly mice (for example, the C57BL/6 strain, DBA2 strain, etc. for pure lines and the B6C3F₁ strain, BDF₁ strain, B6D2F₁ strain, BALB/c strain, ICR strain, etc. for cross lines), rats (for example, Wistar, SD, etc.), etc.

The "mammal" in the case of the recombinant vector expressible in a mammal can be exemplified by humans in addition to the aforesaid non-human mammals.

The exogenous DNA of the present invention refers to the DNA of the present invention once it has been isolated/extracted from a mammal and is not the DNA of the present invention natively present in a non-human mammal.

The variant DNA of the present invention encompasses variants resulting from the generation of variation (for example, mutation etc.) in the original base sequence of DNA of the present invention and specifically DNA resulting from, for example, base addition or deletion or from substitution with another base, and also encompasses abnormal DNA.

Abnormal DNA denotes DNA that causes the expression of abnormal protein of the present invention and can be exemplified by DNA that causes the expression of protein that inhibits the function of normal protein of the present invention.

The exogenous DNA of the present invention may be derived from a mammal that is of the same species as the target mammal or may be derived from a mammal that is of a species different from that of the target mammal. It is generally advantageous with regard to the transfection of the DNA of the present invention into the target animal to use a DNA construct in which said DNA is ligated downstream from a promoter capable of bringing about the expression of said DNA in animal cells. For example, in the case of the transfection of human DNA of the present invention, a DNA transgenic mammal that strongly expresses the DNA of the present invention can be produced by microinjecting the fertilized egg of the target mammal, for example, a fertilized mouse egg, with a DNA construct (for example, a vector etc.) in which the human DNA of the present invention is ligated downstream from a promoter capable of bringing about the expression of DNA and originating from a mammal (for example, the rabbit, dog, cat, guinea pig, hamster, rat, mouse, etc.) having DNA of the present invention that is highly homologous to the human DNA of the present invention.

*Escherichia* coli-derived plasmids, *Bacillus subtilis-derived* plasmids, yeast-derived plasmids, bacteriophages such as λ-phage, retroviruses such as the Moloney leukemia virus etc., and animal viruses such as the vaccinia virus, baculovirus, etc., can be used as expression vectors for the protein of the present invention. The use is preferred thereamong *of Escherichia* coli-derived plasmids, *Bacillus subtilis-derived* plasmids, and yeast-derived plasmids.

The aforementioned promoters that regulate DNA expression can be exemplified by (i) DNA promoters originating from viruses (for example, simian virus, cytomegalovirus, Moloney leukemia virus, JC virus, mammary tumor virus, poliovirus, etc.) and (ii) promoters derived from various mammals (human, rabbit, dog, cat, guinea pig, hamster, rat, mouse, etc.), for example, promoters for albumin, insulin II, uroplakin II, elastase, erythropoietin, endothelin, muscle creatine kinase, glial fibrillary acidic protein, glutathione S-transferase, platelet-derived growth factor β, keratin K1, keratin K10, keratin K14, collagen type I, collagen type II, cyclic AMP-dependent protein kinase βI subunit, dystrophin, tartrate-resistant alkaline phosphatase, atrial natriuretic factor, endothelial receptor tyrosine kinase (generally abbreviated as Tie2), sodium-potassium adenosine triphosphorylase (Na, K-ATPase), neurofilament light chain, metallothionein I, metallothionein IIA, metalloproteinase 1 tissue inhibitor, MHC class I antigen (H-2L), H-ras, renin, dopamine β-hydroxylase, thyroid peroxidase (TPO), peptide chain elongation factor 1α (EF-1α), β-actin, α-myosin heavy chain, β-myosin heavy chain, myosin light chain 1, myosin light chain 2, myelin base protein, thyroglobulin, Thy-1, immunoglobulin, H-chain variable region (VNP), serum amyloid P component, myoglobin, troponin C, smooth muscle α-actin, preproencephalin A, vasopressin, etc. Highly suitable thereamong are the cytomegalovirus promoter, human peptide elongation factor 1α (EF-1α) promoter, and the human and chicken β-actin promoters, which are capable of high expression over the whole body.

The vectors described above preferably have a sequence (generally called a terminator) that terminates the transcription of the desired messenger RNA in the DNA transgenic mammal. For example, DNA sequences of viral or mammalian origin can be used, and the SV40 terminator from the simian virus etc. is preferably used.

In addition, for the purpose of further enhancing expression of the desired exogenous DNA, a splicing signal, an enhancer region, a portion of a eukaryotic DNA intron, etc., of the particular DNA may also be ligated 5' upstream from the promoter region, between the promoter region and the translational region, or 3' downstream from the translational region, depending on purposes.

The translational region for normal protein of the present invention can be acquired as part or all of DNA originating from a human or mammalian (for example, rabbit, dog, cat, guinea pig, hamster, rat, mouse, etc.) liver, kidney, thyroid cell, or fibroblast or as part or all of the genomic DNA from various commercially available genomic DNA libraries, or using as a starting material complementary DNA prepared by a known method from RNA of liver, kidney, thyroid cell, or fibroblast origin. In addition, with regard to exogenous abnormal DNA, the translational region can be produced using a point mutagenesis procedure to introduce abnormality into a normal protein translational region obtained from the previously cited cells or tissue.

The translational region can be prepared as a DNA construct capable of being expressed in transgenic animals by conventional DNA engineering techniques of ligation downstream from a promoter as described above and, as desired, ligation upstream from a transcription termination site.

Transfection of the exogenous DNA of the present invention at the fertilized egg cell stage secures its presence in all the germ cells and somatic cells of the target mammal. The fact that the exogenous DNA of the present invention is present in the germ cells of the constructed animal post-DNA transfection means that all of the progeny of the constructed animal will maintain the exogenous DNA of the present invention in all of their germ cells and somatic cells. The offspring of this type of animal, having inherited the exogenous DNA of the present invention, will have the exogenous DNA of the present invention in all of their germ cells and somatic cells.

A non-human mammal that has been transfected with normal exogenous DNA of the present invention can, while confirming stable maintenance of the exogenous DNA by mating, be successively bred under ordinary breeding conditions as an animal retaining said DNA.

Transfection of the exogenous DNA of the present invention at the fertilized egg cell stage secures its presence in abundance in all of the germ cells and somatic cells of the target mammal. The fact that the exogenous DNA of the present invention is present in abundance in the germ cells of the constructed animal post-DNA transfection means that all of the progeny of the constructed animal will have the exogenous DNA of the present invention in abundance in all of their germ cells and somatic cells. The descendants of this type of animal, having inherited the exogenous DNA of the present invention, will have the exogenous DNA of the present invention in abundance in all of their germ cells and somatic cells.

Homozygotic animals having the transfected DNA on both homologous chromosomes can be obtained and the resulting male and female animals can be mated to give progeny that all have said DNA in abundance.

The normal DNA of the present invention exhibits a high level of expression in a non-human mammal that has the normal DNA of the present invention, and an hyperfunction disease corresponding to the protein of the present invention may ultimately be developed by promoting the function of the endogenous normal DNA and can be used as the animal model of this disease. For example, it is possible using a normal DNA transgenic animal of the present invention to elucidate the upregulated function disease corresponding to the protein of the present invention, to elucidate the pathological mechanism of diseases associated with the protein of the present invention, and to investigate methods for treating these diseases.

Moreover, since a mammal transfected with exogenous normal DNA of the present invention exhibits elevated symptoms from the liberated protein of the present invention, it can also be used in screening tests for agents for the prevention/treatment of disease associated with protein of the present invention, for example, agents for the prevention/treatment of cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), promoters of cancer cell apoptosis, or inhibitors of cancer cell proliferation; for example, for screening tests for agents for the prevention/treatment of tyrosine kinase signaling pathway inhibitor-resistant cancers (for example, MEK inhibitor-resistant cancers, EGFR inhibitor-resistant cancers, HER2 inhibitor-resistant cancers, etc.), promoters of the apoptosis of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.), or inhibitors of the proliferation of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.).

On the other hand, a non-human mammal that has abnormal exogenous DNA of the present invention can, while confirming stable maintenance of the exogenous DNA by mating, be successively bred under ordinary breeding conditions as an animal retaining said DNA. Furthermore, the exogenous DNA of interest can be utilized as a starting material by incorporation into a plasmid as described above. A DNA construct with a promoter can be constructed by conventional DNA engineering techniques. Transfection of the abnormal DNA of the present invention at the fertilized egg cell stage secures its presence in all the germ cells and somatic cells of the target mammal. The fact that the abnormal DNA of the present invention is present in the germ cells of the constructed animal post-DNA transfection means that all of the progeny of the constructed animal will have the abnormal DNA of the present invention in all of their germ cells and somatic cells. The offspring of this type of animal, having inherited the exogenous DNA of the present invention, will have the abnormal DNA of the present invention in all of their germ cells and somatic cells. Homozygotic animals having the transfected DNA on both homologous chromosomes can be obtained and the resulting male and female animals can be mated to give progeny that all have said DNA.

The abnormal DNA of the present invention exhibits a high level of expression in a non-human mammal that has the abnormal DNA of the present invention. A function inactive type inadaptability of protein of the present invention may ultimately occur through an inhibition of the function of the endogenous normal DNA and can be used as the animal model of this pathology. For example, it is possible using an abnormal DNA transgenic animal of the present invention to elucidate the pathological mechanism of a functional deactivation-type insensitivity disease of protein of the present invention and to investigate methods for treating this disease.

As a specific possibility for utilization, an animal expressing the abnormal DNA of the present invention at high levels provides a model for elucidating the functional inhibition (dominant negative action) of normal protein by the abnormal protein of the present invention in a functional deactivation-type insensitivity disease of protein of the present invention.

In addition, a mammal transfected with the abnormal exogenous DNA of the present invention can also be used in screening tests for agents for the prevention/treatment of function inactive type inadaptability of protein of the present invention, for example, agents for the prevention/treatment of cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), promoters of cancer cell apoptosis, or inhibitors of cancer cell proliferation; for example, for screening tests for agents for the prevention/treatment of tyrosine kinase signaling pathway inhibitor-resistant cancers (for example, MEK inhibitor-resistant cancers, EGFR inhibitor-resistant cancers, HER2 inhibitor-resistant cancers, etc.), promoters of the apoptosis of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.), or inhibitors of the proliferation of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.).

Examples of other potential applications that are contemplated for the two types of DNA transgenic animals of the present invention described above are as follows:
(i) use as a source of cells for tissue culture;
(ii) elucidation of the relationship to peptide that is specifically expressed or activated by the protein of the present invention, by direct analysis of the DNA or RNA in the tissues of the DNA transgenic animal of the present invention or by analysis of the peptide tissue expressed by the DNA;
(iii) research on the function of cells from tissues generally refractory to culture, by culturing cells of the DNA-bearing tissue by standard tissue culture techniques and using these;
(iv) screening for drugs that enhance cell function, using the cells described in (iii) above; and,
(v) isolation and purification of variant protein of the present invention and preparation of antibodies therefor.

The DNA transgenic animals of the present invention can also be used to examine the clinical symptoms of diseases related to the protein of the present invention, including function inactive type inadaptability of protein of the present invention. The pathological findings on the individual organs in a disease model associated with the protein of the present invention can also be obtained in greater detail, which can lead to the development of new treatment methods as well as to research on and therapies for secondary diseases associated with this disease.

It will also be possible to acquire free DNA-transfected cells by removing an organ from a DNA transgenic animal of the present invention, mincing the organ, and degrading with a proteinase such as, for example, trypsin; these cells can then be cultured or a line can be established from the cultured cells. Moreover, the DNA transgenic animal of the present invention can be used to identify cells capable of producing the protein of the present invention, to study the relationship with apoptosis, differentiation, or proliferation, and to study the mechanisms of signal transduction in these processes and to study abnormalities therein, and is thus an effective research tool with respect to the protein of the present invention and for elucidating the action of the protein of the present invention.

In order to develop drugs for the treatment of diseases associated with the protein of the present invention, including function inactive type inadaptability of protein of the present invention, the DNA transgenic animal of the present invention can be used to set up an effective and rapid method for screening for drugs for treating these diseases, using, inter alia, the examination and quantification methods described above. DNA therapies for diseases associated with the protein of the present invention can also be investigated and developed using the DNA transgenic animal of the present invention or an expression vector for the exogenous DNA of the present invention.

### (7) Knockout animals

The present invention provides non-human mammal embryonic stem cells in which the DNA of the present invention has been inactivated and non-human mammals in which expression of the DNA of the present invention is deficient.

That is, the present invention provides
(1) a non-human mammal embryonic stem cell in which the DNA of the present invention is inactivated;
(2) the embryonic stem cell according to (1), in which the DNA has been inactivated by the insertion of a reporter gene (for example, a β-galactosidase gene derived from *Escherichia coli);*
(3) the embryonic stem cell according to (1), that is resistant to neomycin;
(4) the embryonic stem cell according to (1), wherein the non-human mammal is a rodent;
(5) the embryonic stem cell according to (4), wherein the rodent is the mouse;
(6) a DNA of the present invention expression-deficient non-human mammal in which the DNA of the present invention is inactivated;
(7) the non-human mammal according to (6), wherein the DNA has been inactivated by the insertion of a reporter gene (for example, a β-galactosidase gene derived from *Escherichia coli)* and the reporter gene can be expressed under control by the promoter for the DNA of the present invention;
(8) the non-human mammal according to (6), wherein the non-human mammal is a rodent;
(9) the non-human mammal according to (8), wherein the rodent is the mouse; and,
(10) a method of screening for a compound or its salt that promotes or inhibits promoter activity relative to the DNA of the present invention, comprising administering a test compound to the animal according to (7) and detecting expression of the reporter gene.

The non-human mammal embryonic stem cell in which the DNA of the present invention is inactivated refers to the embryonic stem cells (abbreviated as ES cells below) of a non-human mammal whose DNA substantially does not have the capacity to express protein of the present invention (in some instances referred to below as the knockout DNA of the present invention), as brought about by the introduction of an artificial mutation in the DNA of the present invention present in the non-human mammal, resulting in an inhibition of the DNA's expression capability or causing the activity of the protein of the present invention that this DNA encodes to be substantially lost.

The same non-human mammals as described above can be used here.

The procedure for introducing an artificial mutation into the DNA of the present invention can be exemplified by the deletion of a portion or all of the aforesaid DNA sequence and the insertion of or substitution with other DNA, as executed by genetic engineering techniques. The knockout DNA of the present invention may be prepared by these mutations, for example, by shifting the codon reading frame or by disrupting the function of a promoter or exon.

A specific example of a DNA of the present invention-inactivated non-human mammal embryonic stem cell (abbreviated below as a DNA of the present invention-inactivated ES cell or a knockout ES cell of the present invention) is the knockout ES cell of the present invention obtained, for example, by isolating the DNA of the present invention that is present in a non-human mammal of interest; inserting into the chromosomes of the animal, for example, by homologous recombination, a DNA strand (abbreviated below as the targeting vector) that has a DNA sequence constructed so as to disrupt exon function by the insertion, into the exon region of the DNA of the present invention, of a drug-resistance gene, typically a neomycin-resistance gene or a hygromycin-resistance gene, or a reporter gene, typically lacZ β-galactosidase gene) or cat (chloramphenicol acetyltransferase gene), or constructed so as to disrupt the gene by disabling the synthesis of a complete messenger RNA through the insertion, in an interexon intron region, of a DNA sequence that terminates gene transcription (for example, a polyA additional signal etc.); and subjecting the resulting ES cells to Southern hybridization analysis using a DNA sequence on or near the DNA of the present invention as a probe, or to PCR analysis using as probes a DNA sequence on the targeting vector and a DNA sequence in a nearby region outside the DNA of the present invention used to construct the targeting vector, in order to select the knockout ES cell of the present invention.

The original ES cells for deactivation of the DNA of the present invention by, for example, homologous recombination, may be, for example, already established as described above or may be established de novo in accordance with the known method of Evans and Kaufman. For example, in the case of mouse ES cells, it is currently common practice to use ES cells from the 129 strain. However, because their immunological background is not well defined, with the goal of acquiring in place thereof ES cells with a clear immunogenetic background as a pure line etc., ES cells are also advantageously used that have been established using the C57BL/6 mouse or the BDF₁ mouse (F₁ from C57BL/6 × DBA/2), in which the low egg collection count of C57BL/6 has been improved by crossing with DBA/2. The BDF₁ mouse offers the advantages of a high egg collection count and robust ova and, because it has the C57BL/6 mouse background, ES cells obtained using it are advantageous for use in that their genetic background can be converted to the C57BL/6 mouse by backcrossing with the C57BL/6 mouse when a mouse disease model is constructed.

Blastocytes at 3.5 days after fertilization are generally used when establishing ES cells, but in addition a large number of early-stage embryos can be efficiently acquired by collecting 8-cell stage embryos and culturing them to blastocyctes.

While ES cells of either sex may be used, male ES cells are generally more convenient for constructing a germ line chimera. In addition, the sexes are desirably distinguished as soon as possible in order to also reduce the time and effort required by the complex culture process.

An example of a method for determining the sex of ES cells is the amplification and detection of genes in the sex-determining region on the Y-chromosome by the PCR process. Since the number ofES cells in approximately 1 colony (approximately 50 cells) is sufficient when using this method, against the requirement for approximately 10⁶ cells in the heretofore used karyotype analysis, it enables primary selection by sex identification of the ES cells at an early stage of culture, thereby enabling a substantial lessening of the time and effort in the early stage of culture by making the early selection of male cells possible.

A secondary selection can be carried out, for example, by verifying the number of chromosomes by the G-banding method. The number of chromosomes in the obtained ES cells is desirably 100% of the normal number; however, in the event of problems during establishment related to, for example, physical operations etc., recloning into normal cells (for example, cells with a chromosome number 2n = 40 for the mouse) is desirable after the gene in the ES cell has been knocked out.

The embryonic stem cell line thus obtained generally has very good growth properties, but must be serially cultured with great care because it readily loses its ontogenic capabilities. For example, culture is carried out at approximately 37°C in a carbon dioxide incubator (preferably 5% carbon dioxide and 95% air, or 5% oxygen, 5% carbon dioxide, and 90% air) in the presence of LIF (1 to 10000 U/mL) on appropriate feeder cells, such as STO fibroblasts. For passage, for example, processing to single cells is carried out by treatment with a trypsin/EDTA solution (generally 0.001 to 0.5% trypsin/0.1 to 5 mM EDTA, preferably approximately 0.1% trypsin/1 mM EDTA), followed by seeding onto freshly prepared feeder cells. This passage is normally conducted every 1 to 3 days, and at this time the cells are desirably inspected and, if morphologically abnormal cells are found, these cultured cells are discarded.

ES cells can be differentiated into various types of cells, for example, parietal muscle, visceral muscle, cardiac muscle, etc., by monolayer culture under appropriate conditions to high density or by suspension cultivation under appropriate conditions up to the formation of cell aggregates (M.J. Evans and M. H. Kaufman, Nature, Volume 292, p. 154, 1981; G. R. Martin, Proc. Natl. Acad. Sci. U.S.A., Volume 78, p. 7634, 1981; T. C. Doetschman et al., Journal of Embryology and Experimental Morphology, Volume 87, p. 27, 1985). The DNA of the present invention expression-deficient cells obtained by differentiation of the ES cells of the present invention are useful for in vitro cell biology investigations of the protein of the present invention.

The DNA of the present invention expression-deficient non-human mammal can be distinguished from normal animals by indirectly comparing expression levels by measuring the mRNA level in the animal using a known method.

The same non-human mammals as described above can be used as the non-human mammal here.

Knock out of the DNA of the present invention can be carried out to give a DNA of the present invention expression-deficient non-human mammal by, for example, transfection of a targeting vector constructed as described above into mouse embryonic stem cells or mouse egg cells and, through genetic homologous recombination, homologous recombination of the DNA of the present invention-inactivated DNA sequence of the transfected targeting vector in place of the DNA of the present invention on the chromosomes of the mouse embryonic stem cells or mouse egg cells.

Cells in which the DNA of the present invention has been knocked out can be identified by Southern hybridization analysis using a DNA sequence on or near the DNA of the present invention as the probe, or by PCR analysis using as primers a DNA sequence on the targeting vector and a mouse-derived DNA sequence in a nearby region outside the DNA of the present invention and used in the targeting vector. When non-human mammal embryonic stem cells are used, the cell line in which the DNA of the present invention has been inactivated by homologous recombination is cloned; the resulting cells are injected into a non-human mammal embryo or blastocyst at an appropriate stage, for example, at the 8-cell stage; and the resulting chimeric embryos are transplanted into the uterus of the pseudopregnant non-human mammal. The resulting animal is a chimeric animal constructed from both cells that have the normal locus of the DNA of the present invention and cells that have the artificially mutated locus of the DNA of the present invention.

When some germ cells of this chimeric animal have the mutated DNA locus of the present invention, individuals in which all the tissues thereof are composed of cells having the artificially mutated DNA locus of the present invention can be obtained by selection, for example, by identification of the coat color, from the group of individuals obtained by crossing this chimeric individual with a normal individual. The individuals thus obtained are generally heterozygous for deficient expression of the protein of the present invention. Individuals homozygous for deficient expression of the protein of the present invention can be obtained from the offspring of a cross between individuals heterozygous for deficient expression of the protein of the present invention.

When egg cells are used, for example, transgenic non-human mammals having the targeting vector introduced into the chromosomes can be obtained by injecting a DNA solution by microinjection into the egg cell nucleus, and animals in which there is a mutation in the DNA locus of the present invention due to genetic homologous recombination are obtained by selection from these transgenic non-human mammals.

Individuals in which the DNA of the present invention has been knocked out proceeding as described above can, while confirming that this DNA is also knocked out in the animals obtained by crossing, be successively bred under ordinary breeding conditions.

In addition, the germ line can also be acquired and maintained by the usual methods. That is, homozygous animals having the inactivated DNA on both homologous chromosomes can be obtained by crossing male and female animals that each hold the inactivated DNA. The obtained homozygous animals can be efficiently obtained by breeding under conditions so as to give one normal individual and a plurality of homozygotes per female parent animal. Heterozygous animals and homozygous animals having the inactivated DNA are bred by crossing male and female heterozygous animals.

The non-human mammal embryonic stem cells in which the DNA of the present invention is inactivated are very useful for constructing DNA of the present invention expression-deficient non-human mammals.

Since a DNA of the present invention expression-deficient non-human mammal exhibits a loss of the various biological activities inducible by the protein of the present invention, it can be a model for diseases caused by inactivation of the biological activity of protein of the present invention and is therefore useful for elucidating the causes of these diseases and studying their treatment.

### (7a) Method for screening for compounds that have a preventive/treatment activity for diseases caused by, for example, the loss of or damage to the DNA of the present invention

DNA of the present invention expression-deficient non-human mammals can be employed for screening for compounds that have a preventive/treatment effect on diseases caused by, for example, the loss of or damage to the DNA of the present invention.

That is, the present invention provides a method for screening for compounds or salts thereof that have a preventive/treatment effect on diseases caused by, for example, the loss of or damage to the DNA of the present invention, for example, cancer, said method comprising administering a test compound to a DNA of the present invention expression-deficient non-human mammal and observing/measuring changes in the animal.

The DNA of the present invention expression-deficient non-human mammal used in this screening method can be exemplified by the same DNA of the present invention expression-deficient non-human mammals described above.

The test compound can be exemplified by peptides, proteins, antibodies, nonpeptide compounds, synthetic compounds, fermentation products, cell extracts, plant extracts, animal tissue extracts, blood plasma, etc., and this compound may be a novel compound or a known compound. The test compound may be in the form of a salt, and these test compound salts may be the same test compound salts as used in, for example, the above-described methods for screening candidate drug compounds for diseases.

In specific terms, the preventive/treatment effects of a test compound can be tested by treating a DNA of the present invention expression-deficient non-human mammal with the test compound and noting changes in, for example, the organs, tissues, disease symptoms, etc. of the test animal in comparison to the untreated control animal.

The procedure for treating a test animal with a test compound can be exemplified by oral administration, intravenous injection, etc., and is selected as appropriate in accordance with the disease state of the test animal, the properties of the test compound, etc. The dose of the test compound can be selected as appropriate in view of the method f administration, the properties of the test compound, etc.

When screening, for example, for a compound that has a preventive/treatment effect on cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), the test compound is administered to a DNA of the present invention expression-deficient non-human mammal and differences in the degree of cancer development and/or differences in the degree of cancer amelioration relative to the group not receiving the test compound are monitored in the above-described tissues with elapsed time.

A test compound that provides at least an approximately 10% improvement, preferably at least an approximately 30% improvement, and more preferably at least an approximately 50% improvement in the aforesaid disease symptoms of the test animal upon administration of the test compound to the test animals in the screening method under consideration can be selected as a compound that has a preventive/treatment effect on a disease as described above.

A compound obtained using this screening method is a compound selected from the test compounds described above, and, because it exhibits a preventive/treatment effect on diseases caused by, for example, the loss of or damage to protein of the present invention, can be used as a safe, low-toxicity drug for the prevention/treatment of such diseases. Furthermore, a compound derived from a compound obtained by this screening method can also be used in the same manner.

A compound obtained by the screening method under consideration may be in the form of a salt, and the salts of this compound may be the same salts as described above for the test compound salts.

A drug containing a compound obtained by the screening method under consideration, or the salt of such a compound, can be produced in the same manner as described above for drugs containing the protein of the present invention.

Formulations obtained proceeding in the described manner are safe and low toxic and can therefore be administered to, for example, humans or mammals (for example, the rat, mouse, guinea pig, rabbit, sheep, pig, cow, horse, cat, dog, monkey, etc.).

The dose of the compound or its salt will vary depending upon the target disease, recipient, state of the disease, route of administration, etc. For example, when the compound is administered orally in order to treat breast cancer, the compound is generally administered to an adult (60 kg body weight) at a daily dose of approximately 0.1 to 100 mg, preferably approximately 1.0 to 50 mg, and more preferably approximately 1.0 to 20 mg. In the case of non-oral administration, the dose of the compound will vary depending upon the target disease, recipient, state of the disease, route of administration, etc. For example, when the compound is administered as an injectable in order to treat breast cancer, the compound is generally advantageously administered to an adult (60 kg body weight) at a daily dose of approximately 0.01 to 30 mg, preferably approximately 0.1 to 20 mg, and more preferably approximately 0.1 to 10 mg by intravenous injection. For other animal species, an amount calculated per 60 kg can be administered.

In addition, the compound under consideration can be used in combination with a hormone therapy drug, an anticancer agent (for example, a chemotherapeutic drug, an immunotherapeutic drug, a tyrosine kinase signaling pathway inhibitor (a drug that inhibits the action of a cell growth factor and its receptor)), etc. (abbreviated below as the concomitant drug). This "concomitant drug" is the same as the "concomitant drug" described above in the section "(1) Screening of candidate drug compounds for diseases".

### (7b) Method of screening for compounds that promote or inhibit promoter activity with respect to the DNA of the present invention

The present invention provides a method for screening for a compound or its salt that promotes or inhibits promoter activity with respect to the DNA of the present invention, comprising administering a test compound to a DNA of the present invention expression-deficient non-human mammal and detecting the expression of a reporter gene.

The DNA of the present invention expression-deficient non-human mammal used in this screening method is a DNA of the present invention expression-deficient non-human mammals as described above in which the DNA of the present invention has been inactivated by the introduction of a reporter gene and in which this reporter gene can be expressed under the control of the promoter for the DNA of the present invention.

The test compound can be exemplified by the same test compounds as cited above.

The same reporter genes as described above are used as the reporter gene here, and the β-galactosidase gene (lacZ), soluble alkaline phosphatase gene, luciferase gene, etc., are very suitable.

Since the reporter gene is present under the control of the promoter for the DNA of the present invention in the DNA of the present invention expression-deficient non-human mammal in which the DNA of the present invention has been substituted by the reporter gene, the activity of the promoter can be detected by tracking the expression of the substance encoded by the reporter gene.

For example, when a portion of the DNA region encoding the protein of the present invention is replaced with the β-galactosidase gene (lacZ) derived from *Escherichia coli,* β-galactosidase is expressed rather than the protein of the present invention in those tissues where the protein of the present invention would be natively expressed. As a consequence, an expression map of the protein of the present invention within the animal can be readily observed by staining with a reagent, for example, 5-bromo-4-chloro-3-indolyl-β-galactopyranoside (X-gal), that is substrate for β-galactosidase. Specifically, a protein of the present invention-deficient mouse or a tissue section therefrom is fixed with, for example, glutaraldehyde; after washing with phosphate buffered saline (PBS), a reaction is carried out at room temperature or about 37°C for approximately 30 minutes to 1 hour with a staining solution containing X-gal; the β-galactosidase reaction is stopped by washing the tissue preparation with 1 mM EDTA/PBS solution; and the color formed is observed. In addition, mRNA encoding lacZ may be detected according to the usual methods.

A compound or its salt obtained using the above-described screening method is a compound selected from the test compounds described above and is a compound that promotes or inhibits the promoter activity for the DNA of the present invention.

A compound obtained by the screening method under consideration may be in the form of a salt, and the salts of this compound may be the same salts as described above for the test compound salts used in the above-described method for screening candidate drug compounds for diseases.

A compound or its salt that promotes or inhibits the promoter activity for the DNA of the present invention can modulate the expression of protein of the present invention and can thereby modulate the function of this protein and is therefore useful as, for example, an agent for the prevention/treatment of cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), a promoter of cancer cell apoptosis, or an inhibitor of cancer cell proliferation.

In addition, such a compound or its salt exhibits an even stronger action against, for example, tyrosine kinase signaling pathway inhibitor-resistant cancers (for example, MEK inhibitor-resistant cancers, EGFR inhibitor-resistant cancers, HER2 inhibitor-resistant cancers, etc.) and tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.) and is therefore particularly useful as a drug such as an agent for the prevention/treatment of tyrosine kinase signaling pathway inhibitor-resistant cancers (for example, MEK inhibitor-resistant cancers, EGFR inhibitor-resistant cancers, HER2 inhibitor-resistant cancers, etc.), a promoter of the apoptosis of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.), and an inhibitor of the proliferation of tyrosine kinase signaling pathway inhibitor-resistant cancer cells (for example, MEK inhibitor-resistant cancer cells, EGFR inhibitor-resistant cancer cells, HER2 inhibitor-resistant cancer cells, etc.).

A compound derived from a compound obtained by the screening method described above can also be used in the same manner.

A drug comprising a compound or its salt obtained by this screening method can be produced in the same manner as the above-described drug comprising protein of the present invention or salt thereof.

Formulations obtained proceeding in the described manner are safe and low toxic and can therefore be administered, for example, to humans or mammals (for example, the rat, mouse, guinea pig, rabbit, sheep, pig, cow, horse, cat, dog, monkey, etc.).

The dose of the compound or its salt will vary depending upon the target disease, recipient, state of the disease, route of administration, etc. For example, when a compound that inhibits promoter activity for the DNA of the present invention is administered orally in order to treat breast cancer, the compound is generally administered to an adult (60 kg body weight) at a daily dose of approximately 0.1 to 100 mg, preferably approximately 1.0 to 50 mg, and more preferably approximately 1.0 to 20 mg. In the case of non-oral administration, the dose of the compound will vary depending upon the target disease, recipient, state of the disease, route of administration, etc. For example, when a compound that inhibits promoter activity for the DNA of the present invention is administered as an injectable in order to treat breast cancer, the compound is generally advantageously administered to an adult (60 kg body weight) at a daily dose of approximately 0.01 to 30 mg, preferably approximately 0.1 to 20 mg, and more preferably approximately 0.1 to 10 mg by intravenous injection. For other animal species, an amount calculated per 60 kg can be administered.

A DNA of the present invention expression-deficient non-human mammal such as this is extremely useful for screening for compounds or salts thereof that promote or inhibit the promoter activity for the DNA of the present invention and can substantially contribute to an elucidation of the causes of various diseases originating with a deficiency in the expression of the DNA of the present invention and to the development of preventive/treatment agents for these diseases.

Moreover, the construction of a so-called transgenic animal (gene transferred animal) using DNA containing the promoter region for protein of the present invention, ligating a gene encoding any of various proteins downstream therefrom, and injecting this into the egg cell of an animal, also enables the specific synthesis of the particular protein and the investigation of its function in vivo. When an appropriate reporter gene is ligated to the subject promoter region and a cell line expressing same is established, this can be used as a system for investigating low molecular weight compounds that have the ability to specifically promote or inhibit the in vivo production capacity of the protein of the present invention.

In addition, the compound under consideration can be used in combination with a hormone therapy drug, an anticancer agent (for example, a chemotherapeutic drug, an immunotherapeutic drug, a tyrosine kinase signaling pathway inhibitor (a drug that inhibits the action of a cell growth factor and its receptor)), etc. (abbreviated below as the concomitant drug). The same "concomitant drugs" as described above in the section "(1) Screening of candidate drug compounds for diseases" can be likewise used as the "concomitant drug" under consideration here.

### (8) "A cancer preventive/treatment agent comprising a compound or its salt that inhibits the activity of acyl-CoA synthetase" and "a cancer cell apoptosis promoter or cancer cell proliferation inhibitor comprising a compound or its salt that inhibits the activity of acyl-CoA synthetase"

The "compound that inhibits the activity of acyl-CoA synthetase" in the "cancer preventive/treatment agent comprising a compound or its salt that inhibits the activity of acyl-CoA synthetase" and the "cancer cell apoptosis promoter or cancer cell proliferation inhibitor comprising a compound or its salt that inhibits the activity of acyl-CoA synthetase" may be any substance (for example, a peptide, protein, antibody, nonpeptide compound, synthetic compound, fermentation product, cell extract, plant extract, animal tissue extract, blood plasma, etc.) that inhibits the activity of acyl-CoA synthetase.

The "compound that inhibits the expression of an acyl-CoA synthetase gene" in a "cancer preventive/treatment agent comprising a compound or its salt that inhibits the expression of an acyl-CoA synthetase gene" and a "cancer cell apoptosis promoter or cancer cell proliferation inhibitor comprising a compound or its salt that inhibits the expression of an acyl-CoA synthetase gene" may be any substance (for example, a peptide, protein, antibody, nonpeptide compound, synthetic compound, fermentation product, cell extract, plant extract, animal tissue extract, blood plasma, etc.) that inhibits the expression of an acyl-CoA synthetase gene.

The aforementioned agents can be formulated according to conventional means.

In addition, the aforementioned compounds can be used in combination with a hormone therapy drug, an anticancer agent (for example, a chemotherapeutic drug, an immunotherapeutic drug, a tyrosine kinase signaling pathway inhibitor (a drug that inhibits the action of a cell growth factor and its receptor)), etc. (abbreviated below as the concomitant drug). The same "concomitant drugs" as described above in the section "(1) Screening of candidate drug compounds for diseases" can be likewise used as the "concomitant drug" under consideration here.

### (9) "A cancer preventive/treatment agent comprising a compound or its salt that inhibits the activity of a fatty acid transporter protein" and "a cancer cell apoptosis promoter or cancer cell proliferation inhibitor comprising a compound or its salt that inhibits the activity of a fatty acid transporter protein"

The "compound that inhibits the activity of a fatty acid transporter protein" in the "cancer preventive/treatment agent comprising a compound or its salt that inhibits the activity of a fatty acid transporter protein" and the "cancer cell apoptosis promoter or cancer cell proliferation inhibitor comprising a compound or its salt that inhibits the activity of a fatty acid transporter protein" may be any substance (for example, a peptide, protein, antibody, nonpeptide compound, synthetic compound, fermentation product, cell extract, plant extract, animal tissue extract, blood plasma, etc.) that inhibits the activity of a fatty acid transporter protein (for example, fatty acid transport activity).

The "compound that inhibits the expression of a gene for a fatty acid transporter protein" in a "cancer preventive/treatment agent comprising a compound or its salt that inhibits the expression of a gene for a fatty acid transporter protein" and a "cancer cell apoptosis promoter or cancer cell proliferation inhibitor comprising a compound or its salt that inhibits the expression of a gene for a fatty acid transporter protein" may be any substance (for example, a peptide, protein, antibody, nonpeptide compound, synthetic compound, fermentation product, cell extract, plant extract, animal tissue extract, blood plasma, etc.) that inhibits the expression of a gene for a fatty acid transporter protein.

The aforementioned agents can be formulated according to conventional means.

In addition, the aforementioned compounds can be used in combination with a hormone therapy drug, an anticancer agent (for example, a chemotherapeutic drug, an immunotherapeutic drug, a tyrosine kinase signaling pathway inhibitor (a drug that inhibits the action of a cell growth factor and its receptor)), etc. (abbreviated below as the concomitant drug). The same "concomitant drugs" as described above in the section "(1) Screening of candidate drug compounds for diseases" can be likewise used as the "concomitant drug" under consideration here.

In this Specification and the sequence listings, abbreviations for the bases, amino acids, etc., where used, are based on the abbreviations according to the IUPAC-IUB Commission on Biochemical Nomenclature or the common abbreviations in the pertinent field, and examples are provided below. Where optical isomers are possible for an amino acid, the L form is shown unless otherwise indicated.
DNA: deoxyribonucleic acid
cDNA: complementary deoxyribonucleic acid
A: adenine
T: thymine
G: guanine
C: cytosine
RNA: ribonucleic acid
mRNA: messenger ribonucleic acid
dATP: deoxyadenosine triphosphate
dTTP: deoxythymidine triphosphate
dGTP: deoxyguanosine triphosphate
dCTP: deoxycytidine triphosphate
ATP: adenosine triphosphate
EDTA: ethylenediaminetetraacetic acid
SDS: sodium dodecyl sulfate
Gly: glycine
Ala: alanine
Val: valine
Leu: leucine
Ile: isoleucine
Ser: serine
Thr: threonine
Cys: cysteine
Met: methionine
Glu: glutamic acid
Asp: aspartic acid
Lys: lysine
Arg: arginine
His: histidine
Phe: phenylalanine
Tyr: tyrosine
Trp: tryptophan
Pro: proline
Asn: asparagine
Gln: glutamine
pGlu: pyroglutamic acid
Sec: selenocysteine

The frequently cited substituents, protecting groups, and reagents in this Specification are indicated by the following designations.
Me: methyl
Et: ethyl
Bu: butyl
Ph: phenyl
TC: thiazolidine-4(R)-carboxamido group
Tos: p-toluenesulfonyl
CHO: formyl
Bzl: benzyl
Cl₂-Bzl: 2,6-dichlorobenzyl
Bom: benzyloxymethyl
Z: benzyloxycarbonyl
Cl-Z: 2-chlorobenzyloxycarbonyl
Br-Z: 2-bromobenzyloxycarbonyl
Boc: t-butoxycarbonyl
DNP: dinitrophenol
Trt: trityl
Bum: t-butoxymethyl
Fmoc: N-9-fluorenylmethoxycarbonyl
HOBt: 1-hydroxybenztriazole
HOOBt: 3,4-dihydro-3-hydroxy-4-oxo-1,2,3-benzotriazine
HONB: 1-hydroxy-5-norbornene-2,3-dicarboxyimide
DCC: N,N'-dicyclohexylcarbodiimide

The sequence identification numbers in the sequence listing of the Specification of this application denote the following sequences.
[SEQ ID NO: 1]
   This shows the amino acid sequence of human SLC27A2.
[SEQ ID NO: 2]
   This shows the base sequence of cDNA encoding human SLC27A2.
[SEQ ID NO: 3]
   This shows the amino acid sequence of mouse SLC27A2.
[SEQ ID NO: 4]
   This shows the base sequence of cDNA encoding mouse SLC27A2.
[SEQ ID NO: 5]
   This shows the amino acid sequence of rat SLC27A2.
[SEQ ID NO: 6]
   This shows the base sequence of cDNA encoding rat SLC27A2.
[SEQ ID NO: 7]
   This shows the base sequence of the siRNA-1 used in Example 1.
[SEQ ID NO: 8]
   This shows the base sequence of the siRNA-2 used in Example 1.
[SEQ ID NO: 9]
   This shows the base sequence of the nonsilencing oligonucleotide used in Example 1.

### EXAMPLES

The invention is described more specifically by the examples that follow, but this invention is not limited to these examples.

### EXAMPLE 1

### Inhibition of cancer cell proliferation by SLC27A2 gene knockdown

The human lung cancer cell line VMRC-LCD (purchased from the Japanese Collection of Research Bioresources (JCRB)) was inoculated at a concentration of 2 × 10³/well into a 96-well plate and cultured overnight at 37°C under 5% carbon dioxide on Dulbecco's Modified Eagle Medium (DMEM) containing 10% fetal bovine serum and was then used for an experiment on the inhibition of SLC27A2 expression by RNA interference (RNAi).

The siRNA-1 (SEQ ID NO: 7) and siRNA-2 (SEQ ID NO: 8) used to inhibit the expression of SLC27A2 were purchased from Dharmacon. The nonsilencing oligonucleotide (UUCUCCGAACGUGUCACGUdTdT: SEQ ID NO: 9) used as the negative control was purchased from Qiagen. The reagent Lipofectamine 2000 (Invitrogen) was used for cell transfection. 0.1 µL of the Lipofectamine 2000 reagent and siRNA-1, siRNA-2, or the nonsilencing oligonucleotide, in each case to a final concentration of 50 µM, were added to 20 µL Opti-MEM (Invitrogen), and, after standing at quiescence for 20 minutes at room temperature, this mixed solution was added to the aforementioned cells being cultured on 100 µL culture medium.

The RNA was extracted from the cells 24 hours after the introduction of the siRNA into the cells, and the gene knockdown effect was confirmed by quantitative RT-PCR. RNA extraction was carried out using an RNeasy minikit (Qiagen). Using the extracted RNA as template, cDNA was synthesized from random primers using TaqMan Reverse Transcription Reagents (Applied Biosystems). PCR was run on the synthesized cDNA according to the included instructions using an Assays-on-Demand Gene Expression Assay Mix (Applied Biosystems) to quantify the SLC27A2 expression level, and the PCR product was quantified using an ABI PRISM 7700 Sequence Detection System.

It was found as a result that siRNA-1 and siRNA-2 produced an at least 80% inhibition of the expression of the gene for SLC27A2.

With regard to cancer cell proliferation, the number of cells was measured by the Sulforhodamine B method (Skehan et al., J. Natl. Cancer Inst., Volume 82, pp. 1107-1112, 1990). Each of the siRNAs was introduced under the conditions given above, and after 5 days the cells were fixed with glutaraldehyde at a final concentration of 2.5%. The cells were then washed once with phosphate-buffered saline (PBS) and were then further fixed with 10% trichloroacetic acid/PBS by holding for at least 1 hour at 4°C. After the cells had been washed with water, 50 µL of a Sulforhodamine B solution (prepared by the addition to a 1% acetic acid solution of Sulforhodamine B so as to give 4%) was added followed by holding for 15 minutes at room temperature. The Sulforhodamine B solution was then removed; the cells were washed 3 times with 1% acetic acid solution; 100 µL 10 mM Tris solution was added to these cells; and the amount of eluted Sulforhodamine B was measured by the absorbance at 549 nm.

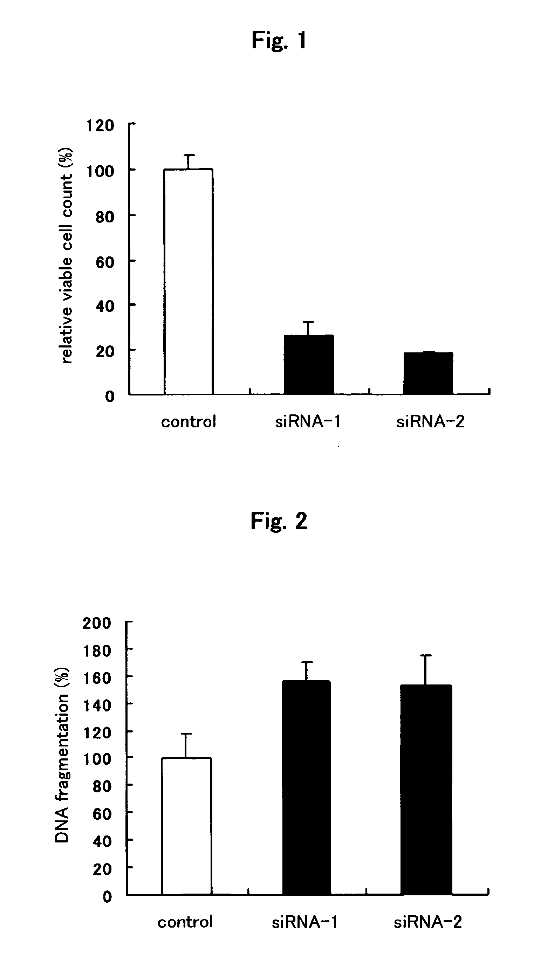

The results are shown in Figure 1.

Based on Figure 1, it is shown that the viable cell count after cells transfected with siRNA against SLC27A2 had been cultured for 5 days was reduced approximately 80% in comparison to the cell count for the nonsilencing oligonucleotide-transfected control. It was thereby confirmed that siRNA against SLC27A2 has a cell proliferation inhibiting activity on cancer cells.

### EXAMPLE 2

### Promotion of apoptosis by SLC27A2 gene knockdown

siRNA transfection was carried out by the method described in Example 1, and the promotion of cell apoptosis at the second day was measured through the amount of DNA fragmentation in the cells.

Measurement was carried out using a Cell Death Detection Kit (Roche). The siRNA-transfected cells were lysed with the supplied cell lysis buffer and the lysis solution was transferred to a streptavidin-coated microtiter plate. A biotin-labeled anti-histone antibody and a peroxidase-labeled anti-DNA antibody were added to the lysis solution and reacted with the intracellular mononucleosomes or oligonucleosomes produced by apoptosis. After eliminating the nonspecific adsorption by washing three times with the supplied washing solution, ABTS (2,2'-azino-di[3-ethylbenzthiazolinsulfonate]), a substrate for the peroxidase, was added and reacted and the absorbance of the reaction solution at 405 nm was measured.

The results are shown in Figure 2.

Based on Figure 2, it is shown that the amount of DNA fragmentation for cells transfected with siRNA against SLC27A2 was increased by about 150% in comparison to the amount of DNA fragmentation for the nonsilencing oligonucleotide-transfected control. It was thereby confirmed that siRNA against SLC27A2 has an apoptosis promoting action on cancer cells.

### EXAMPLE 3

### (1) Comparison of MEK inhibitor sensitivity in human lung cancer cell lines

The cell proliferation inhibiting effect due to the MEK inhibitor U0126 (available from BIOMOL Research Laboratories) was investigated in the following human lung cancer cell lines: VMRC-LCD cells (described in Example 1), NCI-H23 cells (purchased from the American Type Culture Collection (ATCC)), NCI-H520 cells (purchased from the ATCC), and NCI-H522 cells (purchased from the ATCC).

Each of the cells was inoculated into 96-well plates at a concentration of 2 × 10³/well and cultured overnight at 37°C under 5% carbon dioxide on DMEM containing 10% fetal bovine serum. This was followed by the addition to the cells of U0126 diluted so as to provide a final concentration of 97.7 nM to 25.0 µM. Dimethyl sulfoxide (DMSO) was the solvent used to dissolve the U0126, and the procedure was carried out in such a manner that the final DMSO concentration in the culture medium was 0.25% in all cases. Cell proliferation was measured using the Sulforhodamine B method for each cell line at 5 days after the addition of the U0126. The measurement was carried out by the same method as described in Example 1.

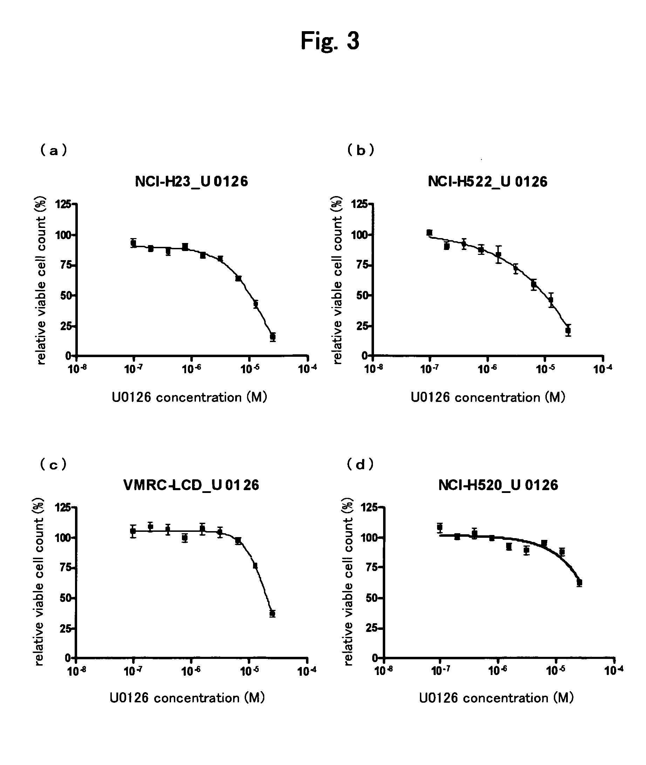

The results are shown in Figure 3.

Based on Figure 3, it is shown that an approximately 50% inhibition of cell proliferation is observed in the NCI-H23 cells and NCI-H522 cells by the addition of 10 µM U0126, as opposed to a weak cell proliferation inhibition for the VMCR-LCD cells and NCI-H520 cells at the same addition.

It was therefore confirmed that the NCI-H23 cells and NCI-H520 cells are cell lines sensitive to the MEK inhibitor and the VMRC-LCD cells and NCI-H520 cells are cell lines that are resistant to the MEK inhibitor.

### (2) Comparison of the level of expression of the SLC27A2 gene in human lung cancer cell lines

The RNA was extracted from the NCI-H23 cells, NCI-H522 cells, VMRC-LCD cells, and NCI-H520 cells and the level of expression of the SLC27A2 gene was measured using quantitative RT-PCR. RNA extraction, cDNA synthesis, and PCR with respect to SLC27A2 were carried out by the same methods as described in Example 1. The expression level of human β-actin was measured as an internal reference using an Assays-on-Demand Gene Expression Assay Mix (Applied Biosystems) and was used for correction.

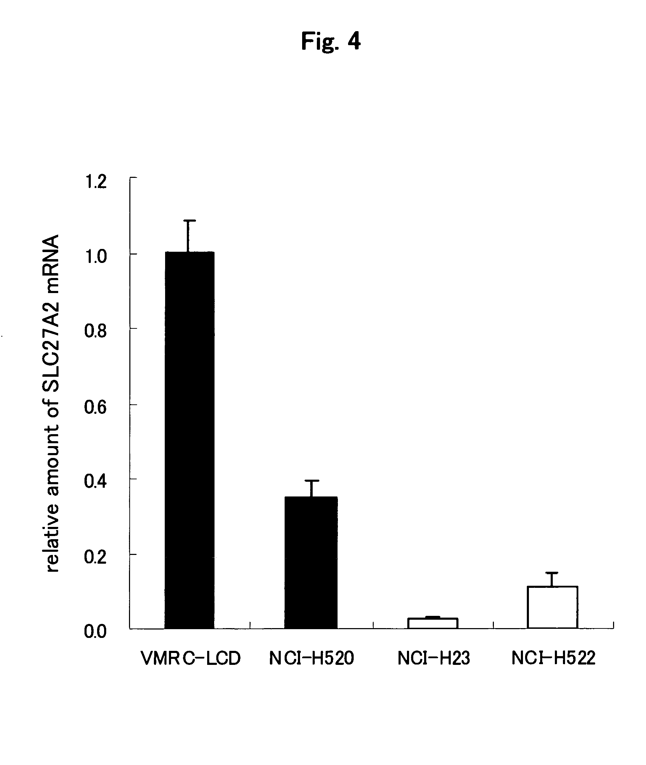

The results are shown in Figure 4.

Based on Figure 4, it is shown that the level of expression of SLC27A2 mRNA in the VMRC-LCD cells and NCI-H520 cells is higher than in the NCI-H23 cells and NCI-H522 cells.

### (3) Inhibition of cancer cell proliferation by SLC27A2 gene knockdown

NCI-H23 cells, NCI-H522 cells, and NCI-H520 cells were inoculated to 96-well plates at a concentration of 2 × 10³/well and were cultured overnight at 37°C under 5% carbon dioxide on DMEM containing 10% fetal bovine serum and then used for an experiment on the inhibition of SLC27A2 expression by RNA interference (RNAi).

The siRNA and nonsilencing oligonucleotide described in Example 1 were used for inhibition of SLC27A2 expression. Transfection of the NCI-H522 cells was carried out by same method as described in Example 1 for the VMRC-LCD cells. The NCI-H23 cells and NCI-H520 cells were transfected using 0.05 µL of the Lipofectamine 2000 reagent (available from Invitrogen), but the other conditions were the same as in the method described in Example 1. Each of the siRNAs was transfected as in Example 1, and cell proliferation was measured after 5 days using the Sulforhodamine B method. In addition, in a preliminary orienting investigation of transfection, it was confirmed that an at least 80% cell proliferation inhibiting activity was observed for the addition of siTOX (available from Dharmacon) for each cell line under the conditions described above.

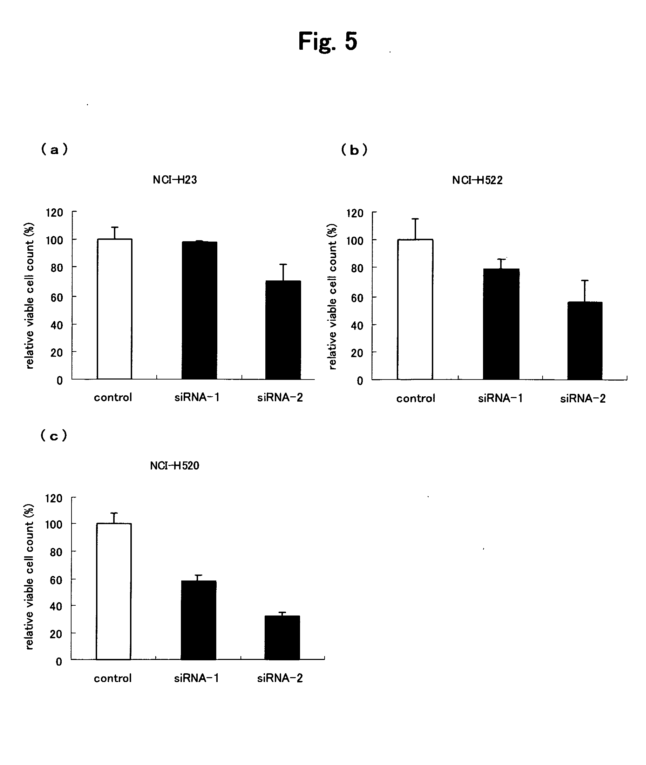

Figure 5 shows the results for the inhibition of cancer cell proliferation by SLC27A2 gene knockdown.

For the NCI-H23 cells and NCI-H522 cells, which are MEK inhibitor-sensitive cancer cell lines, the viable cell count in the experiments in which transfection was carried out with siRNA against SLC27A2 was reduced approximately 3% to about 45% in comparison to the cell count in the nonsilencing oligonucleotide-transfected control experiment. On the other hand, with the NCI-H520 cells, which is an MEK inhibitor-resistant cancer cell line, the viable cell count in the experiments in which transfection was carried out with siRNA against SLC27A2 was reduced approximately 40% to 70% in comparison to the cell count in the control. In addition, as described in Example 1, the reduction was also at least 80% for the VMRC-LCD cells, which are also MEK inhibitor-resistant cancer cells.

This demonstrated that the effect of SLC27A2 gene inhibition is seen much more substantially in the MEK inhibitor-resistant cancer cells than in the MEK inhibitor-sensitive cancer cells.

It is hypothesized that the same effects will also be seen with HER2 inhibitor-resistant cancer cells and with the EGF receptor, which like MEK is positioned upstream in the tyrosine kinase signaling pathway.

### INDUSTRIAL APPLICABILITY

Protein (the protein used by the present invention) comprising the same or substantially the same amino acid sequence as the amino acid sequence represented by SEQ ID NO: 1 exhibits an upregulated expression in cancer cells. In addition, because suppressing its expression results in an inhibition of cancer cell proliferation and a promotion of cancer cell apoptosis, compounds and salts thereof that inhibit the activity of this protein, compounds and salts thereof that inhibit the expression of the gene for this protein, antibodies against this protein, antisense polynucleotide for polynucleotide that encodes this protein, etc., can be used as safe drugs as, for example, an agent for the prevention/treatment of cancer (for example, brain tumors, pituitary adenoma, glioma, acoustic neurinoma, pharyngeal cancer, laryngeal cancer, cancer of the tongue, thymic carcinoma, mesothelioma, breast cancer, lung cancer, non-small cell lung cancer, small cell lung cancer, stomach cancer, esophageal cancer, colorectal cancer, colon cancer, rectal cancer, liver cancer, hepatocellular carcinoma, pancreatic cancer, pancreatic endocrine tumor, biliary canal cancer, gall bladder cancer, penile cancer, kidney cancer, renal pelvic cancer, ureteral cancer, renal cell carcinoma, testicular tumor, prostate cancer, bladder cancer, vulvar cancer, uterine cancer, cervical cancer, endometrial cancer, uterine sarcoma, trophoblastic diseases, vaginal cancer, ovarian cancer, germ cell tumor of the ovary, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myelogenous leukemia, chronic myelogenous leukemia, acute lymphoid leukemia, chronic lymphoid leukemia, adult T-cell leukemia, chronic myeloproliferative disorders, pancreatic endocrine tumor, cancer of unknown primary, etc.), as a promoter of cancer cell apoptosis, or as an inhibitor of cancer cell proliferation.

## Claims

1. A prophylactic/therapeutic agent for cancer, which comprises a compound or its salt inhibiting the activity of a protein comprising the same or substantially the same amino acid sequence as the amino acid sequence represented by SEQ ID NO: 1, its partial peptide, or a salt thereof.

2. A prophylactic/therapeutic agent for cancer, which comprises a compound or its salt inhibiting the expression of a gene for a protein comprising the same or substantially the same amino acid sequence as the amino acid sequence represented by SEQ ID NO: 1, or its partial peptide, or a salt thereof.

3. The prophylactic/therapeutic agent according to claim 1 or 2, wherein said cancer is a tyrosine kinase signaling pathway inhibitor-resistant cancer.

4. An antisense polynucleotide comprising the entire or part of a base sequence complementary or substantially complementary to a base sequence of a polynucleotide encoding a protein comprising the same or substantially the same amino acid sequence as the amino acid sequence represented by SEQ ID NO: 1, or its partial peptide.

5. A pharmaceutical comprising the antisense polynucleotide according to claim 4.

6. The pharmaceutical according to claim 5, which is a prophylactic/therapeutic agent for cancer.

7. The pharmaceutical according to claim 6, wherein said cancer is a tyrosine kinase signaling pathway inhibitor-resistant cancer.

8. A pharmaceutical comprising siRNA or shRNA against a polynucleotide encoding a protein comprising the same or substantially the same amino acid sequence as the amino acid sequence represented by SEQ ID NO: 1, or its partial peptide.

9. The pharmaceutical according to claim 8, which is a prophylactic/therapeutic agent for cancer.

10. The pharmaceutical according to claim 9, wherein said cancer is a tyrosine kinase signaling pathway inhibitor-resistant cancer.

11. A prophylactic/therapeutic agent for cancer, which comprises an antibody against a protein comprising the same or substantially the same amino acid sequence as the amino acid sequence represented by SEQ ID NO: 1, or its partial peptide, or a salt thereof.

12. The pharmaceutical according to claim 11, wherein said cancer is a tyrosine kinase signaling pathway inhibitor-resistant cancer.

13. A diagnostic agent for cancer, which comprises an antibody against a protein comprising the same or substantially the same amino acid sequence as the amino acid sequence represented by SEQ ID NO: 1, or its partial peptide, or a salt thereof.

14. The diagnostic agent according to claim 13, wherein said cancer is a tyrosine kinase signaling pathway inhibitor-resistant cancer.

15. A diagnostic agent for cancer, which comprises a polynucleotide encoding a protein comprising the same or substantially the same amino acid sequence as the amino acid sequence represented by SEQ ID NO: 1, or its partial peptide.

16. The diagnostic agent according to claim 15, wherein said cancer is a tyrosine kinase signaling pathway inhibitor-resistant cancer.

17. A method of diagnosing cancer, which comprises using an antibody against a protein comprising the same or substantially the same amino acid sequence as the amino acid sequence represented by SEQ ID NO: 1, its partial peptide, or a salt thereof, or a polynucleotide encoding said protein, or its partial peptide.

18. The diagnostic method according to claim 17, wherein said cancer is a tyrosine kinase signaling pathway inhibitor-resistant cancer.

19. Use of a protein comprising the same or substantially the same amino acid sequence as the amino acid sequence represented by SEQ ID NO: 1, its partial peptide, or a salt thereof, as a diagnostic marker for cancer.

20. A method of screening a prophylactic/therapeutic agent for cancer, which comprises using a protein comprising the same or substantially the same amino acid sequence as the amino acid sequence represented by SEQ ID NO: 1, its partial peptide, or a salt thereof.

21. A kit for screening a prophylactic/therapeutic agent for cancer, comprising a protein comprising the same or substantially the same amino acid sequence as the amino acid sequence represented by SEQ ID NO: 1, its partial peptide, or a salt thereof.

22. A method of screening a prophylactic/therapeutic agent for cancer, which comprises using a polynucleotide encoding a protein comprising the same or substantially the same amino acid sequence as the amino acid sequence represented by SEQ ID NO: 1, or its partial peptide.

23. A kit for screening a prophylactic/therapeutic agent for cancer, comprising a polynucleotide encoding a protein comprising the same or substantially the same amino acid sequence as the amino acid sequence represented by SEQ ID NO: 1, or its partial peptide.

24. The method of screening according to claim 20 or 22, wherein said cancer is a tyrosine kinase signaling pathway inhibitor-resistant cancer.

25. The kit for screening according to claim 21 or 23, wherein said cancer is a tyrosine kinase signaling pathway inhibitor-resistant cancer.

26. A method of screening for a prophylactic/therapeutic agent for cancer, wherein said method comprises measuring the activity of a protein comprising the same or substantially the same amino acid sequence as the amino acid sequence represented by SEQ ID NO: 1, its partial peptide, or a salt thereof.

27. The method of screening according to claim 26, wherein said activity is acyl-CoA synthase activity or (and) fatty acid transport activity.

28. A method of screening for a prophylactic/therapeutic agent for cancer, wherein said method comprises measuring the amount of a protein comprising the same or substantially the same amino acid sequence as the amino acid sequence represented by SEQ ID NO: 1, its partial peptide, or a salt thereof.

29. A method of preventing/treating cancer, which comprises inhibiting the activity of a protein comprising the same or substantially the same amino acid sequence as the amino acid sequence represented by SEQ ID NO: 1, its partial peptide, or a salt thereof, or inhibiting the expression of a gene for said protein, its partial peptide, or a salt thereof.

30. The preventing/treating method according to claim 29, wherein said cancer is a tyrosine kinase signaling pathway inhibitor-resistant cancer.

31. A method of preventing/treating cancer, comprising administering to a mammal an effective amount of (i) a compound or its salt that inhibits the activity of a protein comprising the same or substantially the same amino acid sequence as the amino acid sequence represented by SEQ ID NO: 1, its partial peptide, or a salt thereof, (ii) a compound or its salt that inhibits the expression of a gene for said protein, or its partial peptide, or a salt thereof, (iii) an antibody against said protein, or its partial peptide, or a salt thereof, or (iv) an antisense polynucleotide comprising the entire or part of a base sequence complementary or substantially complementary to a base sequence of a polynucleotide encoding said protein, or its partial peptide.

32. The preventing/treating method according to claim 31, wherein said cancer is a tyrosine kinase signaling pathway inhibitor-resistant cancer.

33. Use of (i) a compound or its salt that inhibits the activity of a protein comprising the same or substantially the same amino acid sequence as the amino acid sequence represented by SEQ ID NO: 1, its partial peptide, or a salt thereof, (ii) a compound or its salt that inhibits the expression of a gene for said protein, or its partial peptide, or a salt thereof, (iii) an antibody against said protein, or its partial peptide, or a salt thereof, or (iv) an antisense polynucleotide comprising the entire or part of a base sequence complementary or substantially complementary to a base sequence of a polynucleotide encoding said protein, or its partial peptide, to manufacture a prophylactic/therapeutic agent for cancer.

34. The use according to claim 33, wherein said cancer is a tyrosine kinase signaling pathway inhibitor-resistant cancer.

35. A prophylactic/therapeutic agent for cancer, comprising a compound or its salt that inhibits the activity of a fatty acid transporter protein.

36. A prophylactic/therapeutic agent for cancer, comprising a compound or its salt that inhibits the expression of a gene for a fatty acid transporter protein.

37. A method of preventing/treating cancer, which comprises inhibiting the activity of a fatty acid transporter protein, or inhibiting the expression of a gene for a fatty acid transporter protein.

38. A cancer cell apoptosis promoter or a cancer cell proliferation inhibitor, comprising a compound or its salt that inhibits the activity of a protein comprising the same or substantially the same amino acid sequence as the amino acid sequence represented by SEQ ID NO: 1, its partial peptide, or a salt thereof.

39. A cancer cell apoptosis promoter or a cancer cell proliferation inhibitor, comprising a compound or its salt that inhibits the expression of a gene for a protein comprising the same or substantially the same amino acid sequence as the amino acid sequence represented by SEQ ID NO: 1, its partial peptide, or a salt thereof.

40. The pharmaceutical according to claim 5, which is a cancer cell apoptosis promoter or a cancer cell proliferation inhibitor.

41. The pharmaceutical according to claim 8, which is a cancer cell apoptosis promoter or a cancer cell proliferation inhibitor.

42. A cancer cell apoptosis promoter or a cancer cell proliferation inhibitor, comprising an antibody against a protein comprising the same or substantially the same amino acid sequence as the amino acid sequence represented by SEQ ID NO: 1, or its partial peptide, or a salt thereof.

43. A method of screening a pharmaceutical for promoting apoptosis of cancer cells or a pharmaceutical for inhibiting proliferation of cancer cells, wherein said method comprises using a protein comprising the same or substantially the same amino acid sequence as the amino acid sequence represented by SEQ ID NO: 1, its partial peptide, or a salt thereof.

44. A kit for screening a pharmaceutical for promoting apoptosis of cancer cells or a pharmaceutical for inhibiting proliferation of cancer cells, wherein said kit comprises a protein comprising the same or substantially the same amino acid sequence as the amino acid sequence represented by SEQ ID NO: 1, its partial peptide, or a salt thereof.

45. A method of screening a pharmaceutical for promoting apoptosis of cancer cells or a pharmaceutical for inhibiting proliferation of cancer cells, wherein said method comprises using a polynucleotide encoding a protein comprising the same or substantially the same amino acid sequence as the amino acid sequence represented by SEQ ID NO: 1, or its partial peptide.

46. A kit for screening a pharmaceutical for promoting apoptosis of cancer cells or a pharmaceutical for inhibiting proliferation of cancer cells, wherein said kit comprises a polynucleotide encoding a protein comprising the same or substantially the same amino acid sequence as the amino acid sequence represented by SEQ ID NO: 1, or its partial peptide.

47. A method of screening a pharmaceutical for promoting apoptosis of cancer cells or a pharmaceutical for inhibiting proliferation of cancer cells, wherein said method comprises measuring the activity of a protein comprising the same or substantially the same amino acid sequence as the amino acid sequence represented by SEQ ID NO: 1, its partial peptide, or a salt thereof.

48. A method of promoting apoptosis of cancer cells or inhibiting proliferation of cancer cells, wherein said method comprises inhibiting the activity of a protein comprising the same or substantially the same amino acid sequence as the amino acid sequence represented by SEQ ID NO: 1, its partial peptide, or a salt thereof, or inhibiting the expression of a gene for said protein, its partial peptide, or a salt thereof

49. A method of promoting apoptosis of cancer cells or inhibiting proliferation of cancer cells, wherein said method comprises administering to a mammal an effective amount of (i) a compound or its salt that inhibits the activity of a protein comprising the same or substantially the same amino acid sequence as the amino acid sequence represented by SEQ ID NO: 1, its partial peptide, or a salt thereof, (ii) a compound or its salt that inhibits the expression of a gene for said protein, or its partial peptide, or a salt thereof, (iii) an antibody against said protein, or its partial peptide, or a salt thereof, or (iv) an antisense polynucleotide comprising the entire or part of a base sequence complementary or substantially complementary to a base sequence of a polynucleotide encoding said protein, or its partial peptide.

50. Use of (i) a compound or its salt that inhibits the activity of a protein comprising the same or substantially the same amino acid sequence as the amino acid sequence represented by SEQ ID NO: 1, its partial peptide, or a salt thereof, (ii) a compound or its salt that inhibits the expression of a gene for said protein, or its partial peptide, or a salt thereof, (iii) an antibody against said protein, or its partial peptide, or a salt thereof, or (iv) an antisense polynucleotide comprising the entire or part of a base sequence complementary or substantially complementary to a base sequence of a polynucleotide encoding said protein, or its partial peptide, to manufacture a cancer cell apoptosis promoter or a cancer cell proliferation inhibitor.

51. A cancer cell apoptosis promoter or a cancer cell proliferation inhibitor, comprising a compound or its salt that inhibits the activity of a fatty acid transporter protein.

52. A cancer cell apoptosis promoter or a cancer cell proliferation inhibitor, comprising a compound or its salt that inhibits the expression of a gene for a fatty acid transporter protein.

53. A method of promoting apoptosis of cancer cells or inhibiting proliferation of cancer cells, wherein said method comprises inhibiting the activity of a fatty acid transporter protein, or inhibiting the expression of a gene for a fatty acid transporter protein.


SEQUENCE LISTING

<110> Takeda Pharmaceutical Company Limited

<120> Preventing and/or treating agent for cancer

<130> G07-0074

<140> PCT/JP2006/303927
<141> 2006-02-23

<150> JP2005-49331
<151> 2005-02-24

<160> 9

<210> 1
<211> 620
<212> PRT
<213> Homo sapiens

<400> 1
Met Leu Ser Ala Ile Tyr Thr Val Leu Ala Gly Leu Leu Phe Leu Pro
1               5                   10                  15
Leu Leu Val Asn Leu Cys Cys Pro Tyr Phe Phe Gln Asp Ile Gly Tyr
            20                  25                  30
Phe Leu Lys Val Ala Ala Val Gly Arg Arg Val Arg Ser Tyr Gly Gln
        35                  40                  45
Arg Arg Pro Ala Arg Thr Ile Leu Arg Ala Phe Leu Glu Lys Ala Arg
    50                  55                  60
Gln Thr Pro His Lys Pro Phe Leu Leu Phe Arg Asp Glu Thr Leu Thr
65                  70                  75                  80
Tyr Ala Gln Val Asp Arg Arg Ser Asn Gln Val Ala Arg Ala Leu His
                85                  90                  95
Asp His Leu Gly Leu Arg Gln Gly Asp Cys Val Ala Leu Leu Met Gly
            100                 105                 110
Asn Glu Pro Ala Tyr Val Trp Leu Trp Leu Gly Leu Val Lys Leu Gly
        115                 120                 125
Cys Ala Met Ala Cys Leu Asn Tyr Asn Ile Arg Ala Lys Ser Leu Leu
    130                 135                 140
His Cys Phe Gln Cys Cys Gly Ala Lys Val Leu Leu Val Ser Pro Glu
145                 150                 155                 160
Leu Gln Ala Ala Val Glu Glu Ile Leu Pro Ser Leu Lys Lys Asp Asp
                165                 170                 175
Val Ser Ile Tyr Tyr Val Ser Arg Thr Ser Asn Thr Asp Gly Ile Asp
            180                 185                 190
Ser Phe Leu Asp Lys Val Asp Glu Val Ser Thr Glu Pro Ile Pro Glu
        195                 200                 205
Ser Trp Arg Ser Glu Val Thr Phe Ser Thr Pro Ala Leu Tyr Ile Tyr
    210                 215                 220
Thr Ser Gly Thr Thr Gly Leu Pro Lys Ala Ala Met Ile Thr His Gln
225                 230                 235                 240
Arg Ile Trp Tyr Gly Thr Gly Leu Thr Phe Val Ser Gly Leu Lys Ala
                245                 250                 255
Asp Asp Val Ile Tyr Ile Thr Leu Pro Phe Tyr His Ser Ala Ala Leu
            260                 265                 270
Leu Ile Gly Ile His Gly Cys Ile Val Ala Gly Ala Thr Leu Ala Leu
        275                 280                 285
Arg Thr Lys Phe Ser Ala Ser Gln Phe Trp Asp Asp Cys Arg Lys Tyr
    290                 295                 300
Asn Val Thr Val Ile Gln Tyr Ile Gly Glu Leu Leu Arg Tyr Leu Cys
305                 310                 315                 320
Asn Ser Pro Gln Lys Pro Asn Asp Arg Asp His Lys Val Arg Leu Ala
                325                 330                 335
Leu Gly Asn Gly Leu Arg Gly Asp Val Trp Arg Gln Phe Val Lys Arg
            340                 345                 350
Phe Gly Asp Ile Cys Ile Tyr Glu Phe Tyr Ala Ala Thr Glu Gly Asn
        355                 360                 365

Ile Gly Phe Met Asn Tyr Ala Arg Lys Val Gly Ala Val Gly Arg Val
    370                 375                 380
Asn Tyr Leu Gln Lys Lys Ile Ile Thr Tyr Asp Leu Ile Lys Tyr Asp
385                 390                 395                 400
Val Glu Lys Asp Glu Pro Val Arg Asp Glu Asn Gly Tyr Cys Val Arg
                405                 410                 415
Val Pro Lys Gly Glu Val Gly Leu Leu Val Cys Lys Ile Thr Gln Leu
            420                 425                 430
Thr Pro Phe Asn Gly Tyr Ala Gly Ala Lys Ala Gln Thr Glu Lys Lys
        435                 440                 445
Lys Leu Arg Asp Val Phe Lys Lys Gly Asp Leu Tyr Phe Asn Ser Gly
    450                 455                 460
Asp Leu Leu Met Val Asp His Glu Asn Phe Ile Tyr Phe His Asp Arg
465                 470                 475                 480
Val Gly Asp Thr Phe Arg Trp Lys Gly Glu Asn Val Ala Thr Thr Glu
                485                 490                 495
Val Ala Asp Thr Val Gly Leu Val Asp Phe Val Gln Glu Val Asn Val
            500                 505                 510
Tyr Gly Val His Val Pro Asp His Glu Gly Arg Ile Gly Met Ala Ser
        515                 520                 525
Ile Lys Met Lys Glu Asn His Glu Phe Asp Gly Lys Lys Leu Phe Gln
    530                 535                 540
His Ile Ala Asp Tyr Leu Pro Ser Tyr Ala Arg Pro Arg Phe Leu Arg
545                 550                 555                 560
Ile Gln Asp Thr Ile Glu Ile Thr Gly Thr Phe Lys His Arg Lys Met
                565                 570                 575
Thr Leu Val Glu Glu Gly Phe Asn Pro Ala Val Ile Lys Asp Ala Leu
            580                 585                 590
Tyr Phe Leu Asp Asp Thr Ala Lys Met Tyr Val Pro Met Thr Glu Asp
        595                 600                 605
Ile Tyr Asn Ala Ile Ser Ala Lys Thr Leu Lys Leu
    610                 615                 620

<210> 2
<211> 1860
<212> DNA
<213> Homo sapiens

<400> 2
atgctttccg ccatctacac agtcctggcg ggactgctgt tcctgccgct cctggtgaac      60
ctctgctgcc catacttctt ccaggacata ggctacttct tgaaggtggc cgccgtgggc     120
cggagggtgc gcagctacgg gcagcggcgg ccggcgcgca ccatcctgcg ggcgttcctg     180
gagaaagcgc gccagacgcc acacaagcct tttctgctct tccgcgacga gactctcacc     240
tacgcgcagg tggaccggcg cagcaatcaa gtggcccggg cgctgcacga ccacctcggc     300
ctgcgccagg gagactgcgt ggcgctcctt atgggtaacg agccggccta cgtgtggctg     360
tggctggggc tggtgaagct gggctgtgcc atggcgtgcc tcaattacaa catccgcgcg     420
aagtccctgc tgcactgctt ccagtgctgc ggggcgaagg tgctgctggt gtcgccagaa     480
ctacaagcag ctgtcgaaga gatactgcca agccttaaaa aagatgatgt gtccatctat     540
tatgtgagca gaacttctaa cacagatggg attgactctt tcctggacaa agtggatgaa     600
gtatcaactg aacctatccc agagtcatgg aggtctgaag tcactttttc cactcctgcc     660
ttatacattt atacttctgg aaccacaggt cttccaaaag cagccatgat cactcatcag     720
cgcatatggt atggaactgg cctcactttt gtaagcggat tgaaggcaga tgatgtcatc     780
tatatcactc tgccctttta ccacagtgct gcactactga ttggcattca cggatgtatt     840
gtggctggtg ctactcttgc cttgcggact aaattttcag ccagccagtt ttgggatgac     900
tgcagaaaat acaacgtcac tgtcattcag tatatcggtg aactgcttcg gtatttatgc     960
aactcaccac agaaaccaaa tgaccgtgat cataaagtga gactggcact gggaaatggc    1020
ttacgaggag atgtgtggag acaatttgtc aagagatttg gggacatatg catctatgag    1080
ttctatgctg ccactgaagg caatattgga tttatgaatt atgcgagaaa agttggtgct    1140
gttggaagag taaactacct acagaaaaaa atcataactt atgacctgat taaatatgat    1200
gtggagaaag atgaacctgt ccgtgatgaa aatggatatt gcgtcagagt tcccaaaggt    1260
gaagttggac ttctggtttg caaaatcaca caacttacac catttaatgg ctatgctgga    1320
gcaaaggctc agacagagaa gaaaaaactg agagatgtct ttaagaaagg agacctctat    1380
ttcaacagtg gagatctctt aatggttgac catgaaaatt tcatctattt ccacgacaga    1440
gttggagata cattccggtg gaaaggggaa aatgtggcca ccactgaagt tgctgataca    1500
gttggactgg ttgattttgt ccaagaagta aatgtttatg gagtgcatgt gccagatcat    1560
gagggtcgca ttggcatggc ctccatcaaa atgaaagaaa accatgaatt tgatggaaag    1620
aaactctttc agcacattgc tgattaccta cctagttatg caaggccccg gtttctaaga    1680
atacaggaca ccattgagat cactggaact tttaaacacc gcaaaatgac cctggtggag    1740

gagggcttta accctgctgt catcaaagat gccttgtatt tcttggatga cacagcaaaa 1800
atgtatgtgc ctatgactga ggacatctat aatgccataa gtgctaaaac cctgaaactc 1860

<210> 3
<211> 620
<212> PRT
<213> Mus musculus

<400> 3
Met Leu Pro Val Leu Tyr Thr Gly Leu Ala Gly Leu Leu Leu Leu Pro
1               5                   10                  15
Leu Leu Leu Thr Cys Cys Cys Pro Tyr Leu Leu Gln Asp Val Arg Tyr
            20                  25                  30
Phe Leu Gln Leu Ala Asn Met Ala Arg Arg Val Arg Ser Tyr Arg Gln
        35                  40                  45
Arg Arg Pro Val Arg Thr Ile Leu Arg Ala Phe Leu Glu Gln Ala Arg
    50                  55                  60
Lys Thr Pro His Lys Pro Phe Leu Leu Phe Arg Asp Glu Thr Leu Thr
65                  70                  75                  80
Tyr Ala Gln Val Asp Arg Arg Ser Asn Gln Val Ala Arg Ala Leu His
                85                  90                  95
Asp Gln Leu Gly Leu Arg Gln Gly Asp Cys Val Ala Leu Phe Met Gly
            100                 105                 110
Asn Glu Pro Ala Tyr Val Trp Ile Trp Leu Gly Leu Leu Lys Leu Gly
        115                 120                 125
Cys Pro Met Ala Cys Leu Asn Tyr Asn Ile Arg Ala Lys Ser Leu Leu
    130                 135                 140
His Cys Phe Gln Cys Cys Gly Ala Lys Val Leu Leu Ala Ser Pro Asp
145                 150                 155                 160
Leu Gln Glu Ala Val Glu Glu Ala Leu Pro Thr Leu Lys Lys Asp Ala
                165                 170                 175
Val Ser Val Phe Tyr Val Ser Arg Thr Ser Asn Thr Asn Gly Val Asp
            180                 185                 190
Thr Ile Leu Asp Lys Val Asp Gly Val Ser Ala Glu Pro Thr Pro Glu
        195                 200                 205
Ser Trp Arg Ser Glu Val Thr Phe Thr Thr Pro Ala Val Tyr Ile Tyr
    210                 215                 220
Thr Ser Gly Thr Thr Gly Leu Pro Lys Ala Ala Thr Ile Asn His His
225                 230                 235                 240
Arg Leu Trp Tyr Gly Thr Gly Leu Ala Met Ser Ser Gly Ile Thr Ala
                245                 250                 255
Gln Asp Val Ile Tyr Thr Thr Met Pro Leu Tyr His Ser Ala Ala Leu
            260                 265                 270
Met Ile Gly Leu His Gly Cys Ile Val Val Gly Ala Thr Leu Ala Leu
        275                 280                 285
Arg Ser Lys Phe Ser Ala Ser Gln Phe Trp Asp Asp Cys Arg Lys Tyr
    290                 295                 300
Asn Val Thr Val Ile Gln Tyr Ile Gly Glu Leu Leu Arg Tyr Leu Cys
305                 310                 315                 320
Asn Thr Pro Gln Lys Pro Asn Asp Arg Asp His Lys Val Lys Lys Ala
                325                 330                 335
Leu Gly Asn Gly Leu Arg Gly Asp Val Trp Arg Glu Phe Ile Lys Arg
            340                 345                 350
Phe Gly Asp Ile His Val Tyr Glu Phe Tyr Ala Ser Thr Glu Gly Asn
        355                 360                 365
Ile Gly Phe Val Asn Tyr Pro Arg Lys Ile Gly Ala Val Gly Arg Ala
    370                 375                 380
Asn Tyr Leu Gln Arg Lys Val Ala Arg Tyr Glu Leu Ile Lys Tyr Asp
385                 390                 395                 400
Val Glu Lys Asp Glu Pro Val Arg Asp Ala Asn Gly Tyr Cys Ile Lys
                405                 410                 415
Val Pro Lys Gly Glu Val Gly Leu Leu Val Cys Lys Ile Thr Gln Leu
            420                 425                 430
Thr Pro Phe Ile Gly Tyr Ala Gly Gly Lys Thr Gln Thr Glu Lys Lys
        435                 440                 445
Lys Leu Arg Asp Val Phe Lys Lys Gly Asp Ile Tyr Phe Asn Ser Gly
    450                 455                 460
Asp Leu Leu Met Ile Asp Arg Glu Asn Phe Val Tyr Phe His Asp Arg

465                 470                 475                 480
Val Gly Asp Thr Phe Arg Trp Lys Gly Glu Asn Val Ala Thr Thr Glu
                485                 490                 495
Val Ala Asp Ile Val Gly Leu Val Asp Phe Val Glu Glu Val Asn Val
            500                 505                 510
Tyr Gly Val Pro Val Pro Gly His Glu Gly Arg Ile Gly Met Ala Ser
        515                 520                 525
Leu Lys Ile Lys Glu Asn Tyr Glu Phe Asn Gly Lys Lys Leu Phe Gln
    530                 535                 540
His Ile Ala Glu Tyr Leu Pro Ser Tyr Ala Arg Pro Arg Phe Leu Arg
545                 550                 555                 560
Ile Gln Asp Thr Ile Glu Ile Thr Gly Thr Phe Lys His Arg Lys Val
                565                 570                 575
Thr Leu Met Glu Glu Gly Phe Asn Pro Thr Val Ile Lys Asp Thr Leu
            580                 585                 590
Tyr Phe Met Asp Asp Ala Glu Lys Thr Phe Val Pro Met Thr Glu Asn
        595                 600                 605
Ile Tyr Asn Ala Ile Ile Asp Lys Thr Leu Lys Leu
    610                 615                 620

<210> 4
<211> 1860
<212> DNA
<213> Mus musculus

<400> 4
atgctgccag tgctctacac cggcctggcg gggctgctgc tgctgcctct gctgctcacc      60
tgctgctgcc cctacctcct ccaagatgtg cggtacttcc tgcggctggc caacatggcc     120
cggcgggtgc gcagctaccg gcagcggcga cccgtgcgta ccatcctgcg ggccttcctg     180
gaacaagcgc gcaagacccc acacaagccc ttcctgctgt tccgagacga gacgctcacc     240
tacgcccagg tggaccggcg cagcaaccaa gtggcgcggg cgctgcacga tcaactgggc     300
ctacgacagg gggattgcgt agccctcttc atgggcaatg agccggccta cgtgtggatc     360
tggctgggac tgctcaaact gggctgtccc atggcgtgcc tcaactacaa cattcgtgcc     420
aagtctctgc tgcactgctt tcaatgctgc ggggcgaagg tgctgctggc ctccccagat     480
ctacaagaag ctgtggagga ggttcttcca accctgaaaa aggatgccgt gtccgtcttt     540
tacgtaagca gaacttctaa cacaaatggt gtggacacaa tactggacaa agtagacgga     600
gtgtcggcgg aacccacccc ggagtcgtgg aggtctgaag tcacttttac cacgccagca     660
gtatacattt atacttcggg aaccacaggt cttccaaaag cggcaaccat caatcatcat     720
cgcctatggt atgggacagg ccttgctatg tcgagtggaa tcacggccca ggatgtcatc     780
tataccacaa tgcccctgta ccacagtgca gcgctcatga tcggccttca cggatgcatc     840
gtggttgggg ctactttagc tttgcggagc aaattctcag ccagccagtt ttgggacgac     900
tgcaggaaat acaacgtcac ggtcattcag tacattggtg aactgcttcg gtacctgtgc     960
aacacaccgc agaaaccaaa tgaccgggac cacaaagtga aaaaagccct gggaaatggc    1020
ttacgaggag atgtgtggag agagttcatc aagagatttg gggacatcca cgtgtatgag    1080
ttctacgcat ccactgaagg caacattgga tttgtgaact atccaaggaa aatcggtgct    1140
gtcgggagag caaactacct acaaagaaaa gttgcaaggt atgagctgat caagtatgac    1200
gtggagaagg acgagccggt ccgtgacgca aatggatatt gcatcaaagt ccccaaaggt    1260
gaggttggac tcttggtttg caaaatcaca cagctcacac catttattgg ctatgctgga    1320
ggaaagaccc agacagagaa gaaaaaactc agagatgtct ttaagaaagg cgacatctac    1380
ttcaacagcg gagacctcct gatgatcgac cgtgagaact tcgtctactt tcacgacagg    1440
gttggagata ctttccggtg gaaaggagag aacgtagcta ccacagaagt cgctgacatc    1500
gtgggactgg tagattttgt tgaagaagtg aatgtgtatg gcgtgcctgt gccaggtcat    1560
gagggtcgaa ttgggatggc ctccctcaag atcaaagaaa actacgagtt caatggaaag    1620
aaactctttc aacacatcgc ggagtacctg cccagttacg cgaggcctcg gttcctgagg    1680
atacaagata ccattgagat cactgggact tttaaacacc gcaaagtgac cctgatggaa    1740
gagggcttca atcccacagt catcaaagat accttgtatt tcatggatga tgcagagaaa    1800
acatttgtgc ccatgactga gaacatttat aatgccataa ttgataaaac tctgaagctc    1860

<210> 5
<211> 620
<212> PRT
<213> Rattus norvegicus

<400> 5
Met Leu Pro Val Leu Tyr Thr Gly Leu Ala Gly Leu Leu Leu Leu Pro
1               5                   10                  15
Leu Leu Leu Thr Cys Cys Cys Pro Tyr Leu Leu Gln Asp Val Arg Phe
            20                  25                  30

Phe Leu Gln Leu Ala Asn Met Ala Arg Gln Val Arg Ser Tyr Arg Gln
            35                  40                  45
Arg Arg Pro Val Arg Thr Ile Leu His Val Phe Leu Glu Gln Ala Arg
        50                  55                  60
Lys Thr Pro His Lys Pro Phe Leu Leu Phe Arg Asp Glu Thr Leu Thr
65                  70                  75                  80
Tyr Ala Gln Val Asp Arg Arg Ser Asn Gln Val Ala Arg Ala Leu His
                85                  90                  95
Asp His Leu Gly Leu Arg Gln Gly Asp Cys Val Ala Leu Phe Met Gly
            100                 105                 110
Asn Glu Pro Ala Tyr Val Trp Leu Trp Leu Gly Leu Leu Lys Leu Gly
        115                 120                 125
Cys Pro Met Ala Cys Leu Asn Tyr Asn Ile Arg Ala Lys Ser Leu Leu
    130                 135                 140
His Cys Phe Gln Cys Cys Gly Ala Lys Val Leu Leu Ala Ser Pro Glu
145                 150                 155                 160
Leu His Glu Ala Val Glu Glu Val Leu Pro Thr Leu Lys Lys Glu Gly
                165                 170                 175
Val Ser Val Phe Tyr Val Ser Arg Thr Ser Asn Thr Asn Gly Val Asp
            180                 185                 190
Thr Val Leu Asp Lys Val Asp Gly Val Ser Ala Asp Pro Ile Pro Glu
        195                 200                 205
Ser Trp Arg Ser Glu Val Thr Phe Thr Thr Pro Ala Val Tyr Ile Tyr
    210                 215                 220
Thr Ser Gly Thr Thr Gly Leu Pro Lys Ala Ala Thr Ile Asn His His
225                 230                 235                 240
Arg Leu Trp Tyr Gly Thr Ser Leu Ala Leu Arg Ser Gly Ile Lys Ala
                245                 250                 255
His Asp Val Ile Tyr Thr Thr Met Pro Leu Tyr His Ser Ala Ala Leu
            260                 265                 270
Met Ile Gly Leu His Gly Cys Ile Val Val Gly Ala Thr Phe Ala Leu
        275                 280                 285
Arg Ser Lys Phe Ser Ala Ser Gln Phe Trp Asp Asp Cys Arg Lys Tyr
    290                 295                 300
Asn Ala Thr Val Ile Gln Tyr Ile Gly Glu Leu Leu Arg Tyr Leu Cys
305                 310                 315                 320
Asn Thr Pro Gln Lys Pro Asn Asp Arg Asp His Lys Val Lys Ile Ala
                325                 330                 335
Leu Gly Asn Gly Leu Arg Gly Asp Val Trp Arg Glu Phe Ile Lys Arg
            340                 345                 350
Phe Gly Asp Ile His Ile Tyr Glu Phe Tyr Ala Ser Thr Glu Gly Asn
        355                 360                 365
Ile Gly Phe Met Asn Tyr Pro Arg Lys Ile Gly Ala Val Gly Arg Glu
    370                 375                 380
Asn Tyr Leu Gln Lys Lys Val Val Arg His Glu Leu Ile Lys Tyr Asp
385                 390                 395                 400
Val Glu Lys Asp Glu Pro Val Arg Asp Ala Asn Gly Tyr Cys Ile Lys
                405                 410                 415
Val Pro Lys Gly Glu Val Gly Leu Leu Ile Cys Lys Ile Thr Glu Leu
            420                 425                 430
Thr Pro Phe Phe Gly Tyr Ala Gly Gly Lys Thr Gln Thr Glu Lys Lys
        435                 440                 445
Lys Leu Arg Asp Val Phe Lys Lys Gly Asp Val Tyr Phe Asn Ser Gly
    450                 455                 460
Asp Leu Leu Met Ile Asp Arg Glu Asn Phe Ile Tyr Phe His Asp Arg
465                 470                 475                 480
Val Gly Asp Thr Phe Arg Trp Lys Gly Glu Asn Val Ala Thr Thr Glu
                485                 490                 495
Val Ala Asp Ile Val Gly Leu Val Asp Phe Val Glu Glu Val Asn Val
            500                 505                 510
Tyr Gly Val Pro Val Pro Gly His Glu Gly Arg Ile Gly Met Ala Ser
        515                 520                 525
Ile Lys Met Lys Glu Asn Tyr Glu Phe Asn Gly Lys Lys Leu Phe Gln
    530                 535                 540
His Ile Ser Glu Tyr Leu Pro Ser Tyr Ser Arg Pro Arg Phe Leu Arg
545                 550                 555                 560
Ile Gln Asp Thr Ile Glu Ile Thr Gly Thr Phe Lys His Arg Lys Val
                565                 570                 575

Thr Leu Met Glu Glu Gly Phe Asn Pro Ser Val Ile Lys Asp Thr Leu
            580                 585                 590
Tyr Phe Met Asp Asp Thr Glu Lys Thr Tyr Val Pro Met Thr Glu Asp
        595                 600                 605
Ile Tyr Asn Ala Ile Ile Asp Lys Thr Leu Lys Leu
    610                 615                 620

<210> 6
<211> 1860
<212> DNA
<213> Rattus norvegicus

<400> 6
atgctgcctg tgctctacac cggcctggcg gggctgctgc tgctgcctct gctgctcacc      60
tgctgctgcc cctacctcct ccaggacgtg cggttcttcc tgcaactggc caacatggcc     120
cggcaggtgc gcagctaccg gcagcggcga cccgtgcgca ccatcctgca tgtcttcttg     180
gagcaagcgc gcaagacccc gcacaagccc ttcctgctgt ttcgcgacga gacgcttacc     240
tacgcccagg tagaccggcg cagcaaccaa gtagcgcgag cgctgcatga tcacctgggc     300
ctgcggcagg gggattgcgt ggccctcttc atgggcaatg agccggccta cgtgtggctc     360
tggctgggac tgctcaaact gggctgtccc atggcgtgcc tcaactacaa catccgtgcc     420
aagtctctgc tacactgctt tcagtgctgc ggggcgaagg tgctgctggc ctccccagag     480
ctacacgaag ctgtcgagga ggttcttcca accctgaaaa aggagggcgt gtccgtcttc     540
tacgtaagca gaacttctaa cactaatggc gtggacacag tactggacaa agtagacggg     600
gtgtcggcgg accccatccc ggagtcgtgg aggtctgaag tcacgttcac cacacccgca     660
gtctacatat atacttcggg caccacaggt cttccaaagg ctgcaaccat taatcaccat     720
cgcctctggt atgggaccag ccttgccctg aggtccggaa ttaaggctca tgacgtcatc     780
tacaccacca tgcccctgta ccacagcgcg gcgctcatga ttggcctcca cggatgcatt     840
gtggttgggg ctacatttgc tttgcggagc aaattttcag ccagccagtt ttgggacgac     900
tgcaggaaat acaacgccac tgtcattcag tacatcggtg aactgcttcg gtacctctgc     960
aacacgcccc agaaaccaaa tgaccgggac cacaaagtga aaatagcact aggaaatggc    1020
ttacgaggag atgtgtggag agagttcatc aagagatttg gggacattca catttatgag    1080
ttctacgctt ccactgaagg caacattgga tttatgaact atccaagaaa aatcggagct    1140
gttggaagag aaaattacct acaaaaaaaa gttgtaaggc acgagctgat caagtatgac    1200
gtggagaagg atgagcctgt ccgtgatgca aatggatatt gcatcaaagt ccccaaagga    1260
gaggttggac tcttgatttg caaaatcaca gagctcacac cattttttgg ctatgctgga    1320
ggaaagaccc agacagagaa gaaaaagctc agagatgttt ttaagaaagg agacgtctac    1380
ttcaacagtg gcgatctcct gatgatcgac cgtgaaaatt tcatctattt tcacgacaga    1440
gttggagaca ccttccggtg gaaaggagag aatgtagcta ccacggaagt cgctgacatt    1500
gtgggactgg tagattttgt tgaagaagtg aatgtttacg gtgtgcccgt gccaggtcat    1560
gaaggtcgca tcgggatggc ctcgatcaag atgaaagaaa actacgagtt caatggaaag    1620
aaactctttc agcacatctc ggagtacctg cccagttact cgaggcctcg gttcctgaga    1680
atacaagata ccattgagat caccgggact tttaaacacc gcaaagtgac cctgatggaa    1740
gagggcttta acccctcagt catcaaagat accttgtatt tcatggatga cacagaaaaa    1800
acatacgtgc ccatgactga ggacatttat aatgccataa ttgataagac tctgaagctc    1860


<210> 7
<211> 20
<212> RNA
<213> Artificial Sequence

<220>
<223> siRNA

<400> 7
ggacaucuau aaugcauauu                                                  20

<210> 8
<211> 21
<212> RNA
<213> Artificial Sequence

<220>
<223> siRNA

<400> 8
caaaguggau gaaguaucau u                                                21

<210> 9
<211> 21
<212> DNA
<213> Artificial Sequence

<220>
<223> DNA:RNA hybrid molecule

<400> 9
uucuccgaac gugucacgut t                                                21